# EUROPEAN PATENT SPECIFICATION

(11) **EP 1 297 151 B1**
(45) Date of publication and mention of the grant of the patent: **21.06.2006**
(21) Application number: 01930916.0
(22) Date of filing: 25.04.2001
(51) Int. Cl.: C12N 15/54, C12N 9/12, C12N 5/10, C07K 16/40, C12Q 1/68, G01N 33/50

(54) **14911 NOVEL PROTEIN KINASE MOLECULES AND USES THEREFOR**
PROTEINKINASE-MOLEKÜL 14911 UND DESSEN ANWENDUNGEN
NOUVELLES MOLECULES DE PROTEINE KINASE 14911 ET LEURS UTILISATIONS

(30) Priority: 25.04.2000 US 199391 P; 15.06.2000 US 593927
(43) Date of publication of application: 02.04.2003
(73) Proprietor: MILLENNIUM PHARMACEUTICALS, INC., Cambridge, MA 02139 (US)
(72) Inventor: MEYERS, Rachel, Newton, MA 02468 (US); HUNTER, John, Joseph, Somerville, MA 02143 (US)
(74) Representative: Harrison Goddard Foote
(86) International application number: PCT/US2001/013785
(87) International publication number: WO 2001/081589

(56) References cited:
- WO-A-01/38503
- WO-A-01/55356
- WO-A-01/57188
- WO-A-01/81557
- RUIZ-PEREZ V L ET AL: "Mutations in a new gene in Ellis-van Creveld syndrome and Weyers acrodental dysostosis" NATURE GENETICS, NATURE AMERICA, NEW YORK, US, vol. 24, no. 3, March 2000 (2000-03), pages 283-286, XP002157498 ISSN: 1061-4036
- DATABASE EMBL [Online] "Homo sapiens chromosome 5 clone CTC-255N20, complete sequence." Database accession no. AC011338 XP002206535

## Description

### Background of the Invention

Phosphate tightly associated with protein has been known since the late nineteenth century. Since then, a variety of covalent linkages of phosphate to proteins have been found. The most common involve esterification of phosphate to serine, threonine, and tyrosine with smaller amounts being linked to lysine, arginine, histidine, aspartic acid, glutamic acid, and cysteine. The occurrence of phosphorylated proteins implies the existence of one or more protein kinases capable of phosphorylating amino acid residues on proteins, and also of protein phosphatases capable of hydrolyzing phosphorylated amino acid residues on proteins.

Kinases play a critical role in the mechanism of intracellular signal transduction. They act on the hydroxyamino acids of target proteins to catalyze the transfer of a high energy phosphate group from adenosine triphosphate (ATP). This process is known as protein phosphorylation. Along with phosphatases, which remove phosphates from phosphorylated proteins, kinases participate in reversible protein phosphorylation. Reversible phosphorylation acts as the main strategy for regulating protein activity in eukaryotic cells.

Protein kinases play critical roles in the regulation of biochemical and morphological changes associated with cell proliferation, differentiation, growth and division (D'Urso, G*. et al.* (1990) *Science* 250: 786-791; Birchmeier. C*. et al.* (1993) *Bioessays* 15: 185-189). They serve as growth factor receptors and signal transducers and have been implicated in cellular transformation and malignancy (Hunter, T. *et al.* (1992) *Cell* 70: 375-387; Posada, J. *et al.* (1992) *Mol. Biol. Cell* 3: 583-592; Hunter, *T. et al.* (1994) *Cell* 79: 573-582). For example, protein kinases have been shown to participate in the transmission of signals from growth-factor receptors (Sturgill, T. W. *et al.* (1988) *Nature* 344: 715-718; Gomez, N. *et al.* (1991) *Nature* 353: 170-173), control of entry of cells into mitosis (Nurse, P. (1990) *Nature* 344: 503-508; Maller, J. L. (1991) *Curr. Opin. Cell Biol.* 3: 269-275) and regulation of actin bundling (Husain-Chishti, A. *et al.* (1988) *Nature* 334: 718-721).

Kinases vary widely in their selectivity and specificity of target proteins. They still may, however, comprise the largest known enzyme superfamily. Protein kinases can be divided into two main groups based on either amino acid sequence similarity or specificity for either serine/threonine or tyrosine residues. Serine/threonine specific kinases are often referred to as STKs while tyrosine specific kinases are referred to as PTKs. A small number of dual-specificity kinases are structurally like the serine/threonine-specific group. Within the broad classification, kinases can be further sub-divided into families whose members share a higher degree of catalytic domain amino acid sequence identity and also have similar biochemical properties. Most protein kinase family members also share stnictural features outside the kinase domain that reflect their particular cellular roles. These include regulatory domains that control kinase activity or interaction with other proteins (Hanks, S.K. *et al.* (1988) *Science* 241: 42-52).

Almost all kinases contain a catalytic domain composed of 250-300 conserved amino acids. This catalytic domain may be viewed as composed of 11 subdomains. Some of these subdomains apparently contain distinct amino acid motifs which confer specificity as a STK or PTK or both. Kinases may also contain additional amino acid sequences, usually between 5 and 100 residues, flanking or occurring within the catalytic domain. These residues apparently act to regulate kinase activity and to determine substrate specificity. (Reviewed in Hardie, G. and Hanks, S. (1995) The Protein Kinase Facts Book, Vol 1:7-20 Academic Press, San Diego, Calif.)

Approximately one third of the known oncogenes encode PTKs. PTKs may occur as either transmembrane or soluble proteins. Transmembrane PTKs act as receptors for many growth factors. Interaction of a growth factor to its cognate receptor initiates the phosphorylation of specific tyrosine residues in the receptor itself as well as in certain second messenger proteins. Growth factors found to associate with such PTK receptors include epidermal growth factor, platelet-derived growth factor, fibroblast growth factor, hepatocyte growth factor, insulin and insulin-like growth factors, nerve growth factor, vascular endothelial growth factor, and macrophage colony stimulating factor.

Soluble PTKs often interact with the cytosolic domains of plasma membrane receptors. Receptors that signal through such PTKs include cytokine, hormone, and antigen-specific lymphocytic receptors. Many PTKs were identified as oncogene products by the observation that PTK activation was no longer subject to normal cellular controls. Also, increased tyrosine phosphorylation activity is often observed in cellular transformation, or oncogenesis, (Carbonneau, H. and Tonks, N. K. (1992) Annu. Rev. Cell Biol. 8:463-93.) PTK regulation may therefore be an important strategy in controlling some types of cancer. A human serine/threonine kinase, AJ250840 and a Mus musculus serine/threonine kinase, AJ250839 are disclosed in Ruiz-Perez, V.L., *et al .* (2000) Nature Genetics 24, 283-286. The complete sequence of Homo sapien chromosome 5 clone CTC-255N20, is disclosed in EMBL database accession number: AC011338. WO01/55356 discloses kinase polypeptides, nucleotides sequences encoding the kinase polypeptides, as well as products and methods useful for the diagnosis and treatment of kinase-related diseases and conditions

Further deregulated cell proliferation is the hallmark of cancer. Kinases play a role in the transduction of signals for cell proliferation, differentiation, and apoptosis. Alterations in such genes and their products are frequent in human cancer, and a number of classic proto-oncogenes are members of the kinase family.

### Summary of the Invention

The present invention is based, at least in part, on the discovery of novel nucleic acid molecules and proteins encoded by such nucleic acid molecules, referred to herein as "kinases" or by the individual clone names "14911". The present invention provides methods for the diagnosis and treatment of cancer, including but not limited to, lung, breast, colon and brain cancer. The 14911 nucleic acid and protein molecules of the present invention are useful as modulating agents in regulating a variety of cellular processes, e.g., including cell proliferation, differentiation, growth and division. In particular; the kinase and its related nucleic acids will be advantageous in the regulation of any cellular function uncontrolled proliferation and differentiation, such as in cases of cancer. Accordingly, in one aspect, this invention provides isolated nucleic acid molecules encoding 14911 proteins or biologically active portions thereof, as well as nucleic acid fragments suitable as primers or hybridization probes for the detection of 14911-encoding nucleic acids.

In another preferred embodiment, an isolated nucleic acid molecule encodes the amino acid sequence of a human 14911. In yet another preferred embodiment, the nucleic acid molecule includes a nucleotide sequence encoding a protein which includes the amino acid sequence of SEQ ID NO:2. In yet another preferred embodiment, the nucleic acid molecule includes a nucleotide sequence encoding a protein having the amino acid sequence of SEQ ID NO:2.

Another embodiment of the invention features nucleic acid molecules, preferably 14911 nucleic acid molecules, which specifically detect 14911 nucleic acid molecules relative to nucleic acid molecules encoding non-14911 proteins. For example, in one embodiment, such a nucleic acid molecule is at least 50, 100, 150, 200, 250, 300, 350, 400, 450, 500, 550, 600, 650, 700, 750, or 800 nucleotides in length and hybridizes under stringent conditions to a nucleic acid molecule comprising the nucleotide sequence shown in SEQ ID NO:1, or a complement thereof.

In other preferred embodiments, the nucleic acid molecule encodes a naturally occurring allelic variant of a polypeptide which includes the amino acid sequence of SEQ ID NO:2, wherein the nucleic acid molecule hybridizes to a nucleic acid molecule which includes SEQ ID NO: 1 or SEQ ID NO:3 under stringent conditions.

Another embodiment of the invention provides an isolated nucleic acid molecule which is antisense to a 14911 nucleic acid molecule, e.g., the coding strand of a 14911 nucleic acid molecule.

Another aspect of the invention provides a vector comprising a 14911 nucleic acid molecule. In certain embodiments, the vector is a recombinant expression vector. In another embodiment, the invention provides a host cell containing a vector of the invention. The invention also provides a method for producing a protein, preferably a 14911 protein, by culturing in a suitable medium, a host cell, e.g., a mammalian host cell such as a non-human mammalian cell, of the invention containing a recombinant expression vector, such that the protein is produced.

Another aspect of this invention features isolated or recombinant 14911 proteins and polypeptides.

In another embodiment, the protein, preferably a 14911 protein, has the amino acid sequence of SEQ ID No:2.

The proteins of the present invention or biologically active portions thereof, can be operatively linked to a non-14911 polypeptide (e.g., heterologous amino acid sequences) to form fusion proteins. The invention further features antibodies, such as monoclonal or polyclonal antibodies, that specifically bind proteins of the invention, preferably 14911 proteins. In addition, the 14911 proteins or biologically active portions thereof can be incorporated into pharmaceutical compositions, which optionally include pharmaceutically acceptable carriers.

In another aspect, the present invention provides a method for detecting the presence of a 14911 nucleic acid molecule, protein or polypeptide in a biological sample by contacting the biological sample with an agent capable of detecting a 14911 nucleic acid molecule, protein or polypeptide such that the presence of a 14911 nucleic acid molecule, protein or polypeptide is detected in the biological sample.

In another aspect, the present invention provides a method for detecting the presence of 14911 activity in a biological sample by contacting the biological sample with an agent capable of detecting an indicator of 14911 activity such that the presence of 14911 activity is detected in the biological sample.

In another aspect, the invention provides a method for modulating 14911 activity comprising contacting a cell capable of expressing 14911 with an agent that modulates 14911 activity such that 14911 activity in the cell is modulated. In one embodiment, the agent inhibits 14911 activity. In another embodiment, the agent stimulates 14911 activity. In one embodiment, the agent is an antibody that specifically binds to a 14911 protein. In another embodiment, the agent modulates expression of 14911 by modulating transcription of a 14911 gene or translation of a 14911 mRNA. In yet another embodiment, the agent is a nucleic acid molecule having a nucleotide sequence that is antisense to the coding strand of a 14911 mRNA or a 14911 gene.

In one embodiment, the methods of the present invention are used to treat a subject having a disorder characterized by aberrant 14911 protein or nucleic acid expression or activity by administering an agent which is a 14911 modulator to the subject. In one embodiment, the 14911 modulator is a 14911 protein. In another embodiment the 14911 modulator is a 14911 nucleic acid molecule. In yet another embodiment, the 14911 modulator is a peptide, peptidomimetic, or other small molecule. In a preferred embodiment, the disorder characterized by aberrant 14911 protein or nucleic acid expression is a cellular growth related disorder.

In still another aspect, the invention provides a process for modulating 14911 polypeptide or nucleic acid expression or activity, e.g. using the screened compounds described herein. Compounds that modulate expression and/or activity of the receptors are used for treatment and diagnosis of kinase-related disorders. These compounds are useful for the treatment of tumors such as colon, lung, breast and brain tumors.

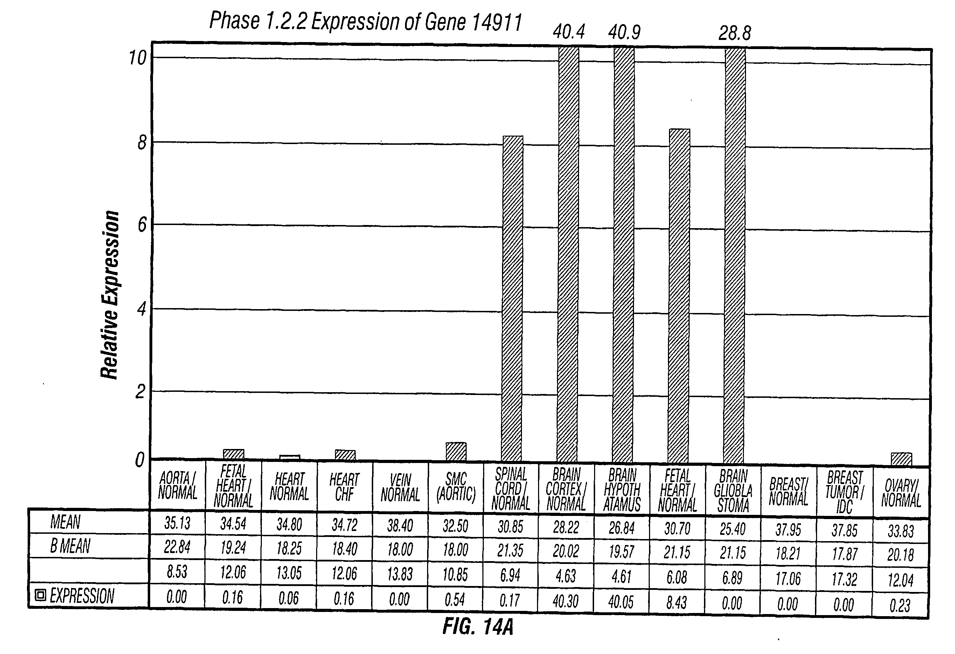

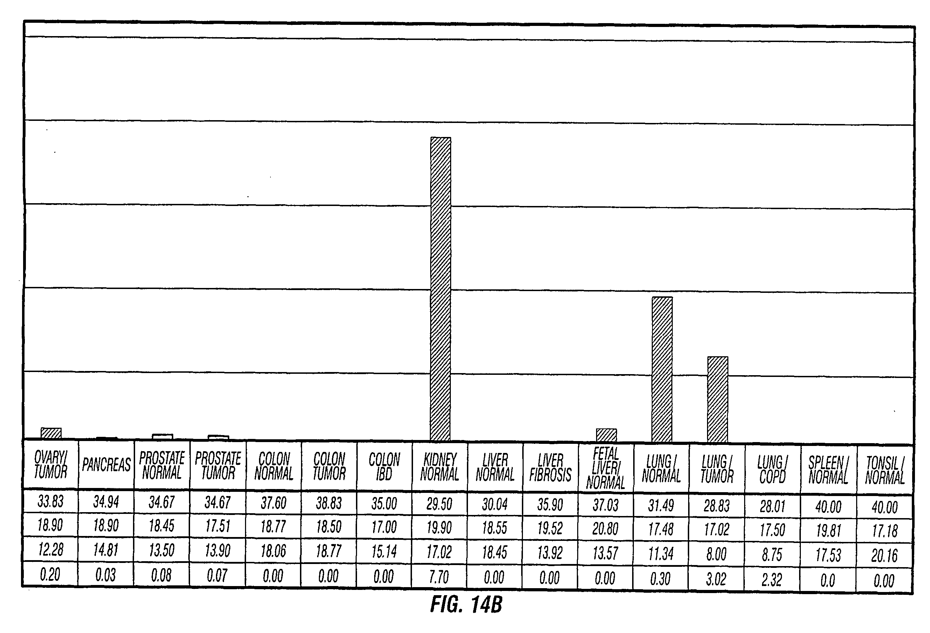

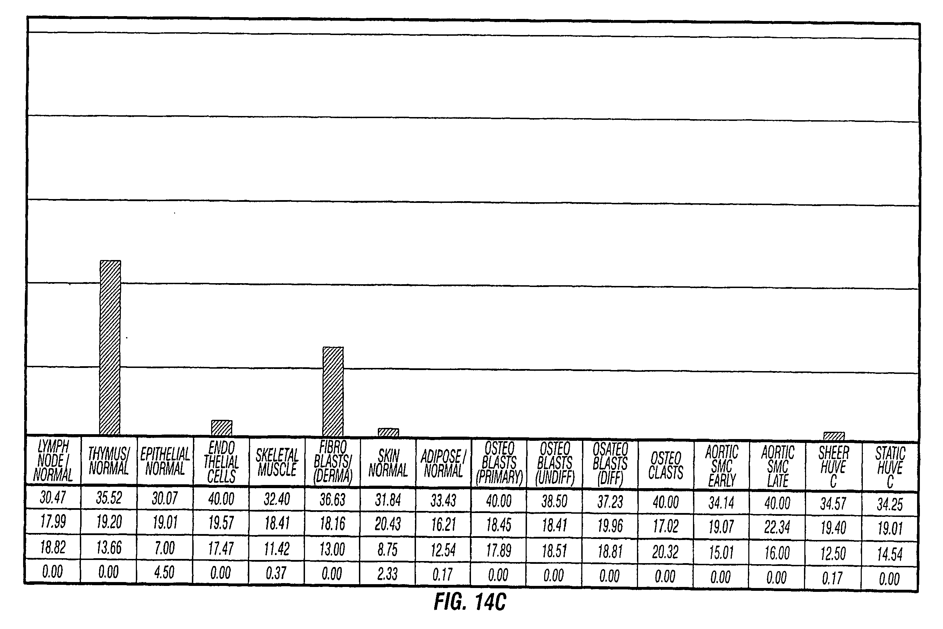

Disorders associated with the following cells or tissues, in which 14911 has been expressed as shown in Figure 14, are also encompassed: spinal cord, normal brain cortex, brain hypothalamus, glial cells, brain glioblastoma, kidney, lung, epithelial cells and skin, among others. Other disorders are those characterized by aberrant activity or expression of the 14911 polypeptides or nucleic acids, as well as aberrant or deficient mobilization of an intracellular molecule that participates in a phosphorylation; and/or aberrant or deficient modulation of function, survival, morphology, proliferation and/or differentiation of cells of tissues in which 14911 molecules are expressed.

The present invention also provides a diagnostic assay for identifying the presence or absence of a genetic alteration characterized by at least one of (i) aberrant modification or mutation of a gene encoding a 14911 protein; (ii) mis-regulation of the gene; and (iii) aberrant post-translational modification of a 14911 protein, wherein a wild-type form of the gene encodes a protein with a 14911 activity.

In another aspect the invention provides a method for identifying a compound that binds to or modulates the activity of a 14911 protein, by providing an indicator composition comprising a 14911 protein having 1491 activity, contacting the indicator composition with a test compound, and determining the effect of the test compound on 14911 activity in the indicator composition to identify a compound that modulates the activity of a 14911 protein.

Other features and advantages of the invention will be apparent from the following detailed description and claims.

### Brief Description of the Drawings

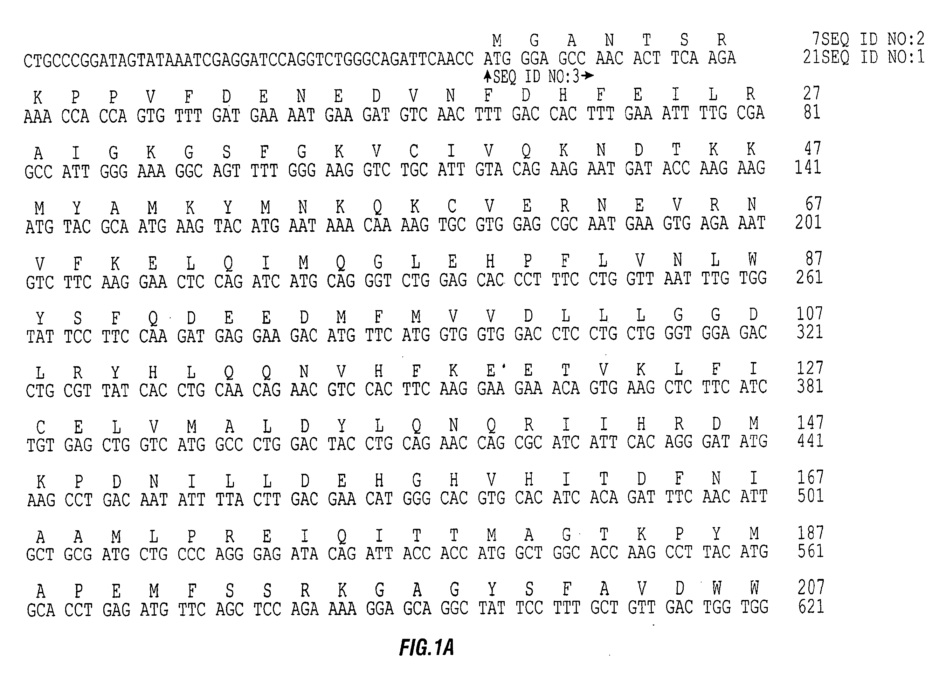

*Figures 1a-b* depict a cDNA sequence (SEQ ID NO:1) and predicted amino acid sequence (SEQ ID NO:2) of human 14911. The location of the methionine-initiated open reading frame of human 14911 (without the 5' and 3' untranslated regions) is also indicated in the Figure (SEQ ID NO:3).

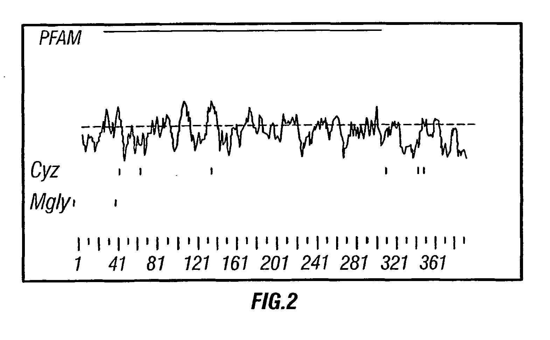

*Figure 2* depicts a hydropathy plot of human 14911. Relatively hydrophobic residues are shown above the dashed horizontal line, and relatively hydrophilic residues are below the dashed horizontal line. The cysteine residues (cys) and N-glycosylation sites (Ngly) are indicated by short vertical lines just below the hydropathy trace. The numbers corresponding to the amino acid sequence of human 14911 are indicated. Polypeptides of the invention include fragments which include: all or part of a hydrophobic sequence, e.g., a sequence above the dashed line, e.g., the sequence from about amino acid 95 to 105, from about 125 to 140, and from about 200 to 215 of SEQ ID NO:2; all or part of a hydrophilic sequence, e.g., a sequence below the dashed line, e.g., the sequence from about amino acid 106 to 120, from about 215 to 230, and from about 320 to 340 of SEQ ID NO:2; a sequence which includes a Cys, or a glycosylation site.

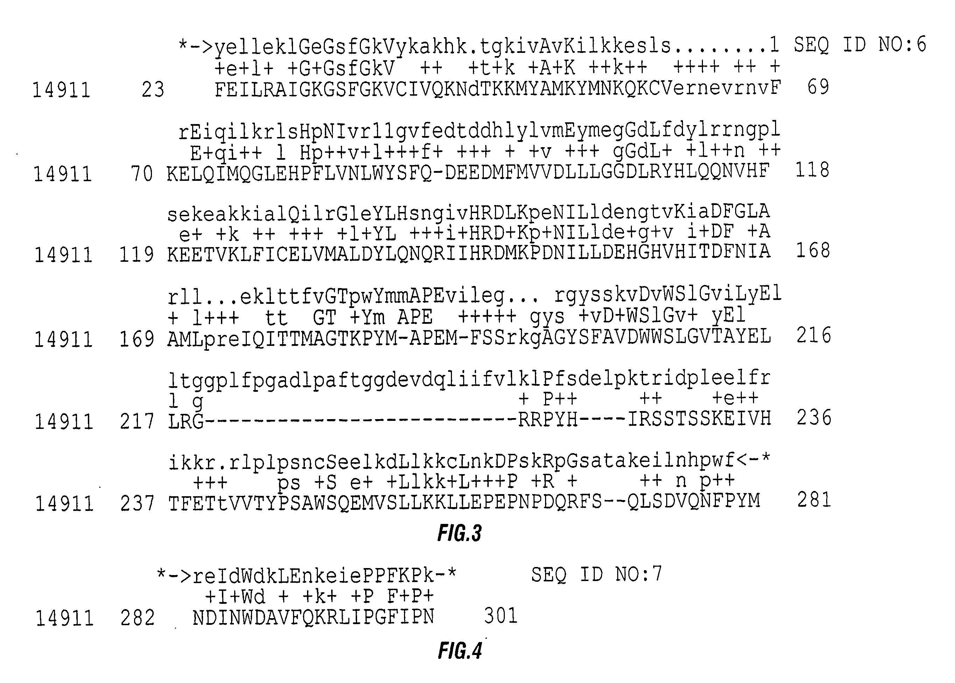

*Figure 3* depicts an alignment of the eukaryotic protein kinase family domain of human 14911 with a consensus amino acid sequence derived from a hidden Markov model (HMM) from PFAM. The upper sequence is the consensus amino acid sequence (SEQ ID NO:6), while the lower amino acid sequence corresponds to amino acids 23 to 281 of SEQ ID NO:2.
*Figure 4* depicts an alignment of the protein kinase C terminal domain of human 14911 with a consensus amino acid sequence derived from a hidden Markov model (HMM) from PFAM. The upper sequence is the consensus amino acid sequence (SEQ ID NO:7), while the lower amino acid sequence corresponds to amino acids 282 to 301 of SEQ ID NO:2.

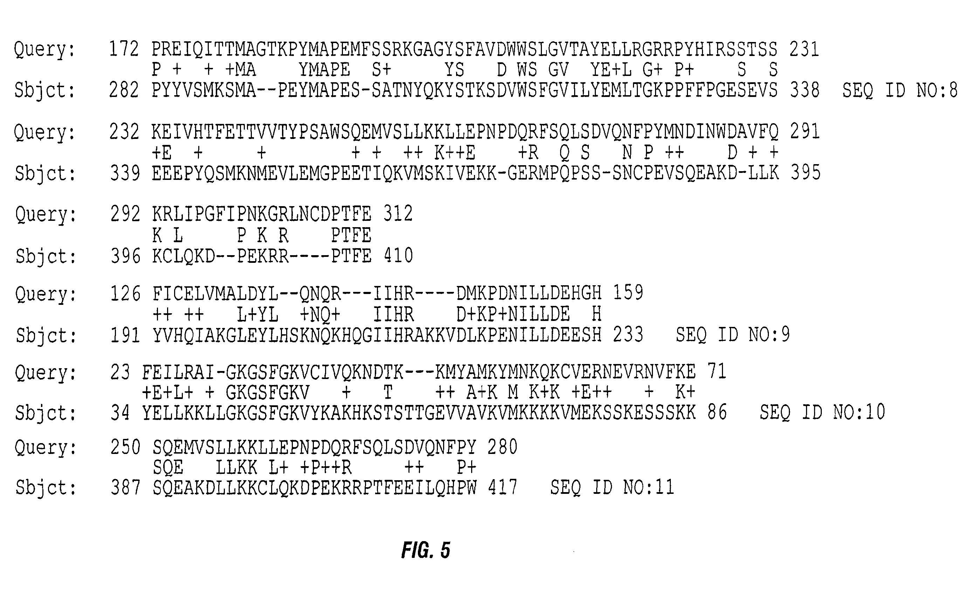

*Figures 5a-d* depict a BLAST alignment of human 14911 with a consensus amino acid sequence derived from a ProDomain "kinase protein transferase ATP-binding serine/threonine - protein phosphorylation receptor tyrosine - protein precursor transmembrane" (Release 1999.2; see also ProDomain Release 2000.1; http://www.toulouse.inra.fr/prodom.html). The lower sequence is amino acid residues 34 to 410 of the amino acid consensus sequence (SEQ ID NOS:8-11), while the upper amino acid sequence corresponds to the "kinase protein transferase ATP-binding serine/threonine - protein phosphorylation receptor tyrosine - protein precursor transmembrane" domain of human 14911, amino acid residues 23 to 312 of SEQ ID NO:2. The BLAST algorithm identifies multiple local alignments between the consensus amino acid sequence and human 14911. Figure 5A depicts the first local alignment, Figure 5B the second, Figure 5C the third, and Figure 5D the fourth.

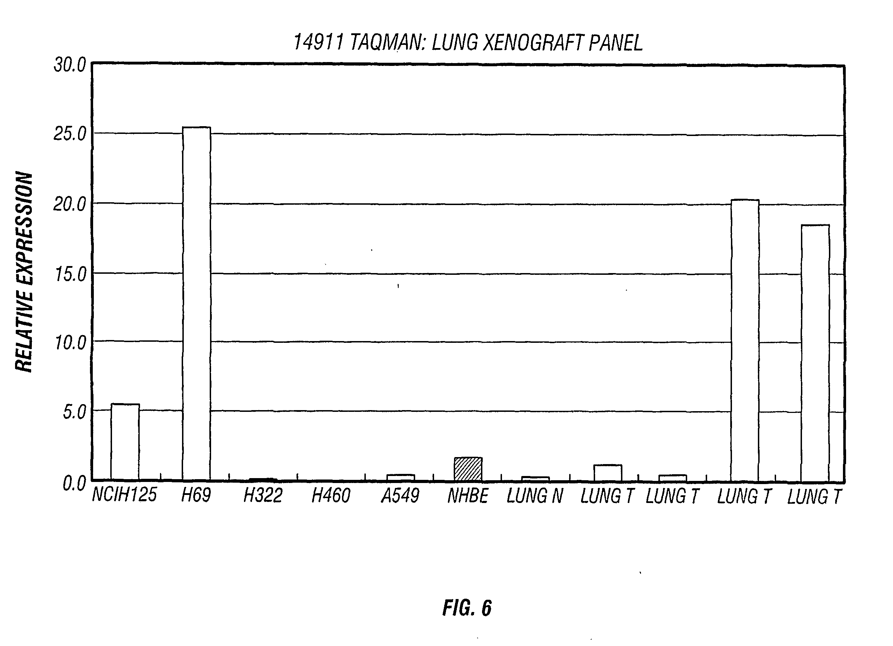

*Figure 6* is a lung xenograph panel bar graph depicting the expression of 14911 RNA relative to a no template control showing an increased expression in 2/5 lung tumor cell lines in comparison with a normal human bronchial epithelium (NHBE) control, which expression was detected using Taq Man analysis.

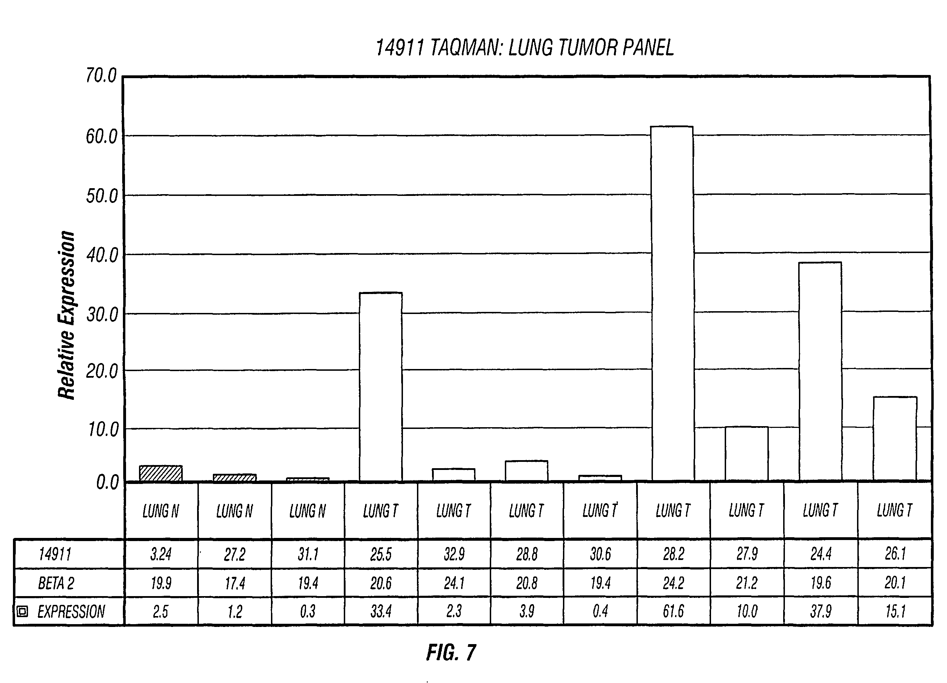

*Figure 7* is an oncology panel bar graph depicting the expression of 14911 RNA relative to a no template control showing an increased expression in 5/8 lung tumor samples in comparison with normal lung tissue, which expression was detected using Taq Man analysis.

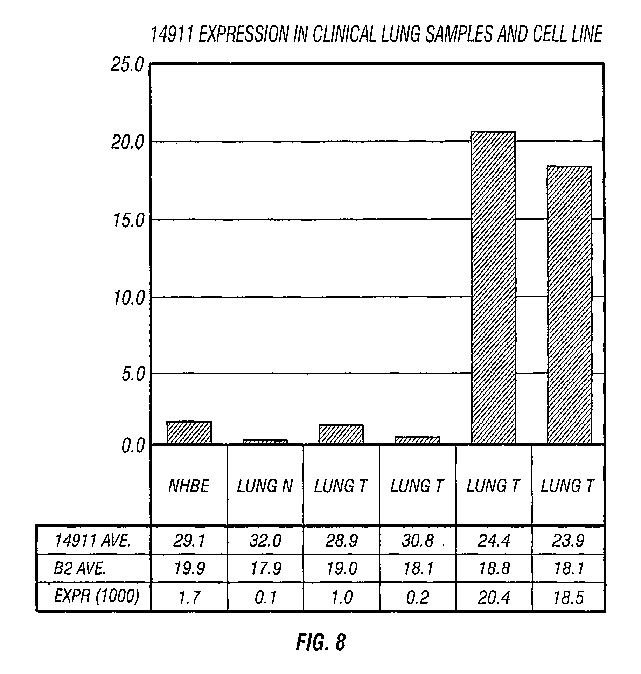

*Figure 8* is an oncology panel bar graph depicting the expression of 14911 RNA relative to a no template control showing an increased expression in 2/5 clinical lung tumor samples in comparison with a normal human bronchial epithelium (NHBE) control, which expression was detected using Taq Man analysis.

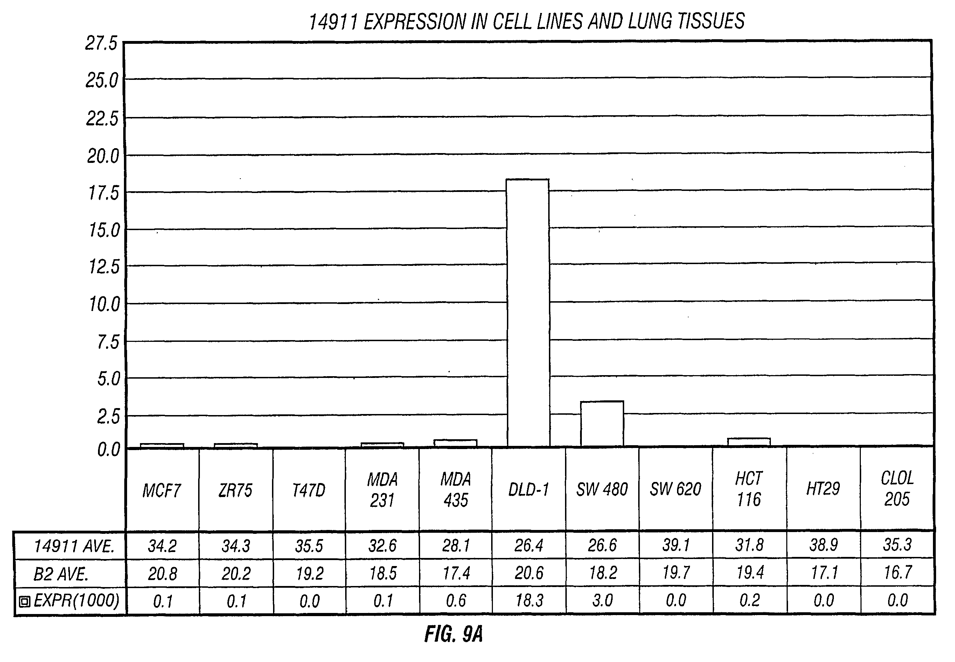

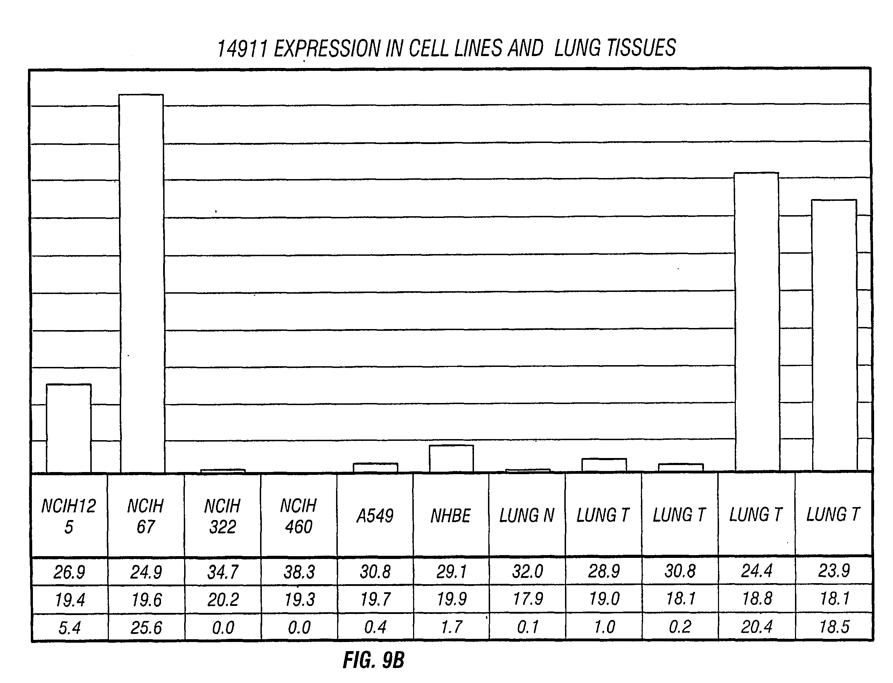

*Figure 9* is an oncology panel bar graph depicting the expression of 14911 RNA relative to a no template control showing an increased expression in various cells lines and lung tumors in comparison with a normal human bronchial epithelium (NHBE) control, which expression was detected using Taq Man analysis.

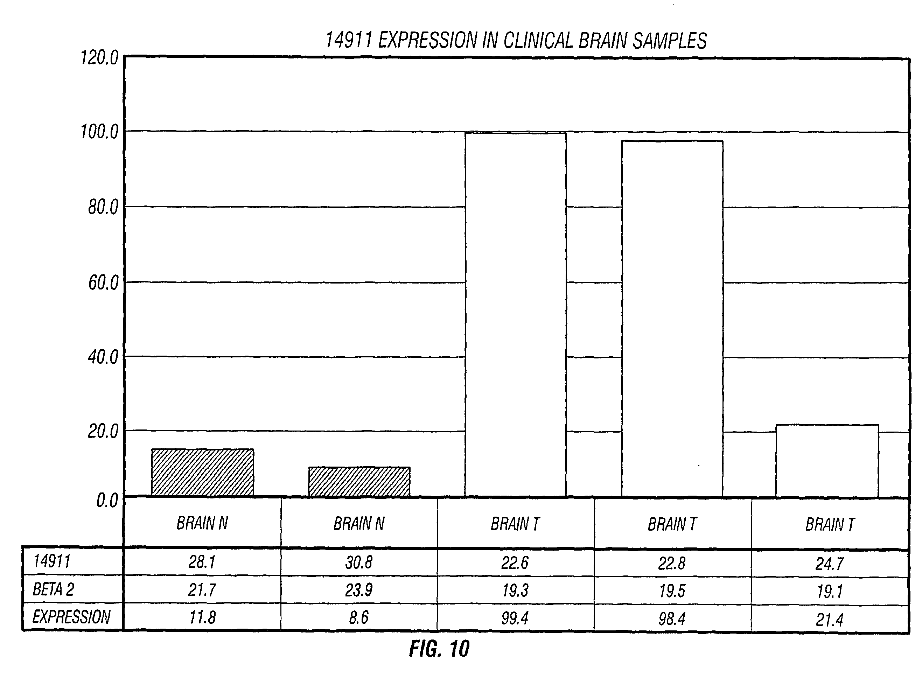

*Figure 10* is an oncology panel bar graph depicting the expression of 14911 RNA relative to a no template control showing an increased expression in 3/3 glioma tumor samples in comparison with normal brain tissue, which expression was detected using Taq Man analysis.

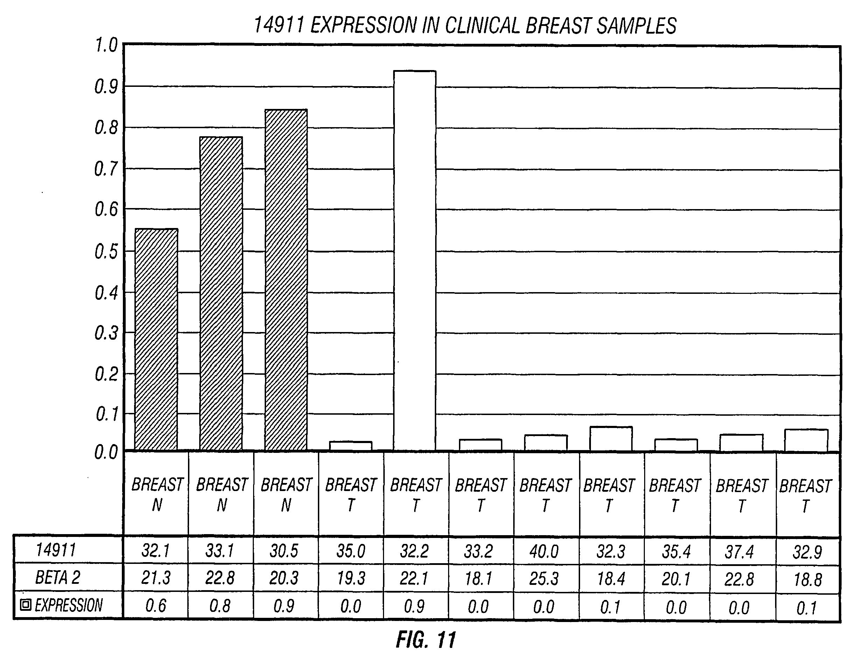

*Figure 11* is an oncology panel bar graph depicting the expression of 14911 RNA relative to a no template control showing a decreased expression in 7/8 breast tumor samples in comparison with normal breast tissue, which expression was detected using Taq Man analysis.

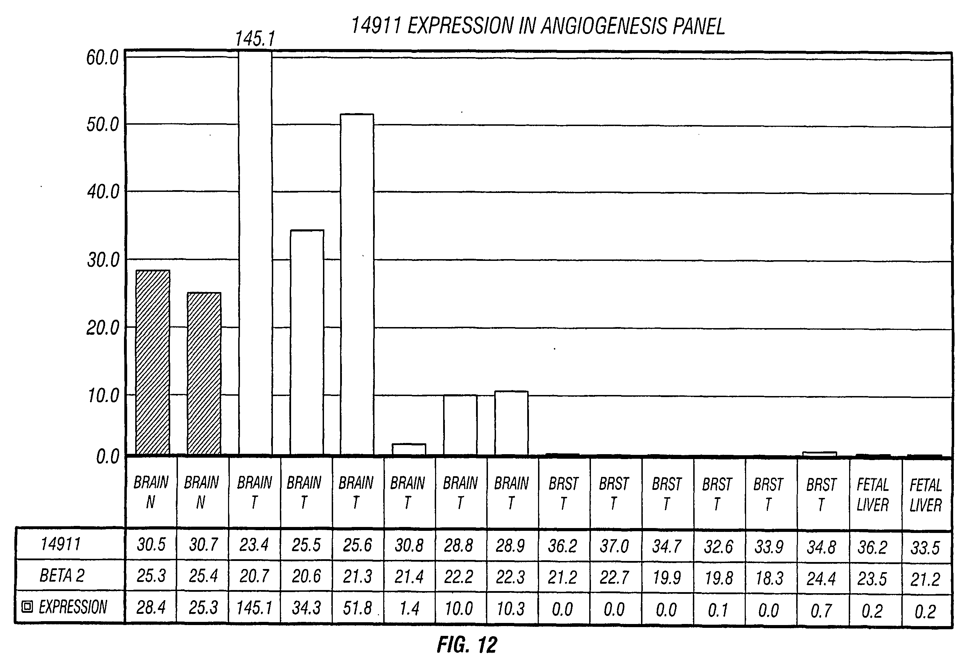

*Figure 12* is an oncology panel bar graph depicting the expression of 14911 RNA relative to a no template control showing an increased expression in 3/6 brain tumor samples in comparison with normal brain tissue, and low expression in breast tumor and fetal liver tissue, which expression was detected using Taq Man analysis.

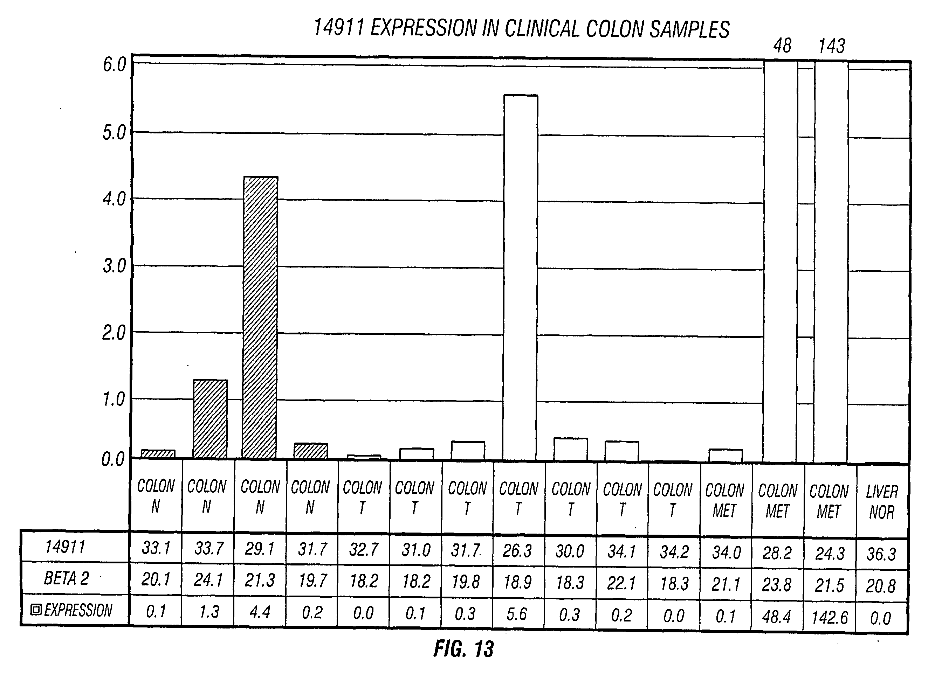

*Figure 13* is an oncology panel bar graph depicting the expression of 14911 RNA relative to a no template control showing an increased expression in 2/3 clinical colon metastases samples in comparison with normal colon tissue, and some expression in clinical colon tumor samples, which expression was detected using Taq Man analysis.
*Figure 14* is a Phase I panel bar graph depicting the relative expression of 14911 RNA relative to a no template controls in a panel of human tissues or cells, including but not limited to heart, brain, breast, ovary, pancreas, prostate, colon, kidney, liver, fetal liver, lung, spleen, tonsil, lymph node, epithelial, endothelial, skeletal, fibroblasts, skin, adipose, bone cells (e.g., osteoclasts and osteoblasts), among others, detected using real-time quantitative RT-PCR Taq Man analysis.

### Detailed Description of the Invention

The present invention is based, at least in part, on the discovery of novel molecules, referred to herein as "14911" nucleic acid and polypeptide molecules, which play a role in or function in the transduction of signals for cell proliferation, differentiation and apoptosis. In one embodiment, the 14911 molecules modulate the activity of one or more proteins involved in cellular growth or differentiation, e.g., cell growth or differentiation. In another embodiment, the 14911 molecules of the present invention are capable of modulating the phosphorylation state of a 14911 molecule or one or more proteins involved in cellular growth or differentiation.

As used herein, the term "protein kinase" includes a protein or polypeptide which is capable of modulating its own phosphorylation state or the phosphorylation state of another protein or polypeptide. Protein kinases can have a specificity for (i.e., a specificity to phosphorylate) serine/threonine residues, tyrosine residues, or both serine/threonine and tyrosine residues, e.g., the dual specificity kinases. As referred to herein, protein kinases preferably include a catalytic domain of about 200-400 amino acid residues in length, preferably about 200-300 amino acid residues in length, or more preferably about 250-300 amino acid residues in length, which includes preferably 5-20, more preferably 5-15, or preferably 11 highly conserved motifs or subdomains separated by sequences of amino acids with reduced or minimal conservation. Specificity of a protein kinase for phosphorylation of either tyrosine or serine/threonine can be predicted by the sequence of two of the subdomains (VIb and VIII] in which different residues are conserved in each class (as described in, for example, Hanks *et al.* (1988) *Science* 241:42-52) the contents of which are incorporated herein by reference). These subdomains are also described in further detail herein.

Protein kinases play a role in signaling pathways associated with cellular growth. For example, protein kinases are involved in the regulation of signal transmission from cellular receptors, e.g., growth-factor receptors; entry of cells into mitosis; and the regulation of cytoskeleton function, e.g., actin bundling. Thus, the 14911 molecules of the present invention may be involved in: 1) the regulation of transmission of signals from cellular receptors, e.g., growth factor receptors; 2) the modulation of the entry of cells into mitosis; 3) the modulation of cellular differentiation; 4) the modulation of cell death; and 5) the regulation of cytoskeleton function.

Additionally, and without being bound by theory, 14911 molecules have been found to be overexpressed in tumor cells, where the molecules may be inappropriately propagating either cell proliferation or cell survival signals. As such, 14911 molecules may serve as specific and novel identifiers of such tumor cells. Further, inhibitors of the 14911 molecules are also useful for the treatment of cancer, preferably lung cancer, and useful as a diagnostic.

Inhibition or over stimulation of the activity of protein kinases involved in signaling pathways associated with cellular growth can lead to perturbed cellular growth, which can in turn lead to cellular growth related disorders. As used herein, a "cellular growth related disorder" includes a disorder, disease, or condition characterized by a deregulation, e.g., an upregulation or a downregulation, of cellular growth. Cellular growth deregulation may be due to a deregulation of cellular proliferation, cell cycle progression, cellular differentiation and/or cellular hypertrophy.

The present invention is based, at least in part, on the discovery of novel molecules, referred to herein as 14911 protein and nucleic acid molecules, which comprise a family of molecules having certain conserved structural and functional features. The term "family" when referring to the protein and nucleic acid molecules of the invention is intended to mean two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin, as well as other, distinct proteins of human origin or alternatively, can contain homologues of non-human origin. Members of a family may also have common functional characteristics.

One embodiment of the invention features 14911 nucleic acid molecules, preferably human 14911 molecules, e.g., 14911. The 14911 nucleic acid and protein molecules of the invention are described in further detail in the following subsections.

### The 14911 Nucleic Acid and Protein Molecules

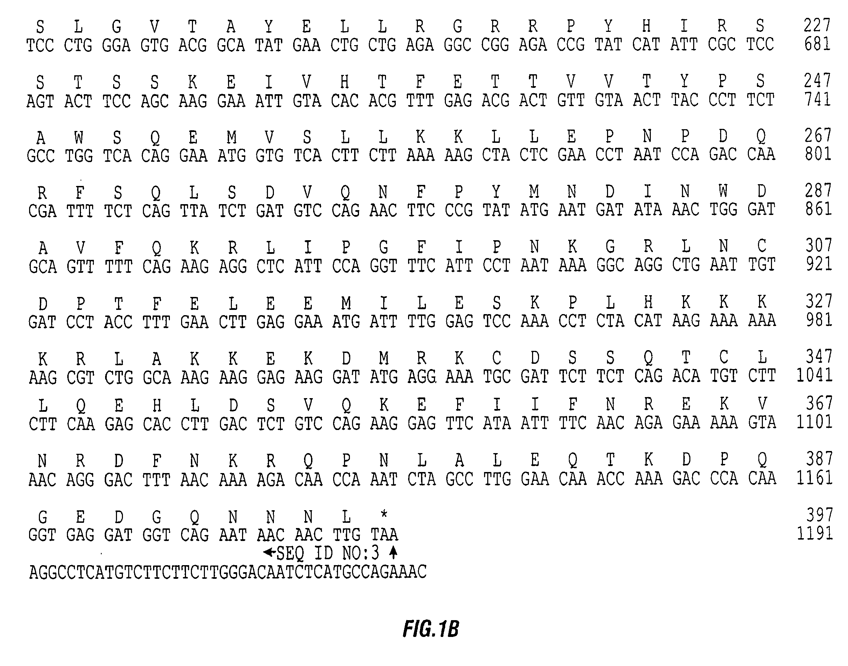

The human 14911 sequence (Figure 1; SEQ ID NO:1), which is approximately 1281 nucleotides long including untranslated regions, contains a predicted methionine-initiated coding sequence of about 1191 nucleotides, including the termination codon (nucleotides indicated as the coding region of SEQ ID NO:1 in Fig. 1; SEQ ID NO:3). The coding sequence encodes a 396 amino acid protein (SEQ ID NO:2).

Human 14911 contains the following regions or other structural features (for general information regarding PFAM identifiers, PS prefix and PF prefix domain identification numbers, refer to Sonnhammer et al. (1997) Protein 28:405-420 and http://www.psc.edu/general/software/packages/pfam/pfam.html):
a eukaryotic protein kinase domain (PFAM Accession Number PF00069) located at about amino acid residues 23 to 281 of SEQ ID NO:2;
a protein kinase C terminal domain (PFAM Accession Number PF00433) located at about amino acid residues 282 to 301 of SEQ ID NO:2;
two N-glycosylation sites (Prosite PS00001) from about amino acids 4 to 7 and 43 to 46 of SEQ ID NO:2;
five protein kinase C phosphorylation sites (Prosite PS00005) at about amino acids 5 to 7, 45 to 47, 122 to 124, 193 to 195, and 230 to 232 of SEQ ID NO:2;
three casein kinase II phosphorylation sites (Prosite PS00006) located at about amino acids S9 to 92, 212 to 215, and 230 to 230 of SEQ ID NO:2;
three N-myristoylation sites (Prosite PS00008) from about amino acids 2 to 7, 197 to 202, and 391 tp 396 of SEQ ID NO:2;
one amidation site (Prosite PS00009) from about amino acids 218 to 221 of SEQ ID NO:2;
one protein kinases ATP-bnding region signature (Prosite PS00107) from about amino acids 29 to 37 of SEQ ID NO:2; and
one serine/threonine protein kinase active-site signal site (Prosite PS00108) from about amino acids 142 to 154 of SEQ ID NO:2.

As used herein, the term "kinase domain" includes an amino acid sequence of about 100 to 275 amino acid residues in length and having a bit score for the alignment of the sequence to the kinase domain (HMM) of at least 100. Preferably a kinase domain mediates intracellular signal transduction. Preferably, a kinase domain includes at least about 100 to 275 amino acids, more preferably about 150 to 275 amino acid residues, or about 200 to 275 amino acids and has a bit score for the alignment of the sequence to the kinase domain (HMM) of at least 100, 150, 200, or greater. An alignment of the kinase domain (amino acids 23 to 281 of SEQ ID NO:2) of human 14911 with a consensus amino acid sequence (SEQ ID NO:2) derived from a hidden Markov model is depicted in Figure 3. The "protein kinase" domain (HMM) has been assigned the PFAM Accession Number PF00069 (http://genome.wustl.edu/Pfam/.html) and corresponds to about amino acids 23 to 281 of SEQ ID NO:2.

In a preferred embodiment, a 14911 polypeptide or protein has a "kinase domain" or a region which includes at least about 100 to 21 more preferably about 150 to 275 or 200 to 275 amino acid residues and has at least about 60%, 70% 80% 90% 95%, 99%, or 100% homology with a "kinase domain," e.g., the kinase domain of human 14911 (e.g., residues 23 to 281 of SEQ ID NO:2).

As used herein, the term "kinase C domain" includes an amino acid sequence of about 5 to 20 amino acid residues in length and having a bit score for the alignment of the sequence to the kinase domain (HMM) of at least 4. Preferably a kinase C domain mediates intracellular signal transduction. Preferably, a kinase C domain includes at least about 5 to 20 amino acids, more preferably about 10 to 20 amino acid residues, or about 15 to 20 amino acids and has a bit score for the alignment of the sequence to the kinase domain (HMM) of at least 4, 6, 8, or greater. An alignment of the kinase C domain (amino acids 282 to 301 of SEQ ID NO:2) of human 14911 with a consensus amino acid sequence (SEQ ID NO:2) derived from a hidden Markov model is depicted in Figure 4. The "protein C kinase" domain (HMM) has been assigned the PFAM Accession Number PF00433 (http://genome.wustl.edu/Pfam/.html) and corresponds to about amino acids 282 to 301 of SEQ ID NO:2.

In a preferred embodiment, a 14911 polypeptide or protein has a "kinase C domain" or a region which includes at least about 5 to 20 more preferably about 10 to 20 or 15 to 20 amino acid residues and has at least about 60%, 70% 80% 90% 95%, 99%, or 100% homology with a "kinase C domain," e.g., the kinase domain of human 14911 (e.g., residues 282 to 301 of SEQ ID NO:2).

To identify the presence of a "kinase" of "kinase C" domain in a 14911 protein sequence, and make the determination that a polypeptide or protein of interest has a particular profile, the amino acid sequence of the protein can be searched against the Pfam database of HMMs (e.g., the Pfam database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Sonhammer et al. (1997) Proteins 28:405-420 and a detailed description ofHMMs can be found, for example, in Gribskov et al. (1990) Meth. Enzymol.183:146-159; Gribskov et al. (1987) Proc. Natl. Acad. Sci. USA 84:4355-4358; Krogh et al. (1994) J. Mol. Biol. 235:1501-1531; and Stultz et al. (1993) Protein Sci. 2:305-314, the contents of which are incorporated herein by reference. A search was performed against the HMM database resulting in the identification of a "kinase domain" and a "kinase C domain" in the amino acid sequence of human 14911 at about residues 23 to 281 of SEQ ID NO:2 (see Figure 1) and a "kinase C domain" at about residues 282 to 301 of SEQ ID NO:2.

To identify the presence of a "kinase" domain in a 14911 protein sequence, and make the determination that a polypeptide or protein of interest has a particular profile, the amino acid sequence of the protein can be searched against a database of domains, e.g., the ProDom database (Corpet et al. (1999), Nucl. Acids Res. 27:263-267). The ProDom protein domain database consists of an automatic compilation of homologous domains. Current versions of ProDom are built using recursive PSI-BLAST searches (Altschul SF et al. (1997) Nucleic Acids Res. 25:3389-3402; Gouzy et al. (1999) Computers and Chemistry 23:333-340) of the SWISS-PROT 38 and TREMBL protein databases. The database automatically generates a consensus sequence for each domain. A BLAST search was performed against the HMM database resulting in the identification of a "kinase" domain in the amino acid sequence of human 14911 at about residues 172 to 312, 126 to 159, 23 to 71 and 250 to 280 of SEQ ID NO:2 (see Figure 1).

The kinase domain is homologous to ProDom family "kinase protein transferase ATP-binding serine/threonine - protein phosphorylation receptor tyrosine - protein precursor transmembrane," SEQ ID NOs:8 and 11, (ProDomain Release 1999.2 http://www.toulouse.inra.fr/prodom.html). The consensus sequence for SEQ ID NO:8 is 29% identical over amino acids 172 to 312 of SEQ ID NO:2 as shown in Figure 5a and the consensus sequence for SEQ ID NO:9 is 44% identical over amino acids 126 to 159 of SEQ ID NO:2 as shown in Figure 5b. The consensus sequence for SEQ ID NO:10 is 33% identical over amino acids 23 to 71 of SEQ ID NO:2 as shown in Figure 5c and the consensus sequence for SEQ ID NO:11 is 35% identical over amino acids 250 to 280 of SEQ ID NO:2 as shown in Figure 5d.

In another embodiment, the isolated proteins of the present invention, preferably 14911 proteins, are identified based on the presence of at least one Ser/Thr kinase site and at least one ATP binding region.

As used herein, the term "Ser/Thr kinase site" includes an amino acid sequence of about 200-400 amino acid residues in length, preferably 200-300 amino acid residues in length, and more preferably 250-300 amino acid residues in length, which is conserved in kinases which phosphorylate serine and threonine residues and found in the catalytic domain of Ser/Thr kinases. Preferably, the Ser/Thr kinase site includes the following amino acid consensus sequence X₉-g-X-G-X₄-V-X₁₂-K-X-₍₁₀₋₁₉₎-E-X₆₆-h-X₈-h-r-D-X-K-X₂-N-X₁₇-K-X₂-D-f-g-X₂₁-p-X₁₃-w-X₃-g-X₅₅-R-X₁₄-h-X₃ (SEQ ID NO:4) (where invariant residues are indicated by upper case letters and nearly invariant residues are indicated by lower case letters). The nearly invariant residues are usually found in most Ser/Thr kinase sites, but can be replaced by other amino acids which, preferably, have similar characteristics. For example, a nearly invariant hydrophobic amino acid in the above amino acid consensus sequence would most likely be replaced by another hydrophobic amino acid. Ser/Thr kinase domains are described in, for example, Levin D.E. *et al.* (1990) *Proc. Natl. Acad. Sci.* USA 87:8272-76, the contents of which are incorporated herein by reference.

As used herein, the term "ATP-binding region" includes an amino acid sequence of about 20-40, preferably 20-30, and more preferably 25-30 amino acid residues in length, present in enzymes which activate their substrates by phosphorylation, and involved in binding adenosine triphosphate (ATP). ATP-binding regions preferably include the following amino acid consensus sequence: G-X-G-X-X-G-X(15-23)-K (SEQ ID NO:5). ATP-binding regions are described in, for example, Samuel K.P. *et al.* (1987) *FEBS Let.* 218(1): 81-86, the contents of which are incorporated herein by reference. Amino acid residues 31 to 39 of comprise an ATP-binding region. Amino acid residues 144-156 of the 14911 protein comprise a Ser/Thr kinase domain.

The nucleic acid encodes a polypeptide with similarities known Ser/Thr kinases. Thus the 14911 encoded polypeptide is expected to be a kinase and function in the phosphorylation of protein substrates. Additionally, the 14911 nucleic acids can be used in known or novel screens and assays for kinase encoding nucleic acids to distinguish it from other distinct nucleic acids. Alternatively, the nucleic acid sequences can be used in the preparation of phylogenetic trees and relationships between organisms.

As used interchangeably herein a "14911 activity", "biological activity of 14911" or "functional activity of 14911", refers to an activity exerted by a 14911 protein, polypeptide or nucleic acid molecule on a 14911 responsive cell or a 14911 protein substrate as determined *in vivo,* or *in vitro,* according to standard techniques. The biological activity of 14911 is described herein.

Accordingly, another embodiment of the invention features isolated 14911 proteins and polypeptides having a 14911 activity. Preferred proteins are 14911 proteins having at least one Ser/Thr kinase and at least one ATP-binding region. Additional preferred proteins have at least one Ser/Thr kinase site, at least one ATP-binding region, and preferably a 14911 activity. Additional preferred proteins have at least one Ser/Thr kinase site, at least one ATP-binding region, and are, preferably, encoded by a nucleic acid molecule having a nucleotide sequence which hybridizes under stringent hybridization conditions to a nucleic acid molecule comprising the nucleotide sequence of SEQ ID NO:1 or SEQ ID NO:3.

Thus, the 14911 molecules can act as novel diagnostic targets and therapeutic agents for controlling one or more disorders. Examples of such disorders, e.g., kinase-associated or other 14911-associated disorders, include but are not limited to, cellular proliferative and/or differentiative disorders, disorders associated with bone metabolism, immune e.g., inflammatory, disorders, cardiovascular disorders, including endothelial cell disorders, liver disorders, viral diseases, pain or metabolic disorders.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of ovary, prostate, colon, lung, breast and liver origin.,

As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, e.g., malignant tumor growth, or may be categorized as non-pathologic, i.e., a deviation from normal but not associated with a disease state, e.g., cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 14911 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, e.g., arising from myeloid, lymphoid or erythroid lineages, or precursor cells thereof. Preferably, the diseases arise from poorly differentiated acute leukemias, e.g., erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L.(1991) Crit Rev. in Oncol./Hemotol. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

The nucleotide sequence of the isolated human 14911 cDNA and the predicted amino acid sequence of the human 14911 polypeptide are shown in Figure 1 and in SEQ ID NOs: 1 and 2, respectively. A plasmid containing the nucleotide sequence encoding human 14911 was deposited with American Type Culture Collection (ATCC), 10801 University Boulevard, Manassas, VA 20110-2209, on June 7, 2001 and assigned Accession Number PTA-3435. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

The 14911 gene, which is approximately 1281 nucleotides in length, encodes a protein having a molecular weight of approximately 43.7 kD and which is approximately 396 amino acid residues in length.

Various aspects of the invention are described in further detail in the following subsections:

### I. Isolated Nucleic Acid Molecules

One aspect of the invention pertains to isolated nucleic acid molecules that encode 14911 proteins or biologically active portions thereof, as well as nucleic acid fragments sufficient for use as hybridization probes to identify 14911-encoding nucleic acids (e.g., 14911 mRNA) and fragments for use as PCR primers for the amplification or mutation of 14911 nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. For example, in various embodiments, the isolated 14911 nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleic acid molecule having the nucleotide sequence of SEQ ID NO:1 or SEQ ID NO:3, or a portion thereof, can be isolated using standard molecular biology techniques and the sequence information provided herein. For example, using all or portion of the nucleic acid sequence of SEQ ID NO: 1, or the nucleotide sequence of SEQ ID NO:3, as a hybridization probe, nucleic acid molecules can be isolated using standard hybridization and cloning techniques (e.g., as described in Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989).

Moreover, a nucleic acid molecule encompassing all or a portion of SEQ ID NO:1 or SEQ ID NO:3 can be isolated by the polymerase chain reaction (PCR) using synthetic oligonucleotide primers designed based upon the sequence of SEQ ID NO:1 or SEQ ID NO:3, respectively.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to 1491 nucleotide sequences can be prepared by standard synthetic techniques, e.g., using an automated DNA synthesizer.

In a preferred embodiment, an isolated nucleic acid molecule of the invention comprises the nucleotide sequence shown in SEQ ID NO: 1. The sequence of SEQ ID NO:1 corresponds to the partial human 14911 cDNA. This cDNA comprises sequences encoding the partial human 14911 protein (i.e., "the coding region", as shown in SEQ ID NO:2), as well as 5' untranslated sequences (48 nucleotides before the coding region) and 3' untranslated sequences (42 nucleotides after the coding region). Alternatively, the nucleic acid molecule can comprise only the coding region of SEQ ID NO:1 (e.g., corresponding to SEQ ID NO:3).

In another preferred embodiment, an isolated nucleic acid molecule of the invention comprises a nucleic acid molecule which is a complement of the nucleotide sequence shown in SEQ ID NO:1 or SEQ ID NO:3, or a portion of any of these nucleotide sequences. A nucleic acid molecule which is complementary to the nucleotide sequence shown in SEQ ID NO: 1 or SEQ ID NO:3, is one which is sufficiently complementary to the nucleotide sequence shown in SEQ ID NO:1 or SEQ ID NO:3, respectively, such that it can hybridize to the nucleotide sequence shown in SEQ ID NO:1 or SEQ ID NO:3, respectively, thereby forming a stable duplex.

Moreover, the nucleic acid molecule of the invention can comprise only a portion of the nucleic acid sequence of SEQ TD NO:1 or SEQ ID NO:3, for example a fragment which can be used as a probe or primer or a fragment encoding a biologically active portion of a 14911 protein. The nucleotide sequence determined from the cloning of the 14911 gene allows for the generation of probes and primers designed for use in identifying and/or cloning other 14911 family members, as well as 14911 homologues from other species. The probe/primer typically comprises substantially purified oligonucleotide. The oligonucleotide typically comprises a region of nucleotide sequence that hybridizes under stringent conditions to at least about 12 or 15, preferably about 20 or 25, more preferably about 30, 35, 40, 45, 50, 55, 60, 65, or 75 consecutive nucleotides of a sense sequence of SEQ ID NO:1 or SEQ ID NO:3, of an anti-sense sequence of SEQ ID NO: or SEQ ID NO: 3, or of a naturally occurring allelic variant or mutant of SEQ ID NO:1 or SEQ ID NO:3. In an exemplary embodiment, a nucleic acid molecule of the present invention comprises a nucleotide sequence which is at least 350, 400, 450, 500, 550, 600, 650, 700, 750, or 800 nucleotides in length and hybridizes under stringent hybridization conditions to a nucleic acid molecule of SEQ ID NO:1 or SEQ ID NO:3.

Probes based on the 14911 nucleotide sequences can be used to detect transcripts or genomic sequences encoding the same or homologous proteins. In preferred embodiments, the probe further comprises a label group attached thereto, e.g., the label group can be a radioisotope, a fluorescent compound, an enzyme, or an enzyme co-factor. Such probes can be used as a part of a diagnostic test kit for identifying cells or tissues which misexpress a 14911 protein, such as by measuring a level of a 14911-encoding nucleic acid in a sample of cells from a subject e.g., detecting 14911 mRNA levels or determining whether a genomic 14911 gene has been mutated or deleted.

A nucleic acid fragment encoding a "biologically active portion of a 14911 protein" can be prepared by isolating a portion of the nucleotide sequence of SEQ ID NO:1 or SEQ ID NO:3, which encodes a polypeptide having a 14911 biological activity (the biological activities of the 14911 proteins are described herein), expressing the encoded portion of the 14911 protein (e.g., by recombinant expression *in vitro)* and assessing the activity of the encoded portion of the 14911 protein.

The invention further encompasses nucleic acid molecules that differ from the nucleotide sequence shown in SEQ ID NO:1 or SEQ ID NO:3, due to the degeneracy of the genetic code and, thus, encode the same 14911 proteins as those encoded by the nucleotide sequence shown in SEQ ID NO:1 or SEQ ID NO:3. In another embodiment, an isolated nucleic acid molecule of the invention has a nucleotide sequence encoding a protein having an amino acid sequence shown in SEQ ID NO:2.

In addition to the 14911 nucleotide sequences shown in SEQ ID NO:1 or SEQ ID NO:3, it will be appreciated by those skilled in the art that DNA sequence polymorphisms that lead to changes in the amino acid sequences of the 14911 proteins may exist within a population (e.g., the human population). Such genetic polymorphism in the 14911 genes may exist among individuals within a population due to natural allelic variation. As used herein, the terms "gene" and "recombinant gene" refer to nucleic acid molecules which include an open reading frame encoding an 14911 protein, preferably a mammalian 14911 protein, and can further include non-coding regulatory sequences, and introns. Such natural allelic variations include both functional and non-functional 14911 proteins and can typically result in 1-5% variance in the nucleotide sequence of a 14911 gene. Any and all such nucleotide variations and resulting amino acid polymorphisms in 14911 genes that are the result of natural allelic variation and that do not alter the functional activity of a 14911 protein are intended to be within the scope of the invention.

Moreover, nucleic acid molecules encoding other 14911 family members and, thus, which have a nucleotide sequence which differs from the 14911 sequences of SEQ ID NO:1 or SEQ ID NO:3 are intended to be within the scope of the invention. For example, another 14911 cDNA can be identified based on the nucleotide sequence of human 14911. Moreover, nucleic acid molecules encoding 14911 proteins from different species, and thus which have a nucleotide sequence which differs from the 14911 sequences of SEQ ID NO:1 or SEQ ID NO:3 are intended to be within the scope of the invention. For example, a mouse 14911 cDNA can be identified based on the nucleotide sequence of a human 14911.

Nucleic acid molecules corresponding to natural allelic variants and homologues of the 14911 cDNAs of the invention can be isolated based on their homology to the 14911 nucleic acids disclosed herein using the cDNAs disclosed herein, or a portion thereof, as a hybridization probe according to standard hybridization techniques under stringent hybridization conditions.

Accordingly, in another embodiment, an isolated nucleic acid molecule of the invention is at least 15, 20, 25, 30 or more nucleotides in length and hybridizes under stringent conditions to the nucleic acid molecule comprising the nucleotide sequence of SEQ ID NO:1 or SEQ ID NO:3. In other embodiment, the nucleic acid is at least 30, 50, 100, 150, 200, 250, 300, 350, 400, 450, 500, 550, or 600 nucleotides in length. As used herein, the term "hybridizes under stringent conditions" is intended to describe conditions for hybridization and washing under which nucleotide sequences at least 30%, 40%, 50%, or 60% homologous to each other typically remain hybridized to each other. Preferably, the conditions are such that sequences at least about 70%, more preferably at least about 80%, even more preferably at least about 85% or 90% homologous to each other typically remain hybridized to each other. Such stringent conditions are known to those skilled in the art and can be found in *Current Protocols in Molecular Biology,* John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1 % SDS at 50-65°C. Preferably, an isolated nucleic acid molecule of the, invention that hybridizes under stringent conditions to the sequence of SEQ ID NO:1 or SEQ ID NO:3 corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally-occurring allelic variants of the 14911 sequences that may exist in the population, the skilled artisan will further appreciate that changes can be introduced by mutation into the nucleotide sequences of SEQ m NO:1 or SEQ ID NO:3, thereby leading to changes in the amino acid sequence of the encoded 14911 proteins, without altering the functional ability of the 14911 proteins. For example, nucleotide substitutions leading to amino acid substitutions at "non-essential" amino acid residues can be made in the sequence of SEQ ID NO: 1 or SEQ ID NO:3. A "non-essential" amino acid residue is a residue that can be altered from the wild-type sequence of 14911 (e.g., the sequence of SEQ ID NO:2) without altering the biological activity, whereas an "essential" amino acid residue is required for biological activity. For example, amino acid residues that are conserved among the 14911 proteins of the present invention, are predicted to be particularly unamenable to alteration. Furthermore, additional amino acid residues that are conserved between the 14911 proteins of the present invention and other 14911 family members are not likely to be amenable to alteration.

An isolated nucleic acid molecule encoding a 14911 protein homologous to the protein of SEQ ID NO:2 can be created by introducing one or more nucleotide substitutions, additions or deletions into the nucleotide sequence of SEQ ID NO: 1, respectively, such that one or more amino acid substitutions, additions or deletions are introduced into the encoded protein. Mutations can be introduced into SEQ ID NO: 1 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 14911 protein is preferably replaced with another amino acid residue from the same side chain family. Alternatively, in another embodiment, mutations can be introduced randomly along all or part of a 14911 coding sequence, such as by saturation mutagenesis, and the resultant mutants can be screened for 14911 biological activity to identify mutants that retain activity. Following mutagenesis of SEQ ID NO:1, the encoded protein can be expressed recombinantly and the activity of the protein can be determined.

In a preferred embodiment, a mutant 14911 protein can be assayed for the ability to: 1) regulate transmission of signals from cellular receptors, e.g., cell growth factor receptors; 2) control entry of cells into mitosis; 3) modulate cellular differentiation; 4) modulate cell death; or 5) regulate cytoskeleton function.

In addition to the nucleic acid molecules encoding 14911 proteins described above, another aspect of the invention pertains to isolated nucleic acid molecules which are antisense thereto. An "antisense" nucleic acid comprises a nucleotide sequence which is complementary to a "sense" nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire 14911 coding strand, or only to a portion thereof. In one embodiment, an antisense nucleic acid molecule is antisense to a "coding region" of the coding strand of a nucleotide sequence encoding 14911. The term "coding region" refers to the region of the nucleotide sequence comprising codons which are translated into amino acid residues (e.g., the coding region of human 14911 corresponds to SEQ ID NO:3). In another embodiment, the antisense nucleic acid molecule is antisense to a "noncoding region" of the coding strand of a nucleotide sequence encoding 14911. The term "noncoding region" refers to 5' and 3' sequences which flank the coding region that are not translated into amino acids (i.e., also referred to as 5' and 3' untranslated regions).

Given the coding strand sequences encoding 14911 disclosed herein (e.g., SEQ ID NO:3), antisense nucleic acids of the invention can be designed according to the rules of Watson and Crick base pairing. The antisense nucleic acid molecule can be complementary to the entire coding region of 14911 mRNA, but more preferably is an oligonucleotide which is antisense to only a portion of the coding or noncoding region of 14911 mRNA. For example, the antisense oligonucleotide can be complementary to the region surrounding the translation start site of 14911 mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a 14911 protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) FEBS *Lett.* 215:327-330).

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) *Nature* 334:585-591)) can be used to catalytically cleave 14911 mRNA transcripts to thereby inhibit translation of 14911 mRNA. A ribozyme having specificity for a 14911-encoding nucleic acid can be designed based upon the nucleotide sequence of a 14911 cDNA disclosed herein (i.e., SEQ ID NO: or SEQ ID NO:3). For example, a derivative of a *Tetrahymena* L-19 IVS RNA can be constructed in which the nucleotide sequence of the active site is complementary to the nucleotide sequence to be cleaved in a 14911-encoding mRNA. See, e.g., Cech *et al.* U.S. Patent No. 4,987,071; and Cech *et al.* U.S. Patent No. 5,116,742. Alternatively, 14911 mRNA can be used to select a catalytic RNA having a specific ribonuclease activity from a pool of RNA molecules. See, e.g., Bartel, D. and Szostak, J.W. (1993) *Science* 261:1411-1418.

Alternatively, 14911 gene expression can be inhibited by targeting nucleotide sequences complementary to the regulatory region of the 14911 (e.g., the 14911 promoter and/or enhancers) to form triple helical structures that prevent transcription of the 14911 gene in target cells. See generally, Helene, C. (1991) *Anticancer Drug Des.* 6(6):569-84; Helene, C. *et al.* (1992) *Ann. N.Y. Acad. Sci.* 660:27-36; and Maher, L.J. (1992) *Bioassays* 14(12):807-15.

In yet another embodiment, the 14911 nucleic acid molecules of the present invention can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. *et al.* (1996) *Bioorganic & Medicinal Chemistry* 4 (1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe *et al.* Proc. Natl. Acad. Sci. 93: 14670-675.

PNAs of 14911 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 14911 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B. (1996) *supra*)); or as probes or primers for DNA sequencing or hybridization (Hyrup B. *et al.* (1996) *supra;* Perry-O'Keefe *supra*)*.*

In another embodiment, PNAs of 14911 can be modified, (e.g., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of 14911 nucleic acid molecules can be generated which may combine the advantageous properties of PNA and DNA. Such chimeras allow DNA recognition enzymes, (e.g., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B. (1996) *supra).* The synthesis of PNA-DNA chimeras can be performed as described in Hyrup B. (1996) *supra* and Finn P.J. *et al.* (1996) *Nucleic Acids Res.* 24 (17): 3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag, *M. et al.* (1989) *Nucleic Acid Res.* 17: 5973-88). PNA monomers are then coupled in a stepwise manner to produce a chimeric molecule with a 5' PNA segment and a 3' DNA segment (Finn P.J. *et al.* (1996) *supra).* Alternatively, chimeric molecules can be synthesized with a 5' DNA segment and a 3' PNA segment (Peterser,.K.H. *et al.* (1975) *Bioorganic Med. Chem. Lett.* 5: 1119-11124).

In other embodiments, the oligonucleotide may include other appended groups such as peptides (e.g., for targeting host cell receptors *in vivo),* or agents facilitating transport across the cell membrane (see, e.g., Letsinger *et al.* (1989) *Proc. Natl. Acad. Sci. US*. 86:6553-6556; Lemaitre *et al.* (1987) *Proc. Natl. Acad. Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (See, e.g., Krol *et al.* (1988) *Bio-Techniques* 6:958-976) or intercalating agents. (See, e.g., Zon (1988) *Pharm. Res.* 5:539-549). To this end, the oligonucleotide may be conjugated to another molecule, (e.g., a peptide, hybridization triggered crosslinking agent, transport agent, or hybridization-triggered cleavage agent).

### II. Isolated 14911 Proteins and Anti-14911 Antibodies

One aspect of the invention pertains to isolated 14911 proteins, and biologically active portions thereof, as well as polypeptide fragments suitable for use as immunogens to raise anti-14911 antibodies. In one embodiment, native 14911 proteins can be isolated from cells or tissue sources by an appropriate purification scheme using standard protein purification techniques. In another embodiment, 14911 proteins are produced by recombinant DNA techniques. Alternative to recombinant expression, a 14911 protein or polypeptide can be synthesized chemically using standard peptide synthesis techniques.

An "isolated" or "purified" protein or biologically active portion thereof is substantially free of cellular material or other contaminating proteins from the cell or tissue source from which the 14911 protein is derived, or substantially free from chemical precursors or other chemicals when chemically synthesized. The language "substantially free of cellular material" includes preparations of 14911 protein in which the protein is separated from cellular components of the cells from which it is isolated or recombinantly produced. In one embodiment, the language "substantially free of cellular material" includes preparations of 14911 protein having less than about 30% (by dry weight) of non-14911 protein (also referred to herein as a "contaminating protein"), more preferably less than about 20% of non-14911 protein, still more preferably less than about 10% of non-14911 protein, and most preferably less than about 5% non-14911 protein. When the 14911 protein or biologically active portion thereof is recombinantly produced, it is also preferably substantially free of culture medium, i.e., culture medium represents less than about 20%, more preferably less than about 10%, and most preferably less than about 5% of the volume of the protein preparation.

The language "substantially free of chemical precursors or other chemicals" includes preparations of 14911 protein in which the protein is separated from chemical precursors or other chemicals which are involved in the synthesis of the protein. In one embodiment, the language "substantially free of chemical precursors or other chemicals" includes preparations of 14911 protein having less than about 30% (by dry weight) of chemical precursors or non-14911chemicals, more preferably less than about 20% chemical precursors or non-14911 chemicals, still more preferably less than about 10% chemical precursors or non-14911chemicals, and most preferably less than about 5% chemical precursors or non-14911 chemicals.

Biologically active portions of a 14911 protein include peptides comprising amino acid sequences sufficiently homologous to or derived from the amino acid sequence of the 14911 protein, e.g., the amino acid sequence shown in SEQ ID NO:2, which include less amino acids than the full length 14911 proteins, and exhibit at least one activity of a 14911 protein. Typically, biologically active portions comprise a domain or motif with at least one activity of the 14911 protein. A biologically active portion of a 14911 protein can be a polypeptide which is, for example, at least 10, 25, 50, 100 or more amino acids in length.

In a preferred embodiment, the 14911 protein has an amino acid sequence shown in SEQ ID NO:2.

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence (e.g., when aligning a second sequence to the 14911, amino acid sequence of SEQ ID NO:2 having 396 amino acid residues, at least about 119, preferably at least 158, more preferably at least 198, even more preferably at least 238, and even more preferably at least 277, 317 or 356 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, *et al.* (1990) *J. Mol. Biol.* 215:403-10. BLAST nucleotide searches can be performed with the NBLAST program, score = 100, wordlength = 12 to obtain nucleotide sequences homologous to 14911 nucleic acid molecules of the invention. BLAST protein searches can be performed with the XBLAST program, score = 50, wordlength = 3 to obtain amino acid sequences homologous to 14911 protein molecules of the invention. To obtain gapped alignments for comparison purposes, Gapped BLAST can be utilized as described in Altschul *et al.,* (1997) *Nucleic Acids Res.* 25(17):3389-3402. When utilizing BLAST and Gapped BLAST programs, the default parameters of the respective programs (e.g., XBLAST and NBLAST) can be used. See http:/lwww.ncbi.nlm.nih.gov.

The invention also provides 14911 chimeric or fusion proteins. As used herein, a 1491 "chimeric protein" or "fusion protein" comprises a 14911 polypeptide operatively linked to a non-14911 polypeptide. An "14911 polypeptide" refers to a polypeptide having an amino acid sequence corresponding to 14911, whereas a "non-14911 polypeptide" refers to a polypeptide having an amino acid sequence corresponding to a protein which is not substantially homologous to the 14911 protein, e.g., a protein which is different from the 14911 protein and which is derived from the same or a different organism. Within a 14911 fusion protein the 14911 polypeptide can correspond to all or a portion of a 14911 protein. In a preferred embodiment, a 14911 fusion protein comprises at least one biologically active portion of a 14911 protein. In another preferred embodiment, a 14911 fusion protein comprises at least two biologically active portions of a 14911 protein. Within the fusion protein, the term "operatively linked" is intended to indicate that the 14911 polypeptide and the non-14911 polypeptide are fused in-frame to each other. The non-14911 polypeptide can be fused to the N-terminus or C-terminus of the 14911 polypeptide.

For example, in one embodiment, the fusion protein is a GST-14911 fusion protein in which the 14911 sequences are fused to the C-terminus of the GST sequences. Such fusion proteins can facilitate the purification of recombinant 14911.

In another embodiment, the fusion protein is a 14911 protein containing a heterologous signal sequence at its N-terminus. In certain host cells (e.g., mammalian host cells), expression and/or secretion of 14911 can be increased through use of a heterologous signal sequence.

The 14911 fusion proteins of the invention can be incorporated into pharmaceutical compositions and administered to a subject *in vivo.* The 14911 fusion proteins can be used to affect the bioavailability of a 14911 substrate. Use of 14911 fusion proteins may be useful therapeutically for the treatment of cellular growth related disorders, e.g., cardiovascular disorders. Moreover, the 14911-fusion proteins of the invention can be used as immunogens to produce anti-14911 antibodies in a subject, to purify 14911 ligands and in screening assays to identify molecules which inhibit the interaction of 14911 with a 14911 substrate.

Preferably, a 14911 chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al.* John Wiley & Sons: 1992). Moreover, many expression vectors are commercially available that already encode a fusion moiety (e.g., a GST polypeptide). A 14911-encoding nucleic acid can be cloned into such an expression vector such that the fusion moiety is linked in-frame to the 14911 protein.

The present invention also pertains to variants of the 14911 proteins which function as either 14911 agonists (mimetics) or as 14911 antagonists. Variants of the 14911 proteins can be generated by mutagenesis, e.g., discrete point mutation or truncation of a 14911 protein. An agonist of the 14911 proteins can retain substantially the same, or a subset, of the biological activities of the naturally occurring form of a 14911 protein. An antagonist of a 14911 protein can inhibit one or more of the activities of the naturally occurring form of the 14911 protein by, for example, competitively modulating a ' cardiovascular system activity of a 14911 protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the 14911 protein.

In one embodiment, variants of a 14911 protein which function as either 14911 agonists (mimetics) or as 14911 antagonists respectively can be identified by screening combinatorial libraries of mutants, e.g., truncation mutants, of a 14911 protein for 14911 protein agonist or antagonist activity. In one embodiment, a variegated library of 14911 variants is generated by combinatorial mutagenesis at the nucleic acid level and is encoded by a variegated gene library. A variegated library of 14911 variants can be produced by, for example, erizymatically ligating a mixture of synthetic oligonucleotides into gene sequences such that a degenerate set of potential 14911 sequences is expressible as individual polypeptides, or alternatively, as a set of larger fusion proteins (e.g., for phage display) containing the set of 14911 sequences therein. There are a variety of methods which can be used to produce libraries of potential 14911 variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential 14911 sequences. Methods for synthesizing degenerate oligonucleotides are known in the art (see, e.g., Narang, S.A. (1983) *Tetrahedron* 39:3; Itakura *et al.* (1984) *Annu. Rev. Biochem.* 53:323; *Itakura et al.* (1984) *Science* 198:1056; Ike *et al.* (1983) *Nucleic Acid Res.* 11:477.

In addition, libraries of fragments of a 14911 protein coding sequence can be used to generate a variegated population of 14911 fragments respectively for screening and subsequent selection of variants of a 14911 protein. In one embodiment, a library of coding sequence fragments can be generated by treating a double stranded PCR fragment of a 14911 coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the 14911 protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of 14911 proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique which enhances the frequency of functional mutants in the libraries, can be used in combination with the screening assays to identify 14911 variants (Arkin and Yourvan (1992) *Proc. Natl. Acad. Sci. USA 89*:7811-7815; *Delgrave et al.* (1993) *Protein Engineering* 6(3):327-331).

In one embodiment, cell based assays can be exploited to analyze a variegated 14911 library. For example, a library of expression vectors can be transfected into a cell line which ordinarily synthesizes and secretes 14911. The transfected cells are then cultured such that 14911 and a particular mutant 14911 are secreted and the effect of expression of the mutant on 14911 activity in cell supernatants can be detected, e.g., by any of a number of enzymatic assays. Plasmid DNA can then be recovered from the cells which score for inhibition, or alternatively, potentiation of 14911 activity, and the individual clones further characterized.

An isolated 14911 protein, or a portion or fragment thereof, can be used as an immunogen to generate antibodies that bind 14911 using standard techniques for polyclonal and monoclonal antibody preparation. A full-length 14911 protein can be used or, alternatively, the invention provides antigenic peptide fragments of 14911 for use as immunogens. The antigenic peptide of 14911 comprises at least 8 amino acid residues of the amino acid sequence shown in SEQ ID NO:2 and encompasses an epitope of 14911 such that an antibody raised against the peptide forms a specific immune complex with 14911. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of 14911 that are located on the surface of the protein, e.g., hydrophilic regions.

A 14911 immunogen typically is used to prepare antibodies by immunizing a suitable subject, (e.g., rabbit, goat, mouse or other mammal) with the immunogen. An appropriate immunogenic preparation can contain, for example, recombinantly expressed 14911 protein or a chemically synthesized 14911 polypeptide. The preparation can further include an adjuvant, such as Freund's complete or incomplete adjuvant, or similar immunostimulatory agent. Immunization of a suitable subject with an immunogenic 14911 preparation induces a polyclonal anti-14911 antibody response.

Accordingly, another aspect of the invention pertains to anti-14911 antibodies. The term "antibody" as used herein refers to immunoglobulin molecules and immunologically active portions of immunoglobulin molecules, i.e., molecules that contain an antigen binding site which specifically binds (immunoreacts with) an antigen, such as 14911. Examples of immunologically active portions of immunoglobulin molecules include F(ab) and F(ab')₂ fragments which can be generated by treating the antibody with an enzyme such as pepsin. The invention provides polyclonal and monoclonal antibodies that bind 14911. The term "monoclonal antibody" or "monoclonal antibody composition", as used herein, refers to a population of antibody molecules that contain only one species of an antigen binding site capable of immunoreacting with a particular epitope of 14911. A monoclonal antibody composition thus typically displays a single binding affinity for a particular 14911 protein with which it immunoreacts.

Polyclonal anti-14911 antibodies can be prepared as described above by immunizing a suitable subject with a 14911 immunogen. The anti-14911 antibody titer in the immunized subject can be monitored over time by standard techniques, such as with an enzyme linked immunosorbent assay (ELISA) using immobilized 14911. If desired, the antibody molecules directed against 14911 can be isolated from the mammal (e.g., from the blood) and further purified by well known techniques, such as protein A chromatography to obtain the IgG fraction. At an appropriate time after immunization, e.g., when the anti-14911 antibody titers are highest, antibody-producing cells can be obtained from the subject and used to prepare monoclonal antibodies by standard techniques, such as the hybridoma technique originally described by Kohler and Milstein (1975) *Nature* 256:495-497) (see also, Brown *et al.* (1981) *J. Immunol.* 127:539-46; Brown *et al.* (1980) *J. Biol. Chem* .255:4980-83; Yeh *et al.* (1976) *Proc. Natl. Acad. Sci. USA* 76:2927-31; and Yeh *et al.* (1982) *Int. J. Cancer* 29:269-75), the more recent human B cell hybridoma technique (Kozbor *et al.* (1983) *Immunol Today* 4:72), the EBV-hybridoma technique (Cole *et al.* (1985), *Monoclonal Antibodies and Cancer Therapy,* Alan R. Liss, Inc., pp. 77-96) or trioma techniques. The technology for producing monoclonal antibody hybridomas is well known (see generally R. H. Kenneth, in *Monoclonal Antibodies: A New Dimension In Biological Analyses,* Plenum Publishing Corp., New York, New York (1980); E. A. Lerner (1981) *Yale J. Biol.* Med., 54:387-402; M. L. Gefter *et al.* (1977) *Somatic* Cell Genet. 3:231-36). Briefly, an immortal cell line (typically a myeloma) is fused to lymphocytes (typically splenocytes) from a mammal immunized with a 14911 immunogen as described above, and the culture supernatants of the resulting hybridoma cells are screened to identify a hybridoma producing a monoclonal antibody that binds 14911.

Any of the many well known protocols used for fusing lymphocytes and immortalized cell lines can be applied for the purpose of generating an anti-14911monoclonal antibody (see, e.g., G. Galfre *et al.* (1977) *Nature* 266:55052; Gefter *et al. Somatic Cell Genet.,* cited *supra;* Lerner, *Yale J. Biol. Med.,* cited *supra;* Kenneth, *Monoclonal Antibodies,* cited *supra).* Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind 14911, e.g., using a standard ELISA assay.

Alternative to preparing monoclonal antibody-secreting hybridomas, a monoclonal anti-14911 antibody can be identified and isolated by screening a recombinant combinatorial immunoglobulin library (e.g., an antibody phage display library) with 14911 to thereby isolate immunoglobulin library members that bind 14911. Kits for generating and screening phage display libraries are commercially available (e.g., the Pharmacia *Recombinant Phage Antibody System,* Catalog No. 27-9400-01; and the Stratagene *SurfZAP*^{*™*} *Phage Display Kit,* Catalog No. 240612). Additionally, examples of methods and reagents particularly amenable for use in generating and screening antibody display library can be found in, for example, *Ladner et al.* U.S. Patent No. 5,223,409; Kang *et al.* PCT International Publication No. WO 92/18619; Dower *et al.* PCT International Publication No. WO 91/17271; Winter *et al.* PCT International Publication WO 92/20791; Markland *et al.* PCT International Publication No. WO 92115679; Breitling *et al.* PCT International Publication WO 93/01288; McCafferty *et al.* PCT International Publication No. WO 92/01047; Garrard *et al.* PCT International Publication No. WO 92/09690; Ladner *et al.* PCT International Publication No. WO 90/02809; Fuchs *et al.* (1991) *Bio*/*Technology* 9:1370-1372; Hay *et al.* (1992) *Hum. Antibod. Hybridomas* 3:81-85; Huse *et al.* (1989) *Science* 246:1275-1281; Griffiths *et al.* (1993) *EMBO J* 12:725-734; Hawkins *et al.* (1992) *J. Mol. Biol.* 226:889-896; Clarkson *et al.* (1991) *Nature* 352:624-628; Gram *et al.* (1992) *Proc. Natl. Acad. Sci. USA* 89:3576-3580; Garrad *et al.* (1991) *Bio*/*Technology* 9:13 73-1377; Hoogenboom *et al.* (1991) *Nuc. Acid Res.* 19:4133-4137; Barbas *et al.* (1991) *Proc. Natl. Acad. Sci. USA* 88:7978-7982; and McCafferty *et al. Nature* (1990) 348:552-554.

Additionally, recombinant anti-14911 antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, M., European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al.* European Patent Application 125,023; Better *et al.* (1988) *Science* 240:1041-1043; Liu *et al.* (1987) *Proc. Natl. Acad. Sci. USA* 84:3439-3443; Liu *et al.* (1987) *J. Immunol.* 139:3521-3526; Sun *et al.* (1987) *Proc. Natl. Acad. Sci. USA* 84:214-218; Nishimura *et al.* (1987) *Canc. Res.* 47:999-1005; *Wood et al.* (1985) *Nature* 314:446-449; and Shaw *et al.* (1988) *J. Natl. Cancer Inst.* 80:1553-1559); Morrison, S. L. (1985) *Science* 229:1202-1207; Oi *et al.* (1986) *BioTechniques* 4:214; Winter U.S. Patent 5,225,539; Jones *et al.* (1986) *Nature* 321:552-525; Verhoeyan *et al.* (1988) *Science* 239:1534; and Beidler *et al.* (1988) *J*. *Immunol.* 141:4053-4060.

An anti-14911 antibody (e.g., monoclonal antibody) can be used to isolate 14911 by standard techniques, such as affinity chromatography or immunoprecipitation. An anti-14911 antibody can facilitate the purification of natural 14911 from cells and of recombinantly produced 14911 expressed in host cells. Moreover, an anti-14911 antibody can be used to detect 14911 protein (e.g., in a cellular lysate or cell supernatant) in order to evaluate the abundance and pattern of expression of the 14911 protein. Anti-14911 antibodies can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, -galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### III. Recombinant Expression Vectors and Host Cells

Another aspect of the invention pertains to vectors, preferably expression vectors, containing a nucleic acid encoding a 14911 protein (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to includes promoters, enhancers and other expression control elements (e.g., polyadenylation signals). Such regulatory sequences are described, for example, in Goeddel; *Gene Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein (e.g., 14911 proteins, mutant forms of 14911 proteins, fusion proteins, and the like).

The recombinant expression vectors of the invention can be designed for expression of 14911 proteins in prokaryotic or eukaryotic cells. For example, 14911 proteins can be expressed in bacterial cells such as *E. coli,* insect cells (using baculovirus expression vectors) yeast cells or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in 14911 activity assays, (e.g., direct assays or competitive assays described in detail below), or to generate antibodies specific for 14911 proteins, for example. In a preferred embodiment, a 14911 fusion protein expressed in a retroviral expression vector of the present invention can be utilized to infect bone marrow cells which are subsequently transplanted into irradiated recipients. The pathology of the subject recipient is then examined after sufficient time has passed (e.g., six (6) weeks).

Examples of suitable inducible non-fusion *E. coli* expression vectors include pTrc (Amann *et al.,* (1988) *Gene* 69:301-315) and pET 11d (Shidier *et al., Gene Expression Technology: Methods in Enzymology* 185, Academic Press, San Diego, California (1990) 60-89). Target gene expression from the pTrc vector relies on host RNA polymerase transcription from a hybrid trp-1ac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21 (DE3) or HMS 174(DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, S., *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada *et al.,* (1992) *Nucleic Acids Res.* 20:2111-2118). Such alteration of nucleic acid sequences of the invention can be carried out by standard DNA synthesis techniques.

In another embodiment, the 14911 expression vector is a yeast expression vector. Examples of vectors for expression in yeast *S*. *cerevisiae* include pYepSec1 (Baldari, *et al.,* (1987) *Embo J.* 6:229-234), pMFa (Kurjan and Herskowitz, (1982) *Cell* 30:933-943), pJRY88 (Schultz *et al.,* (1987) *Gene* 54:113-123), pYES2 (Invitrogen Corporation, San Diego, CA), and picZ (In Vitrogen Corp, San Diego, CA).

Alternatively, 14911 proteins can be expressed in insect cells using baculovirus expression vectors. Baculovirus vectors available for expression of proteins in cultured insect cells (e.g., Sf 9 cells) include the pAc series (Smith *et al.* (1983) *Mol. Cell Biol.* 3:2156-2165) and the pVL series (Lucklow and Summers (1989) *Virology* 170:31-39).

In yet another embodiment, a nucleic acid of the invention is expressed in mammalian cells using a mammalian expression vector. Examples of mammalian expression vectors include pCDM8 (Seed, B. (1987) *Nature* 329:840) and pMT2PC (Kaufinan *et al.* (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; *Pinkert et al.* (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J.* 8:729-733) and immunoglobulins (Banerji *et al.* (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund *et al.* (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to 14911 mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. *et al.,* Antisense RNA as a molecular tool for genetic analysis, *Reviews - Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 14911 protein can be expressed in bacterial cells such as *E*. *coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al. (Molecular Cloning: A Laboratory Manual. 2nd, ed., Cold Spring Harbor Laboratory,* Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding a 14911 protein or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (i.e., express) a 14911 protein. Accordingly, the invention further provides methods for producing a 14911 protein using the host cells of the invention. In one embodiment, the method comprises culturing the host cell of invention (into which a recombinant expression vector encoding a 14911 protein has been introduced) in a suitable medium such that a 14911 protein is produced. In another embodiment, the method further comprises isolating a 14911 protein from the medium or the host cell.

### IV. Pharmaceutical Compositions

The 14911 nucleic acid molecules, 14911 proteins, and anti-14911 antibodies (also referred to herein as "active compounds'') of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a 14911 protein or anti-14911 antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen *et al.* (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic). The isolated nucleic acid molecules of the invention can be used, for example, to express 14911 protein (e.g., via a recombinant expression vector in a host cell in gene therapy applications), to detect 14911 mRNA (e.g., in a biological sample) or a genetic alteration in a 14911 gene, and to modulate 14911 activity, as described further below. The 14911 proteins can be used to treat disorders characterized by insufficient or excessive production of a 14911 substrate or production of 14911 inhibitors. In addition, the 14911 proteins can be used to screen for naturally occurring 14911 substrates, to screen for drugs or compounds which modulate 14911 activity, as well as to treat disorders characterized by insufficient or excessive production of 14911 protein or production of 14911 protein forms which have decreased or aberrant activity compared to 14911 wild type protein. Moreover, the anti-14911 antibodies of the invention can be used to detect and isolate 14911 proteins, regulate the bioavailability of 14911 proteins, and modulate 14911 activity.

### A. Screening Assays:

The invention provides a method (also referred to herein as a "screening assay") for identifying modulators, i.e., candidate or test compounds or agents (e.g., peptides, peptidomimetics, small molecules or other drugs) which bind to 14911 proteins, have a stimulatory or inhibitory effect on, for example, 14911 expression or 14911 activity, or have a stimulatory or inhibitory effect on, for example, the expression or activity of a 14911 substrate.

In one embodiment, the invention provides assays for screening candidate or test compounds which are substrates of a 14911 protein or polypeptide or biologically active portion thereof. In another embodiment, the invention provides assays for screening candidate or test compounds which bind to or modulate the activity of a 14911 protein or polypeptide or biologically active portion thereof, e.g., modulate the ability of 14911 to interact with its cognate ligand. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non peptide oligomer or small molecule libraries of compounds (Lam, K.S. (1997) *Anticancer Drug Des.* 12:145).

Examples of methods for the synthesis of molecular libraries can be found in the art, for example in: DeWitt *et al.* (1993) *Proc. Natl. Acad. Sci. U.S.A.* 90:6909; *Erb et al.* (1994) *Proc. Natl. Acad. Sci. USA* 91:11422; Zuckermann *et al.* (1994). *J. Med. Chem.* 37:2678; Cho *et al.* (1993) *Science* 261:1303; Carrell *et al.* (1994) *Angew. Chem. Int. Ed. Engl.* 33:2059; *Carell et al.* (1994) *Angew. Chem. Int. Ed . Engl.* 33:2061; and in Gallop *et al.* (1994) *J. Med. Chem.* 37:1233.

Libraries of compounds may be presented in solution (e.g., Houghten (1992) *Biotechniques* 13:412-421), or on beads (Lam (1991) *Nature* 354:82-84), chips (Fodor (1993) *Nature* 364:555-556), bacteria (Ladner USP 5,223,409), spores (Ladner USP '409), plasmids (Cull *et al.* (1992) *Proc Natl Acad Sci USA* 89:1865-1869) or on phage (Scott and Smith (1990) *Science* 249:386-390); (Devlin (1990) *Science* 249:404-406); *(Cwirla et al.* (1990) *Proc. Natl. Acad. Sci.* 87:6378-6382); (Felici (1991) *J*. *Mol. Biol.* 222:301-310); (Ladner *supra.*)*.*

In another embodiment, an assay is a cell-based assay comprising contacting a cell expressing a 14911 target molecule (e.g., a 14911 phosphorylation substrate) with a test compound and determining the ability of the test compound to modulate (e.g. stimulate or inhibit) the activity of the 14911 target molecule. Determining the ability of the test compound to modulate the activity of a 14911 target molecule can be accomplished, for example, by determining the ability of the 14911 protein to bind to or interact with the 14911 target molecule, or by determining the ability of the 14911 protein to phosphorylate the 14911 target molecule.

The ability of the 14911 protein to phosphorylate a 14911 target molecule can be determined by, for example, an *in vitro* kinase assay. Briefly, a 14911 target molecule, e.g., an immunoprecipitated 14911 target molecule from a cell line expressing such a molecule, can be incubated with the 14911 protein and radioactive ATP, e.g., [γ⁻³²P] ATP, in a buffer containing MgCl₂ and MnCl₂, e.g., 10 mM MgCl₂ and 5 mM MmCl₂. Following the incubation, the immunoprecipitated 14911 target molecule can be separated by SDS-polyacrylamide gel electrophoresis under reducing conditions, transferred to a membrane, e.g., a PVDF membrane, and autoradiographed. The appearance of detectable bands on the autoradiograph indicates that the 14911 substrate has been phosphorylated. Phosphoaminoacid analysis of the phosphorylated substrate can also be performed in order to determine which residues on the 14911 substrate are phosphorylated. Briefly, the radiophosphorylated protein band can be excised from the SDS gel and subjected to partial acid hydrolysis. The products can then be separated by one-dimensional electrophoresis and analyzed on, for example, a phosphoimager and compared to ninhydrin-stained phosphoaminoacid standards.

Determining the ability of the 14911 protein to bind to or interact with a 14911 target molecule can be accomplished by determining direct binding. Determining the ability of the 14911 protein to bind to or interact with a 14911 target molecule can be accomplished, for example, by coupling the 14911 protein with a radioisotope or enzymatic label such that binding of the 14911 protein to a 14911 target molecule can be determined by detecting the labeled 14911 protein in a complex. For example, 14911 molecules, e.g., 14911 proteins, can be labeled with ¹²⁵I, ³⁵S, ¹⁴C, or ³H, either directly or indirectly, and the radioisotope detected by direct counting of radioemission or by scintillation counting. Alternatively, 14911 molecules can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound to modulate the interaction between 14911 and its target molecule, without the labeling of any of the interactants. For example, a microphysiometer can be used to detect the interaction of 14911 with its target molecule without the labeling of either 14911 or the target molecule. McConnell, H. M*. et al.* (1992) *Science* 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between compound and receptor.

In a preferred embodiment, determining the ability of the 14911 protein to bind to or interact with a 14911 target molecule can be accomplished by determining the activity of the target molecule. For example, the activity of the target molecule can be determined by detecting induction of a cellular second messenger of the target (e.g., intracellular Ca²⁺, diacylglycerol, IP₃, etc.), detecting catalytic/enzymatic activity of the target an appropriate substrate, detecting the induction of a reporter gene (comprising a target-responsive regulatory element operatively linked to a nucleic acid encoding a detectable marker, e.g., chloramphenicol acetyl transferase), or detecting a target-regulated cellular response.

In yet another embodiment, an assay of the present invention is a cell-free assay in which a 14911 protein or biologically active portion thereof is contacted with a test compound and the ability of the test compound to bind to the 14911 protein or biologically active portion thereof is determined. Binding of the test compound to the 14911 protein can be determined either directly or indirectly as described above. In a preferred embodiment, the assay includes contacting the 1491 protein or biologically active portion thereof with a known compound which binds 14911 to form an assay mixture, contacting the assay mixture with a test compound, and determining the ability of the test compound to interact with a 14911 protein, wherein determining the ability of the test compound to interact with a 14911 protein comprises determining the ability of the test compound to preferentially bind to 14911 or biologically active portion thereof as compared to the known compound.

In another embodiment, the assay is a cell-free assay in which a 14911 protein or biologically active portion thereof is contacted with a test compound and the ability of the test compound to modulate (e.g., stimulate or inhibit) the activity of the 14911 protein or biologically active portion thereof is determined. Determining the ability of the test compound to modulate the activity of a 14911 protein can be accomplished, for example, by determining the ability of the 14911 protein to bind to a 14911 target molecule by one of the methods described above for determining direct binding. Determining the ability of the 14911 protein to bind to a 14911 target molecule can also be accomplished using a technology such as real-time Biomolecular Interaction Analysis (BIA). Sjolander, S. and Urbaniczky, C. (1991) *Anal. Chenz.* 63:2338-2345 and *Szabo et al.* (1995) *Curr. Opin. Struct. Biol.* 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In an alternative embodiment, determining the ability of the test compound to modulate the activity of a 14911 protein can be accomplished by determining the ability of the 14911 protein to further modulate the activity of a 14911 target molecule (e.g., a 14911 mediated signal transduction pathway component). For example, the activity of the effector molecule on an appropriate target can be determined, or the binding of the effector to an appropriate target can be determined as previously described.

In yet another embodiment, the cell-free assay involves contacting a 14911 protein or biologically active portion thereof with a known compound which binds the 14911 protein to form an assay mixture, contacting the assay mixture with a test compound, and determining the ability of the test compound to interact with the 14911 protein, wherein determining the ability of the test compound to interact with the 14911 protein comprises determining the ability of the 14911 protein to preferentially bind to or modulate the activity of a 14911 target molecule.

The cell-free assays of the present invention are amenable to use of both soluble and/or membrane-bound forms of proteins (e.g., 14911 proteins or biologically active portions thereof, or receptors to which 14911 binds). In the case of cell-free assays in which a membrane-bound form a protein is used (e.g., a cell surface 14911 receptor) it may be desirable to utilize a solubilizing agent such that the membrane-bound form of the protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit® , Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either 14911 or its target molecule to facilitate separation of complexed from uncomplexed forms of one or both of the proteins, as well as to accommodate automation of the assay. Binding of a test compound to a 14911 protein, or interaction of a 14911 protein with a target molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase/ 14911 fusion proteins or glutathione-S-transferase/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein or 14911 protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of 14911 binding or activity determined using standard techniques.

Other techniques for immobilizing proteins on matrices can also be used in the screening assays of the invention. For example, either a 14911 protein or a 14911 target molecule can be immobilized utilizing conjugation of biotin and streptavidin. Biotinylated 14911 protein or target molecules can be prepared from biotin-NHS (N-hydroxysuccinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with 14911 protein or target molecules but which do not interfere with binding of the 14911 protein to its target molecule can be derivatized to the wells of the plate, and unbound target or 14911 protein trapped in the wells by antibody conjugation. Methods for detecting such complexes, in addition to those described above for the GST-immobilized complexes, include immunodetection of complexes using antibodies reactive with the 14911 protein or target molecule, as well as enzyme-linked assays which rely on detecting an enzymatic activity associated with the 14911 protein or target molecule.

In another embodiment, modulators of 14911 expression are identified in a method wherein a cell is contacted with a candidate compound and the expression of 14911 mRNA or protein in the cell is determined. The level of expression of 14911 mRNA or protein in the presence of the candidate compound is compared to the level of expression of 14911 mRNA or protein in the absence of the candidate compound. The candidate compound can then be identified as a modulator of 14911 expression based on this comparison. For example, when expression of 14911 mRNA or protein is greater (statistically significantly greater) in the presence of the candidate compound than in its absence, the candidate compound is identified as a stimulator of 14911 mRNA or protein expression. Alternatively, when expression of 14911 mRNA or protein is less (statistically significantly less) in the presence of the candidate compound than in its absence, the candidate compound is identified as an inhibitor of 14911 mRNA or protein expression. The level of 14911 mRNA or protein expression in the cells can be determined by methods described herein for detecting 14911 mRNA or protein.

In yet another aspect of the invention, the 14911 proteins can be used as "bait proteins" in a two-hybrid assay or three-hybrid assay (see, e.g., U.S. Patent No. 5,283,317; *Z*ervos *et al.* (1993) *Cell* 72:223-232; *Madura et al.* (1993) *J*. *Biol. Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with 14911 ("14911-binding proteins" or "14911-bp") and are involved in 14911 activity. Such 14911-binding proteins are also likely to be involved in the propagation of signals by the 14911 proteins or 14911 targets as, for example, downstream elements of a 14911-mediated signaling pathway. Alternatively, such 14911-binding proteins are likely to be 14911 inhibitors.

The two-hybrid system is based on the modular nature of most transcription factors, which consist of separable DNA-binding and activation domains. Briefly, the assay utilizes two different DNA constructs. In one construct, the gene that codes for a 14911 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a 14911-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 14911 protein.

This invention further pertains to novel agents identified by the above-described screening assays. Accordingly, it is within the scope of this invention to further use an agent identified as described herein in an appropriate animal model. For example, an agent identified as described herein (e.g., a 14911 modulating agent, an antisense 14911 nucleic acid molecule, a 14911-specific antibody, or a 14911-binding partner) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. This process is called chromosome mapping. Accordingly, portions or fragments of the 14911 nucleotide sequences, described herein, can be used to map the location of the 14911 genes on a chromosome. The mapping of the 14911 sequences to chromosomes is an important first step in correlating these sequences with genes associated with disease.

Briefly, 14911 genes can be mapped to chromosomes by preparing PCR primers (preferably 15-25 bp in length) from the 14911 nucleotide sequences. Computer analysis of the 14911 sequences can be used to predict primers that do not span more than one exon in the genomic DNA, thus complicating the amplification process. These primers can then be used for PCR screening of somatic cell hybrids containing individual human chromosomes. Only those hybrids containing the human gene corresponding to the 14911 sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but human cells can, the one human chromosome that contains the gene encoding the needed enzyme, will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. (D'Eustachio P*. et al.* (1983) *Science* 220:919-924). Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the 14911 nucleotide sequences to design oligonucleotide primers, sublocalization can be achieved with panels of fragments from specific chromosomes. Other mapping strategies which can similarly be used to map a 9o, 1p, or 1v sequence to its chromosome include *in situ* hybridization (described in Fan, Y. *et al.* (1990) *Proc. Natl. Acad. Sci. USA,* 87:6223-27), pre-screening with labeled flow-sorted chromosomes, and pre-selection by hybridization to chromosome specific cDNA libraries.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical such as colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma *et al.,* Human Chromosomes: A Manual of Basic Techniques (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. *et al.* (1987) *Nature,* 325:783-787.

Moreover, differences in the DNA sequences between individuals affected and unaffected with a disease associated with the 14911 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 2. Tissue Typing

The 14911 sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the 14911 nucleotide sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The 14911 nucleotide sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, can comfortably provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from 14911 nucleotide sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial 14911 Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 are particularly appropriate for this use as greater numbers of polymorphisms occur in the noncoding regions, making it easier to differentiate individuals using this technique. Examples of polynucleotide reagents include the 14911 nucleotide sequences or portions thereof, e.g., fragments derived from the noncoding regions of SEQ ID NO:1, having a length of at least 20 bases, preferably at least 30 bases.

The 14911 nucleotide sequences described herein can further be used to provide polynucleotide reagents, e.g., labeled or labelable probes which can be used in, for example, an *in situ* hybridization technique, to identify a specific tissue, e.g., brain tissue. This can be very useful in cases where a forensic pathologist is presented with a tissue of unknown origin. Panels of such 14911 probes can be used to identify tissue by species and/or by organ type.

In a similar fashion, these reagents, e.g., 14911 primers or probes can be used to screen tissue culture for contamination (i.e. screen for the presence of a mixture of different types of cells in a culture).

### C. Predictive Medicine:

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining 14911 protein and/or nucleic acid expression as well as 14911 activity, in the context of a biological sample (e.g., blood, serum, cells, tissue) to thereby determine whether an individual is afflicted with a disease or disorder, or is at risk of developing a disorder, associated with aberrant 14911 expression or activity. The invention also provides for prognostic (or predictive) assays for determining whether an individual is at risk of developing a disorder associated with 14911 protein, nucleic acid expression or activity. For example, mutations in a 14911 gene can be assayed in a biological sample. Such assays can be used for prognostic or predictive purpose to thereby prophylactically treat an individual prior to the onset of a disorder characterized by or associated with 14911 protein, nucleic acid expression or activity.

Another aspect of the invention pertains to monitoring the influence of agents (e.g., drugs, compounds) on the expression or activity of 14911 in clinical trials.

These and other agents are described in further detail in the following sections.

### 1. Diagnostic Assays

An exemplary method for detecting the presence or absence of 14911 protein or nucleic acid in a biological sample involves obtaining a biological sample from a test subject and contacting the biological sample with a compound or an agent capable of detecting 14911 protein or nucleic acid (e.g., mRNA, genomic DNA) that encodes 14911 protein such that the presence of 14911 protein or nucleic acid is detected in the biological sample. A preferred agent for detecting 14911 mRNA or genomic DNA is a labeled nucleic acid probe capable of hybridizing to 14911 mRNA or genomic DNA. The nucleic acid probe can be, for example, a human 14911 nucleic acid, such as the nucleic acid of SEQ ID NO:1, or a portion thereof, such as an oligonucleotide of at least 15, 30, 50, 100, 250 or 500 nucleotides in length and sufficient to specifically hybridize under stringent conditions to 14911 mRNA or genomic DNA. Other suitable probes for use in the diagnostic assays of the invention are described herein.

A preferred agent for detecting 14911 protein is an antibody capable of binding to 14911 protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect 14911 mRNA, protein, or genomic DNA in a biological sample *in vitro* as well as *in vivo.* For example, *in vitro* techniques for detection of 14911 mRNA include Northern hybridizations and *in situ* hybridizations. *In vitro* techniques for detection of 14911 protein include enzyme linked immunosorbent assays (ELISAs), Western blots, immunoprecipitations and immunofluorescence. *In vitro* techniques for detection of 14911 genomic DNA include Southern hybridizations. Furthermore, *in vivo* techniques for detection of 14911 protein include introducing into a subject a labeled anti-14911 antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting 14911 protein, mRNA, or genomic DNA, such that the presence of 14911 protein, mRNA or genomic DNA is detected in the biological sample, and comparing the presence of 14911 protein, mRNA or genomic DNA in the control sample with the presence of 14911 protein, mRNA or genomic DNA in the test sample.

The invention also encompasses kits for detecting the presence of 14911 in a biological sample. For example, the kit can comprise a labeled compound or agent capable of detecting 14911 protein or mRNA in a biological sample; means for determining the amount of 14911 in the sample; and means for comparing the amount of 14911 in the sample with a standard. The compound or agent can be packaged in a suitable container. The kit can further comprise instructions for using the kit to detect 14911 protein or nucleic acid.

### 2. Prognostic Assays

The diagnostic methods described herein can furthermore be utilized to identify subjects having or at risk of developing a disease or disorder associated with aberrant 14911 expression or activity. For example, the assays described herein, such as the preceding diagnostic assays or the following assays, can be utilized to identify a subject having or at risk of developing a disorder associated with 14911 protein, nucleic acid expression or activity. Thus, the present invention provides a method for identifying a disease or disorder associated with aberrant 14911 expression or activity in which a test sample is obtained from a subject and 14911 protein or nucleic acid (e.g., mRNA, genomic DNA) is detected, wherein the presence of 14911 protein or nucleic acid is diagnostic for a subject having or at risk of developing a disease or disorder associated with aberrant 14911 expression or activity. As used herein, a "test sample" refers to a biological sample obtained from a subject of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant 14911 expression or activity. Thus, the present invention provides methods for determining whether a subject can be effectively treated with an agent for a disorder associated with aberrant 14911 expression or activity in which a test sample is obtained and 14911 protein or nucleic acid expression or activity is detected (e.g., wherein the abundance of 14911 protein or nucleic acid expression or activity is diagnostic for a subject that can be administered the agent to treat a disorder associated with aberrant 14911 expression or activity).

The methods of the invention can also be used to detect genetic alterations in a 14911 gene, thereby determining if a subject with the altered gene is at risk for a disorder associated with the 14911 gene. In preferred embodiments, the methods include detecting, in a sample of cells from the subject, the presence or absence of a genetic alteration characterized by at least one of an alteration affecting the integrity of a gene encoding a 14911-protein, or the mis-expression of the 14911 gene. For example, such genetic alterations can be detected by ascertaining the existence of at least one of 1) a deletion of one or more nucleotides from a 14911 gene; 2) an addition of one or more nucleotides to a 14911 gene; 3) a substitution of one or more nucleotides of a 14911 gene, 4) a chromosomal rearrangement of a 14911 gene; 5) an alteration in the level of a messenger RNA transcript of a 14911 gene, 6) aberrant modification of a 14911 gene, such as of the methylation pattern of the genomic DNA, 7) the presence of a non-wild type splicing pattern of a messenger RNA transcript of a 14911 gene, 8) a non-wild type level of a 14911 protein, 9) allelic loss of a 14911 gene, and 10) inappropriate post-translational modification of a 14911 protein. As described herein, there are a large number of assay techniques known in the art which can be used for detecting alterations in a 14911 gene. A preferred biological sample is a tissue or serum sample isolated by conventional means from a subject.

In certain embodiments, detection of the alteration involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, e.g., Landegran *et al.* (1988) *Science* 241:1077-1080; and Nakazawa *et al.* (1994) *Proc. Natl. Acad. Sci. USA* 91:360-364), the latter of which can be particularly useful for detecting point mutations in the 14911 gene (see Abravaya *et al.* (1995) *Nucleic Acids Res* .23:675-682). This method can include the steps of collecting a sample of cells from a patient, isolating nucleic acid (e.g., genomic, mRNA or both) from the cells of the sample, contacting the nucleic acid sample with one or more primers which specifically hybridize to a 14911 gene under conditions such that hybridization and amplification of the 14911 gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. *et al.,* (1990) *Proc. Natl. Acad. Sci. USA* 87:1874-1878), transcriptional amplification system (Kwoh, D.Y. *et al.,* (1989) *Proc. Natl. Acad. Sci. USA* 86:1173-1177), Q-Beta Replicase (Lizardi, P.M. *et al.* (1988) *Bio-Technology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a 14911 gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 14911 can be identified by hybridizing a sample and control nucleic acids, e.g., DNA or RNA, to high density arrays containing hundreds or thousands of oligonucleotides probes (Cronin, M.T. *et al.* (1996) *Human Mutation* 7: 244-255; Kozal, M.J. *et al.* (1996) *Nature Medicine* 2: 753-759). For example, genetic mutations in 14911 can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 14911 gene and detect mutations by comparing the sequence of the sample 14911 with the corresponding wild-type (control) sequence. Examples of sequencing reactions include those based on techniques developed by Maxam and Gilbert ((1977) *Proc. Natl. Acad. Sci. USA* 74:560) or Sanger ((1977) *Proc. Natl. Acad. Sci. USA* 74:5463). It is also contemplated that any of a variety of automated sequencing procedures can be utilized when performing the diagnostic assays ((1995) *Biotechniques* 19:448), including sequencing by mass spectrometry (see, e.g., PCT International Publication No. WO 94/16101; Cohen *et al.* (1996) *Adv. Chromatogr.* 36:127-162; and Griffin *et al.* (1993) *Appl. Biochem. Biotechnol.* 38:147-159).

Other methods for detecting mutations in the 14911 gene include methods in which protection from cleavage agents is used to detect mismatched bases in RNA/RNA or RNA/DNA heteroduplexes (Myers *et al.* (1985) *Science* 230:1242). In general, the art technique of "mismatch cleavage" starts by providing heteroduplexes formed by hybridizing (labeled) RNA or DNA containing the wild-type 14911 sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S 1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton *et al.* (1988) *Proc. Natl Acad Sci USA* 85:4397; Saleeba *et al.* (1992) *Methods Enzymol.* 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in 14911 cDNAs obtained from samples of cells. For example, the mutY enzyme of *E*. *coli* cleaves A at G/A mismatches and the thymidine DNA glycosylase from HeLa cells cleaves T at G/T mismatches (*Hsu et al.* (1994) *Carcinogenesis* 15:1657-1662). According to an exemplary embodiment, a probe based on a 14911 sequence, e.g., a wild-type 14911 sequence, is hybridized to a cDNA or other DNA product from a test-cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in 14911 genes. For example, single strand conformation polymorphism (SSCP) may be used to detect differences in electrophoretic mobility between mutant and wild type nucleic acids (Orita *et al.* (1989) *Proc Natl. Acad. Sci USA:* 86:2766, see also Cotton (1993) *Mutat Res* 285:125-144; and Hayashi (1992) *Genet Anal Tech Appl* 9:73-79): Single-stranded DNA fragments of sample and control 14911 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen *et al.* (1991) *Trends Genet* 7:5).

In yet another embodiment the movement of mutant or wild-type fragments in polyacrylamide gels containing a gradient of denaturant is assayed using denaturing gradient gel electrophoresis (DGGE) *(Myers et al.* (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki *et al.* (1986) *Nature* 324:163); Saiki *et al.* (1989) *Proc. Natl Acad. Sci USA* 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs *et al.* (1989) *Nucleic Acids Res.* 17:2437-2448) or at the extreme 3' end of one primer where, under appropriate conditions, mismatch can prevent, or reduce polymerase extension (Prossner *et al.* (1993) *Tibtech* 11:238). In addition it may be desirable to introduce a novel restriction site in the region of the mutation to create cleavage-based detection (Gasparini *et al.* (1992) *Mol. Cell Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, e.g., in clinical settings to diagnose patients exhibiting symptoms or family history of a disease or illness involving a 14911 gene.

Furthermore, any cell type or tissue in which 14911 is expressed may be utilized in the prognostic assays described herein.

### 3. Monitoring of Effects During Clinical Trials

Monitoring the influence of agents (e.g., drugs or compounds) on the expression or activity of a 14911 protein can be applied not only in basic drug screening, but also in clinical trials. For example, the effectiveness of an agent determined by a screening assay as described herein to increase 14911 gene expression, protein levels, or upregulate 14911 activity, can be monitored in clinical trials of subjects exhibiting decreased 14911 gene expression, protein levels, or downregulated 14911 activity. Alternatively, the effectiveness of an agent determined by a screening assay to decrease 14911 gene expression, protein levels, or downregulate 14911 activity, can be monitored in clinical trials of subjects exhibiting increased 14911 gene expression, protein levels, or upregulated 14911 activity. In such clinical trials, the expression or activity of a 14911 gene, and preferably, other genes that have been implicated in a disorder can be used as a "read out" or markers of the phenotype of a particular cell.

For example, and not by way of limitation, genes, including 14911, that are modulated in cells by treatment with an agent (e.g., compound, drug or small molecule) which modulates 14911 activity (e.g., identified in a screening assay as described herein) can be identified. Thus, to study the effect of agents on a 14911 associated disorder, for example, in a clinical trial, cells can be isolated and RNA prepared and analyzed for the levels of expression of 14911 and other genes implicated in the 14911 associated disorder, respectively. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of 14911 or other genes. In this way, the gene expression pattern can serve as a marker, indicative of the physiological response of the cells to the agent. Accordingly, this response state may be determined before, and at various points during treatment of the individual with the agent.

In a preferred embodiment, the present invention provides a method for monitoring the effectiveness of treatment of a subject with an agent (e.g., an agonist, antagonist; peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of expression of a 14911 protein, mRNA, or genomic DNA in the pre-administration sample; (iii) obtaining one or more post-administration samples from the subject; (iv) detecting the level of expression or activity of the 14911 protein, mRNA, or genomic DNA in the post-administration samples; (v) comparing the level of expression or activity of the 14911 protein, mRNA, or genomic DNA in the pre-administration sample with the 14911 protein, mRNA, or genomic DNA in the post administration sample or samples; and (vi) altering the administration of the agent to the subject accordingly. For example, increased administration of the agent may be desirable to increase the expression or activity of 14911 to higher levels than detected, i.e., to increase the effectiveness of the agent. Alternatively, decreased administration of the agent may be desirable to decrease expression or activity of 14911 to lower levels than detected, i.e. to decrease the effectiveness of the agent. According to such an embodiment, 14911 expression or activity may be used as an indicator of the effectiveness of an agent, even in the absence of an observable phenotypic response.

### 4. Use of 14911 Molecules as Surrogate Markers

The 14911 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 14911 molecules of the invention may be detected, and may be correlated with one or more biological states *in vivo.* For example, the 14911 molecules of the invention may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (*e.g*., with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (*e.g.*, early stage tumors), or when an assessment of disease progression is desired before a potentially dangerous clinical endpoint is reached (*e.g*., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen *et al.* (2000) *J. Mass. Spectrom.* 35: 258-264; and James (1994) *AIDS Treatment News Archive* 209.

The 14911 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug *in vivo.* Pharmacodynamic markers are of particular use in increasing the sensitivity of detection of drug effects, particularly when the drug is administered in low doses. Since even a small amount of a drug may be sufficient to activate multiple rounds of marker (e.g., a 14911 marker) transcription or expression, the amplified marker may be in a quantity which is more readily detectable than the drug itself. Also, the marker may be more easily detected due to the nature of the marker itself; for example, using the methods described herein, anti-14911 antibodies may be employed in an immune-based detection system for a 14911 protein marker, or 14911-specific radiolabeled probes may be used to detect a 14911 mRNA marker. Furthermore, the use of a pharmacodynamic marker may offer mechanism-based prediction of risk due to drug treatment beyond the range of possible direct observations. Examples of the use of pharmacodynamic markers in the art include: Matsuda *et al.* US 6,033,862; Hattis *et al.* (1991) *Env. Health Perspect.* 90: 229-238; Schentag (1999) *Am. J. Health-Syst. Pharm.* 56 Suppl. 3: S21-S24; and Nicolau (1999) *Am, J. Health-Svst. Pharm.* 56 Suppl. 3: S16-S20.

The 14911 molecules of the invention are also useful as pharmacogenomic markers. As used herein, a "pharmacogenomic marker" is an objective biochemical marker which correlates with a specific clinical drug response or susceptibility in a subject (see, e.g., McLeod *et al.* (1999) *Eur. J. Cancer* 35(12): 1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (*e.g*., 14911 protein or RNA) for specific tumor markers in a subject, a drug or course of treatment may be selected that is optimized for the treatment of the specific tumor likely to be present in the subject. Similarly, the presence or absence of a specific sequence mutation in 14911 DNA may correlate 14911 drug response. The use of pharmacogenomic markers therefore permits the application of the most appropriate treatment for each subject without having to administer the therapy.

### C. Methods of Treatment:

The present invention provides for both prophylactic and therapeutic methods of treating a subject at risk of (or susceptible to) a disorder or having a disorder associated with aberrant 14911 expression or activity. With regards to both prophylactic and therapeutic methods of treatment, such treatments may be specifically tailored or modified, based on knowledge obtained from the field of pharmacogenomics. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 14911 molecules of the present invention or 14911 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

Thus, the 14911 molecules can act as novel diagnostic targets and therapeutic agents for controlling one or more disorders. Examples of such disorders, e.g., kinase-associated or other 14911-associated disorders, include but are not limited to, cellular proliferative and/or differentiative disorders, disorders associated with bone metabolism, immune e.g., inflammatory, disorders, cardiovascular disorders, including endothelial cell disorders, liver disorders, viral diseases, pain or metabolic disorders.

Aberrant expression and/or activity of 14911 molecules can mediate disorders associated with bone metabolism. "Bone metabolism" refers to direct or indirect effects in the formation or degeneration of bone structures, e.g., bone formation, bone resorption, etc., which can ultimately affect the concentrations in serum of calcium and phosphate. This term also includes activities mediated by 14911 molecules effects in bone cells, e.g. osteoclasts and osteoblasts, that can in turn result in bone formation and degeneration. For example, 14911 molecules can support different activities of bone resorbing osteoclasts such as the stimulation of differentiation of monocytes and mononuclear phagocytes into osteoclasts. Accordingly, 14911 molecules that modulate the production of bone cells can influence bone formation and degeneration, and thus can be used to treat bone disorders. Examples of such disorders include, but are not limited to, osteoporosis, osteodystrophy, osteomalacia, rickets, osteitis fibrosa cystica, renal osteodystrophy, osteosclerosis, anticonvulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.

The 14911 nucleic acid and protein of the invention can be used to treat and/or diagnose a variety of immune, e.g., inflammatory, (e.g. respiratory inflammatory) disorders. Examples of immune disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, inflammatory bowel disease, e.g. Crohn's disease and ulcerative colitis, aphthous ulcer, iritis, conjunctivitis, keratoconjunctivitis, asthma, allergic asthma, chronic obstructive pulmonary disease, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

Examples of disorders involving the heart or "cardiovascular disorder" include, but are not limited to, a disease, disorder, or state involving the cardiovascular system, e.g., the heart, the blood vessels, and/or the blood. A cardiovascular disorder can be caused by an imbalance in arterial pressure, a malfunction of the heart, or an occlusion of a blood vessel, e.g., by a thrombus. Examples of cardiovascular disorders include but are not limited to, hypertension, atherosclerosis, coronary artery spasm, coronary artery disease, arrhythmias, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, disorders involving cardiac transplantation, and congestive heart failure.

A cardiovasular disease or disorder also includes an endothelial cell disorder.

As used herein, an "endothelial cell disorder" includes a disorder characterized by aberrant, unregulated, or unwanted endothelial cell activity, e.g., proliferation, migration, angiogenesis, or vascularization; or aberrant expression of cell surface adhesion molecules or genes associated with angiogenesis, e.g., TIE-2, FLT and FLK. Endothelial cell disorders include tumorigenesis, tumor metastasis, psoriasis, diabetic retinopathy, endometriosis, Grave's disease, ischemic disease (e.g., atherosclerosis), and chronic inflammatory diseases (e.g., rheumatoid arthritis).

Disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (e.g., bacterial, viral and parasitic). For example, the methods can be used for the early detection of hepatic injury, such as portal hypertension or hepatic fibrosis. In addition, the methods can be employed to detect liver fibrosis attributed to inborn errors of metabolism, for example, fibrosis resulting from a storage disorder such as Gaucher's disease (lipid abnormalities) or a glycogen storage disease, A1-antitrypsin deficiency; a disorder mediating the accumulation (e.g., storage) of an exogenous substance, for example, hemochromatosis (iron-overload syndrome) and copper storage diseases (Wilson's disease), disorders resulting in the accumulation of a toxic metabolite (e.g., tyrosinemia, fructosemia and galactosemia) and peroxisomal disorders (e.g., Zellweger syndrome). Additionally, the methods described herein can be used for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Additionally, 14911 molecules can play an important role in the etiology of certain viral diseases, including but not limited to Hepatitis B, Hepatitis C and Herpes Simplex Virus (HSV). Modulators of 14911 activity could be used to control viral diseases. The modulators can be used in the treatment and/or diagnosis of viral infected tissue or virus-associated tissue fibrosis, especially liver and liver fibrosis. Also, 14911 modulators can be used in the treatment and/or diagnosis of virus-associated carcinoma, especially hepatocellular cancer.

Additionally, 14911 can play an important role in the regulation of metabolism or pain disorders. Diseases of metabolic imbalance include, but are not limited to, obesity, anorexia nervosa, cachexia, lipid disorders, and diabetes. Examples of pain disorders include, but are not limited to, pain response elicited during various forms of tissue injury, e.g., inflammation, infection, and ischemia, usually referred to as hyperalgesia (described in, for example, Fields, H.L. (1987) Pain, New York:McGraw-Hill); pain associated with musculoskeletal disorders, e.g., joint pain; tooth pain; headaches; pain associated with surgery; pain related to irritable bowel syndrome; or chest pain.

### 1. Prophylactic Methods

In one aspect, the invention provides a method for preventing in a subject, a disease or condition associated with an aberrant 14911 expression or activity, by administering to the subject a 14911 or an agent which modulates 14911 expression or at least one 14911 activity. Subjects at risk for a disease which is caused or contributed to by aberrant 14911 expression or activity can be identified by, for example, any or a combination of diagnostic or prognostic assays as described herein. Administration of a prophylactic agent can occur prior to the manifestation of symptoms characteristic of the 14911 aberrancy, such that a disease or disorder is prevented or, alternatively, delayed in its progression. Depending on the type of 14911 aberrancy, for example, a 14911,14911 agonist or 14911 antagonist agent can be used for treating the subject. The appropriate agent can be determined based on screening assays described herein. '

### 2. Therapeutic Methods

Another aspect of the invention pertains to methods of modulating 14911 expression or activity for therapeutic purposes. Accordingly, in an exemplary embodiment, the modulatory method of the invention involves contacting a cell with a 14911 or agent that modulates one or more of the activities of 14911 protein activity associated with the cell. An agent that modulates 14911 protein activity can be an agent as described herein, such as a nucleic acid or a protein, a naturally-occurring target molecule of a 14911 protein (e.g., a 14911 1 phosphorylation substrate), a 14911 antibody, a 14911 agonist or antagonist, a peptidomimetic of a 14911 agonist or antagonist, or other small molecule. In one embodiment, the agent stimulates one or more 14911 activities. Examples of such stimulatory agents include active 14911 protein and a nucleic acid molecule encoding 14911 that has been introduced into the cell. In another embodiment, the agent inhibits one or more 14911 activities. Examples of such inhibitory agents include antisense 14911 nucleic acid molecules, anti-14911 antibodies, and 14911 inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder characterized by aberrant expression or activity of a 14911 protein or nucleic acid molecule. In one embodiment, the method involves administering an agent (e.g., an agent identified by a screening assay described herein), or combination of agents that modulates (e.g., upregulates or downregulates) 14911 expression or activity. In another embodiment, the method involves administering a 14911 protein or nucleic acid molecule as therapy to compensate for reduced or aberrant 14911 expression or activity.

Stimulation of 14911 activity is desirable in situations in which 14911 is abnormally downregulated and/or in which increased 14911 activity is likely to have a beneficial effect. For example, stimulation of 14911 activity is desirable in situations in which a 14911 is downregulated and/or in which increased 14911 activity is likely to have a beneficial effect. Likewise, inhibition of 14911 activity is desirable in situations in which 14911 is abnormally upregulated and/or in which decreased 14911 activity is likely to have a beneficial effect.

### 3. Pharmacogenomics

The 14911 molecules of the present invention, as well as agents, or modulators which have a stimulatory or inhibitory effect on 14911 activity (e.g., 14911 gene expression) as identified by a screening assay described herein can be administered to individuals to treat (prophylactically or therapeutically) disorders (e.g., cardiovascular disorders such as congestive heart failure) associated with aberrant 14911 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 14911 molecule or 14911 modulator as well as tailoring the dosage and/or therapeutic regimen of treatment with a 14911 molecule or 14911 modulator.

Pharmacogenomics deals with clinically significant hereditary variations in the response to drugs due to altered drug disposition and abnormal action in affected persons. See, for example, Eichelbaum, M. *et al.* (1996) *Clin. Exp. Pharmacol. Physiol.* 23(10-11):983-985 and Linder, M.W. *et al.* (1997) *Clin. Chem.* 43(2):254-266. In general, two types of phamlacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict a drug response. According to this method, if a gene that encodes a drug target is known (e.g., a 14911 protein or 14911 receptor of the present invention), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Alternatively, a method termed the "gene expression profiling", can be utilized to identify genes that predict drug response. For example, the gene expression of an animal dosed with a drug (e.g., a 14911 molecule or 14911 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment an individual. This knowledge, when applied to dosing or drug selection, can avoid adverse reactions or therapeutic failure and thus enhance therapeutic or prophylactic efficiency when treating a subject with a 14911 molecule or 14911 modulator, such as a modulator identified by one of the exemplary screening assays described herein.

This invention is further illustrated by the following examples which should not be construed as limiting. The contents of all references, patents and published patent applications cited throughout this application are incorporated herein by reference.

### EXAMPLES

### EXAMPLE 1 EXPRESSION AND TISSUE DISTRIBUTION OF 14911 mRNA

TaqMan real-time quantitative RT-PCR was used to detect the presence of RNA transcript corresponding to human 14911 in several tissues. It was found that the corresponding orthologs of 14911 are expressed in a variety of tissues or cell lines. The results of this screening are shown in Figures 6-14.

Reverse Transcriptase PCR (RT-PCR) was used to detect the presence of RNA transcript corresponding to human 14911 in RNA prepared from tumor and normal tissues. Relative expression levels of the 14911 was assessed in brain and lung cells using TaqMan PCR and increased expression was found in 2/5 lung tumor cell lines in comparison to a normal human bronchial epithelium (NHBE) control; 5/8 lung tumor samples in comparison to normal lung tissues; and 2/4 lung tumor samples in comparison to a normal human bronchial epithelium (NHBE) control. The results of this comparison are shown in Figures 6-8 respectively. Figure 9 indicates some increased expression in DLD-1, NCIH125 and lung tumor tissues. Increased expression was also found in 3/3 glioma samples in comparison to normal brain tissues as shown in Figure 10. Further, increased expression was found in 3/6 brain tumor samples in comparison to normal brain tissues as shown in Figure 12.

Downregulation of expression levels of 14911 in 7/8 breast tumors is show in Figure 11. Figure 12 also shows low expression in breast tumor.

Increased expression was also found in 2/3 colon metastases samples and 1/7 colon tumor samples in comparison to normal colon tissues, as shown in Figure 13.

Figure 14 illustrates the relative expression levels and tissue distribution of the 14911 gene in various tissues using TaqMan PCR.

Expression profiling results using *in situ* hybridization techniques have shown that 14911 mRNA has been detected in human colon, lung, brain and breast tumors. Positive expression of 14911 has been shown in 2/4 lung tumors in comparison with lack of expression, 0/2, in normal lung tissue samples. Further, 14911 has been shown to be expressed both in tumors and normal tissues, specifically in 1/4 colon tumors and 1/1 normal colon tissue samples; 1/2 breast tumors and 1/1 normal breast tissue samples; and 1/3 brain tumors and 2/2 normal brain tissue samples.

As seen by these results, 14911 molecules have been found to be overexpressed or underexpressed in some tumor cells, where the molecules may be inappropriately propagating either cell proliferation or cell survival signals. As such, 14911 molecules may serve as specific and novel identifiers of such tumor cells. Further, inhibitors of the 14911 molecules are also useful for the treatment of cancer, such as brain cancer, colon metastases and lung cancer and preferably lung cancer, and useful as a diagnostic. In addition, activators of the 14911 molecules are also useful for the treatment of cancer, preferably breast cancer, and useful as a diagnostic.

### EXAMPLE 2: EXPRESSION OF RECOMBINANT 14911 PROTEIN IN BACTERIAL CELLS

In this example, 14911 is expressed as a recombinant glutathione-S-transferase (GST) fusion polypeptide in *E*. *coli* and the fusion polypeptide is isolated and characterized. Specifically, 14911 is fused to GST and this fusion polypeptide is expressed in *E*. *coli,* e.g., strain PEB199. Expression of the GST-14911 fusion protein in PEB199 is induced with IPTG. The recombinant fusion polypeptide is purified from crude bacterial lysates of the induced PEB 199 strain by affinity chromatography on glutathione beads. Using polyacrylamide gel electrophoretic analysis of the polypeptide purified from the bacterial lysates, the molecular weight of the resultant fusion polypeptide is determined.

### EXAMPLE 3: EXPRESSION OF RECOMBINANT 14911 PROTEIN IN COS CELLS

To express the 14911 gene in COS cells, the pcDNA/Amp vector by Invitrogen Corporation (San Diego, CA) is used. This vector contains an SV40 origin of replication, an ampicillin resistance gene, an *E*. *coli* replication origin, a CMV promoter followed by a polylinker region, and an SV40 intron and polyadenylation site. A DNA fragment encoding the entire 14911 protein and an HA tag (Wilson *et al.* (1984) *Cell* 37:767) or a FLAG tag fused in-frame to its 3' end of the fragment is cloned into the polylinker region of the vector, thereby placing the expression of the recombinant protein under the control of the CMV promoter.

To construct the plasmid, the 14911 DNA sequence is amplified by PCR using two primers. The 5' primer contains the restriction site of interest followed by approximately twenty nucleotides of the 14911 coding sequence starting from the initiation codon; the 3' end sequence contains complementary sequences to the other restriction site of interest, a translation stop codon, the HA tag or FLAG tag and the last 20 nucleotides of the 14911 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 14911 gene is inserted in the correct orientation. The ligation mixture is transformed into *E. coli* cells (strains HB101, DH5□, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the 14911 -pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. *Molecular Cloning: A Laboratory Manual. 2nd, ed., Cold Spring Harbor Laboratory,* Cold Spring Harbor Laboratory Press, Cold Spring Harbor, NY, 1989. The expression of the VR-3 or VR-5 polypeptide is detected by radiolabelling (³⁵S-methionine or ³⁵S -cysteine available from NEN, Boston, MA, can be used) and immunoprecipitation (Harlow, E. and Lane, D. *Antibodies: A Laboratory Manual,* Cold Spring Harbor Laboratory Press, Cold Spring Harbor, NY, 1988) using an HA specific monoclonal antibody. Briefly, the cells are labelled for 8 hours with ³⁵S-methionine (or ³⁵S-cysteine). The culture media are then collected and the cells are lysed using detergents (RIPA buffer, 150 mM NaCl, 1% NP-40, 0.1 % SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 14911 coding sequence is cloned directly into the polylinker of the pCDNA/Amp vector using the appropriate restriction sites. The resulting plasmid is transfected into COS cells in the manner described above, and the expression of the 14911 polypeptide is detected by radiolabelling and immunoprecipitation using a 14911 specific monoclonal antibody.

## Claims

1. An isolated 14911 nucleic acid molecule selected from the group consisting of:
a) a nucleic acid molecule which encodes a polypeptide comprising the amino acid sequence of SEQ ID NO:2, or the amino acid sequence encoded by the cDNA insert of the plasmid deposited with the ATCC as Accession Number PTA-3435; and
b) a nucleic acid molecule consisting of the nucleotide sequence of SEQ ID NO:1, or a nucleic acid molecule comprising the nucleotide sequence of SEQ ID NO:3, or the nucleotide sequence of the DNA insert of the plasmid deposited with ATCC as Accession Number PTA-3435.

2. The isolated nucleic acid molecule of claim 1, which is the nucleotide sequence SEQ ID NO:1.

3. A host cell which contains the nucleic acid molecule of claim 1.

4. An isolated 14911 polypeptide comprising the amino acid sequence of SEQ ID NO:2.

5. A method for producing a polypeptide comprising the amino acid sequence of SEQ ID NO:2, or the amino acid sequence encoded by the cDNA insert of the plasmid deposited with the ATCC as Accession Number PTA-3435, comprising culturing the host cell of claim 3 under conditions in which the nucleic acid molecule is expressed.

6. An in vitro method for detecting the presence of a nucleic acid molecule of claim 1 or a polypeptide encoded by the nucleic acid molecule in a sample, comprising:
a) contacting the sample with a compound which selectively hybridizes to the nucleic acid molecule of claim 1 or binds to the polypeptide encoded by the nucleic acid molecule; and
b) determining whether the compound hybridizes to the nucleic acid or binds to the polypeptide in the sample.

7. A kit comprising a compound which selectively hybridizes to a nucleic acid molecule of claim 1 or an antibody which specifically binds to a polypeptide encoded by the nucleic acid molecule and instructions for use.

8. A method for identifying a compound which binds to a polypeptide or modulates the activity of the polypeptide of claim 4 comprising the steps of:
a) contacting a polypeptide, or a cell expressing a polypeptide of claim 4 with a test compound; and
b) determining whether the polypeptide binds to the test compound or determining the effect of the test compound on the activity of the polypeptide.

9. An in vitro method of identifying a nucleic acid associated with breast cancer comprising:
a) contacting a first sample from a subject having breast cancer or at risk of developing breast cancer comprising nucleic acid molecules with a hybridization probe comprising at least 25 contiguous nucleotides of SEQ ID NO:1, which specifically hybridizes to a nucleic acid molecule of claim 1 or claim 2;
b) detecting the presence of a nucleic acid molecule in the sample that specifically hybridizes to the probe;
c) contacting a second sample from a control comprising nucleic acid molecules with a hybridization probe comprising at least 25 contiguous nucleotides of SEQ ID NO:1, which specifically hybridizes to a nucleic acid molecule of claim 1 or claim 2;
d) detecting the presence of a nucleic acid molecule in the sample that specifically hybridizes to the probe;
e) comparing the level of nucleic acids which hybridise to the probe in the first sample with the level of nucleic acids which hybridize to the probe in the second sample,
wherein a decreased level of nucleic acids which hybridise in the first sample relative to the second sample indicates that the nucleic acid molecule in the first sample is associated with breast cancer.

10. An in vitro method of identifying a nucleic acid associated with lung, brain or colon cancer comprising:
a) contacting a first sample from a subject having lung, brain or colon cancer or at risk of developing lung, brain or colon cancer comprising nucleic acid molecules with a hybridization probe comprising at least 25 contiguous nucleotides of SEQ ID NO:1 which specifically hybridizes to a nucleic acid molecule of claim 1 or claim 2;
b) detecting the presence of a nucleic acid molecule in the sample that specifically hybridizes to the probe;
c) contacting a second sample from a control comprising nucleic acid molecules with a hybridization probe comprising at least 25 contiguous nucleotides of SEQ ID NO:1 which specifically hybridizes to a nucleic acid molecule of claim 1 or claim 2;
d) detecting the presence of a nucleic acid molecule in the sample that specifically hybridizes to the probe;
e) comparing the level of nucleic acids which hybridise to the probe in the first sample with the level of nucleic acids which hybridize to the probe in the second sample,
wherein an increased level of nucleic acids which hybridise in the first sample relative to the second sample indicates that the nucleic acid molecule in the first sample is associated with lung, brain or colon cancer.

11. An in vitro method of identifying a nucleic acid associated with breast cancer comprising:
a) contacting a first sample from a subject having breast cancer or at risk of developing breast cancer comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1, which specifically hybridizes to a nucleic acid molecule of claim 1 or claim 2;
b) incubating the sample under conditions that allow nucleic acid amplification;
c) contacting a second sample from a control comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1, which specifically hybridizes to a nucleic acid molecule of claim 1 or claim 2;
d) incubating the sample under conditions that allow nucleic acid amplification;
e) comparing the level of nucleic acid amplification in the first sample relative to the level of nucleic acid amplification in the second sample
wherein a decreased level of nucleic acid amplification in the first sample relative to the second sample indicates that the nucleic acid molecule is associated with breast cancer.

12. An in vitro method of identifying a nucleic acid associated with lung, brain or colon cancer comprising:
a) contacting a first sample from a subject having lung, brain or colon cancer or at risk of developing lung, brain or colon cancer comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1, which specifically hybridizes to a nucleic acid molecule of claim 1 or claim 2;
b) incubating the sample under conditions that allow nucleic acid amplification;
c) contacting a second sample from a control comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1, which specifically hybridizes to a nucleic acid molecule of claim 1 or claim 2;
d) incubating the sample under conditions that allow nucleic acid amplification;
e) comparing the level of nucleic acid amplification in the first sample relative to the level of nucleic acid amplification in the second sample
wherein a increased level of nucleic acid amplification in the first sample relative to the second sample indicates that the nucleic acid molecule is associated with lung, brain or colon cancer.

13. An in vitro method of identifying a polypeptide associated with breast cancer comprising:
a) contacting a first sample from a subject having breast cancer or at risk of developing breast cancer comprising polypeptides with an antibody which specifically binds polypeptide of claim 4;
b) detecting the presence of a polypeptide in the sample that binds to the antibody;
c) contacting a second sample from a control comprising polypeptides with an antibody which specifically binds polypeptide of claim 4;
d) detecting the presence of a polypeptide in the sample that binds to the, antibody;
e) comparing the level of antibody binding in the first sample relative to the level of antibody binding in the second sample,
wherein an decreased level of antibody binding in the first sample relative to the second sample indicates that the polypeptide is associated with breast cancer.

14. An in vitro method of identifying a polypeptide associated with lung, brain or colon cancer comprising:
a) contacting a first sample from a subject having lung, brain or colon cancer or at risk of developing lung, brain or colon cancer comprising polypeptides with an antibody which specifically binds polypeptide of claim 4;
b) detecting the presence of a polypeptide in the sample that binds to the antibody;
c) contacting a second sample from a control comprising polypeptides with an antibody which specifically binds polypeptide of claim 4;
d) detecting the presence of a polypeptide in the sample that binds to the antibody;
e) comparing the level of antibody binding in the first sample relative to the level of antibody binding in the second sample,
wherein a increased level of antibody binding in the first sample relative to the second sample indicates that the polypeptide is associated with lung, brain or colon cancer.

15. An in vitro method of identifying a subject having breast cancer or at risk of developing breast cancer comprising:
a) contacting a first sample from a subject having breast cancer or at risk of developing breast cancer comprising nucleic acid molecules with a hybridization probe comprising at least 25 contiguous nucleotides of SEQ ID NO:1, which specifically hybridizes to a nucleic acid molecule of claim 1 or claim 2;
b) detecting the presence of a nucleic acid molecule in the sample that specifically hybridizes to the probe;
c) contacting a second sample from a control comprising nucleic acid molecules with a hybridization probe comprising at least 25 contiguous nucleotides of SEQ ID NO:1, which specifically hybridizes to a nucleic acid molecule of claim 1 or claim 2 2;
d) detecting the presence of a nucleic acid molecule in the sample that specifically hybridizes to the probe;
e) comparing the level of nucleic acids which hybridise to the probe in the first sample with the level of nucleic acids which hybridize to the probe in the second sample,
wherein a decreased level of nucleic acids which hybridise in the first sample relative to the second sample identifies a subject having breast cancer or at risk of developing breast cancer.

16. An in vitro method of identifying a subject having lung, brain or colon cancer or at risk of developing lung, brain or colon cancer comprising:
a) contacting a first sample from a subject having lung, brain or colon cancer or at risk of developing lung, brain or colon cancer comprising nucleic acid molecules with a hybridization probe comprising at least 25 contiguous nucleotides of SEQ ID NO: 1, which specifically hybridizes to a nucleic acid molecule of claim 1 or claim 2;
b) detecting the presence of a nucleic acid molecule in the sample that specifically hybridizes to the probe;
c) contacting a second sample from a control comprising nucleic acid molecules with a hybridization probe comprising at least 25 contiguous nucleotides of SEQ ID NO:1, which specifically hybridizes to a nucleic acid molecule of claim 1 or claim 2;
d) detecting the presence of a nucleic acid molecule in the sample that specifically hybridizes to the probe;
e) comparing the level of nucleic acids which hybridise to the probe in the first sample with the level of nucleic acids which hybridize to the probe in the second sample,
wherein an increased level of nucleic acids which hybridise in the first sample relative to the second sample identifies a subject having lung, brain or colon cancer or at risk of developing lung, brain or colon cancer.

17. An in vitro method of identifying a subject having breast cancer or at risk for developing breast cancer comprising:
a) contacting a first sample from a subject having breast cancer or at risk for developing breast cancer comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1, which specifically hybridizes to a nucleic acid molecule of claim 1 or claim 2;
b) incubating the sample under conditions that allow nucleic acid amplification;
c) contacting a second sample from a control comprising nucleic acid molecules with a first and a second amplification primer, the first primer comprising at least 25 contiguous nucleotides of SEQ ID NO:1, which specifically hybridizes to a nucleic acid molecule of claim 1 or claim 2 and the second primer comprising at least 25 contiguous nucleotides from the complement of SEQ ID NO:1 which specifically hybridizes to a nucleic acid molecule of claim 1 or claim 2;
d) incubating the sample under conditions that allow nucleic acid amplification;
e) comparing the level of nucleic acid amplification in the first sample relative to the level of nucleic acid amplification in the second sample
wherein a decreased level of nucleic acid amplification in the first sample relative to the second sample identifies a subject having breast cancer or at risk of developing breast cancer.

18. An in vitro method of identifying a subject having lung, brain or colon cancer or at risk for developing lung, brain or colon cancer comprising:
a) contacting a first sample from a subject having lung, brain or colon cancer or at risk for developing lung, brain or colon cancer comprising nucleic acid molecules with a first and a second amplification primer, the first primer comprising at least 25 contiguous nucleotides of SEQ ID NO:1, which specifically hybridizes to a nucleic acid molecule of claim 1 or claim 2and the second primer comprising at least 25 contiguous nucleotides from the complement of SEQ ID NO:1, which specifically hybridizes to a nucleic acid molecule of claim 1 or claim 2;
b) incubating the sample under conditions that allow nucleic acid amplification;
c) contacting a second sample from a control comprising nucleic acid molecules with a first and a second amplification primer, the first primer comprising at least 25 contiguous nucleotides of SEQ ID NO:1 which specifically hybridizes to a nucleic acid molecule of claim 1 or claim 2 and the second primer comprising at least 25 contiguous nucleotides from the complement of SEQ ID NO:1 which specifically hybridizes to a nucleic acid molecule of claim 1 or claim 2;
d) incubating the sample under conditions that allow nucleic acid amplification;
e) comparing the level of nucleic acid amplification in the first sample relative to the level of nucleic acid amplification in the second sample
wherein an increased level of nucleic acid amplification in the first sample relative to the second sample identifies a subject having lung, brain or colon cancer or at risk of developing lung, brain or colon cancer.

19. An in vitro method of identifying a subject having breast cancer or at risk for developing breast cancer comprising:
a) contacting a first sample from a subject breast cancer or at risk of developing breast cancer comprising polypeptides with an antibody which specifically binds polypeptide of claim 4;
b) detecting the presence of a polypeptide in the sample that binds to the antibody;
c) contacting a second sample from a control comprising polypeptides with an antibody which specifically binds polypeptide of claim 4;
d) detecting the presence of a polypeptide in the sample that binds to the antibody;
e) comparing the level of antibody binding in the first sample relative to the level of antibody binding in the second sample,
wherein a decreased level of antibody binding in the first sample relative to the second sample indicates that the polypeptide is associated with breast cancer.

20. A in vitro method of identifying a subject having lung, brain or colon cancer or at risk for developing lung, brain or colon cancer comprising:
a) contacting a first sample from a subject having lung, brain or colon cancer or at risk of developing lung, brain or colon cancer comprising polypeptides with an antibody which specifically binds polypeptide of claim 4;
b) detecting the presence of a polypeptide in the sample that binds to the antibody;
c) contacting a second sample from a control comprising polypeptides with an antibody which specifically binds polypeptide of claim 4;
d) detecting the presence of a polypeptide in the sample that binds to the antibody;
e) comparing the level of antibody binding in the first sample relative to the level of antibody binding in the second sample,
wherein an increased level of antibody binding in the first sample relative to the second sample indicates that the polypeptide is associated with lung, brain or colon cancer.

21. A method for identifying an antibody capable of treating cancer or a cellular proliferation and/or differentiation disorder **characterized** activity of the polypeptide of claim 4, comprising assaying the ability of the antibody to modulate the activity of the polypeptide of claim 4, thereby identifying an antibody capable of treating cancer or a cellular proliferation and/or differentiation disorder **characterized by** aberrant activity of the polypeptide of claim 4.

22. A method for identifying an antisense nucleic acid or a ribozyme capable of treating cancer or a cellular proliferation and/or differentiation disorder **characterized by** aberrant expression of the nucleic acid of claim 1 or 2 comprising assaying the ability of the compound to modulate the expression of the nucleic acid of claim 1 or 2, thereby identifying a compound capable of treating cancer or a cellular proliferation and/or differentiation disorder **characterized by** aberrant expression of the nucleic acid of claim 1 or 2.

23. An in vitro method for evaluating the efficacy of a treatment of cancer or a cellular proliferation and/or differentiation disorder, in a sample obtained from a subject being treated with a protocol under evaluation, comprising assessing the expression level of a nucleic acid molecule defined in claim 1 or a polypeptide encoded by the nucleic acid molecule in the sample, wherein a change in the expression level of the nucleic acid or the polypeptide after the treatment, relative to the level before the treatment, is indicative of the efficacy of the treatment of cancer or a cellular proliferation and/or differentiation disorder.

24. A method according to claim 23, wherein the change in the expression level is an increase and the cancer is breast cancer.

25. A method according to claim 23, wherein the change in the expression level is a decrease and the cancer is lung, brain or colon cancer.

26. An in vitro method of diagnosing cancer or a cellular proliferation and/or differentiation disorder in a sample obtained from a subject, comprising:
evaluating the expression or activity of a nucleic acid molecule defined in claim 1 or a polypeptide encoded by the nucleic acid molecule in the sample, such that a difference in the level of the nucleic acid or the polypeptide relative to a normal subject or a cohort of normal subjects is indicative of cancer or a cellular proliferation and/or differentiation disorder.
27 A method according to claim 26, wherein the difference in the expression level is a decrease and the cancer is breast cancer.
28 A method according to claim 26, wherein the difference in the expression level is an increase and the cancer is lung, brain or colon cancer.

## Patentansprüche

1. Isoliertes Nukleinsäuremolekül 14911, ausgewählt aus der Gruppe bestehend aus:
a) einem Nukleinsäuremolekül, welches ein Polypeptid umfassend die Aminosäuresequenz von SEQ ID Nr.:2 oder die Aminosäuresequenz kodiert, die durch das cDNA-Insert des bei der ATCC unter der Zugangsnummer PTA-3435 hinterlegten Plasmids kodiert wird; und
b) einem Nukleinsäuremolekül bestehend aus der Nukleotidsequenz von SEQ ID Nr.: 1 oder einem Nukleinsäuremolekül umfassend die Nukleotidsequenz von SEQ ID Nr.:3 oder der Nukleotidsequenz des DNA-Inserts des bei der ATCC unter der Zugangsnummer PTA-3435 hinterlegten Plasmids.

2. Isoliertes Nukleinsäuremolekül nach Anspruch 1, welches die Nukleotidsequenz SEQ ID Nr.: ist.

3. Wirtszelle, welche das Nukleinsäuremolekül nach Anspruch 1 enthält.

4. Isoliertes 14911-Polypeptid umfassend die Aminosäuresequenz von SEQ ID Nr.:2.

5. Verfahren zum Herstellen eines Polypeptids umfassend die Aminosäuresequenz von SEQ ID Nr.:2 oder die Aminosäuresequenz, die durch das cDNA-Insert des bei der ATCC unter der Zugangsnummer PTA-3435 hinterlegten Plasmids kodiert wird, umfassend das Kultivieren der Wirtszelle nach Anspruch 3 unter Bedingungen, unter denen das Nukleinsäuremolekül exprimiert wird.

6. *In-vitro*-Verfahren zum Nachweis des Vorhandenseins eines Nukleinsäuremoleküls nach Anspruch 1 oder eines Polypeptids, das durch das Nukleinsäuremolekül kodiert wird, in einer Probe, umfassend:
a) In-Kontakt-Bringen der Probe mit einer Verbindung, die selektiv mit dem Nukleinsäuremolekül nach Anspruch 1 hybridisiert, oder die an das Polypeptid, das durch das Nukleinsäuremolekül kodiert wird, bindet; und
b) Bestimmen, ob die Verbindung in der Probe mit der Nukleinsäure hybridisiert oder an das Polypeptid bindet.

7. Kit umfassend eine Verbindung, die selektiv mit einem Nukleinsäuremolekül nach Anspruch 1 hybridisiert, oder einen Antikörper, der spezifisch an ein Polypeptid bindet, welches durch das Nukleinsäuremolekül kodiert wird, und Gebrauchsanweisungen.

8. Verfahren zur Identifizierung einer Verbindung, die an ein Polypeptid bindet oder die Aktivität des Polypeptids nach Anspruch 4 moduliert, umfassend die Schritte von:
a) In-Kontakt-Bringen eines Polypeptids oder einer Zelle, die ein Polypeptid nach Anspruch 4 exprimiert, mit einer Testverbindung; und
b) Bestimmen, ob das Polypeptid an die Testverbindung bindet oder Bestimmen der Wirkung der Testverbindung auf die Aktivität des Polypeptids.

9. *In-vitro*-Verfahren zur Identifizierung einer Nukleinsäure, die mit Brustkrebs assoziiert ist, umfassend:
a) In-Kontakt-Bringen einer ersten Probe eines Probanden mit Brustkrebs oder mit dem Risiko Brustkrebs zu entwickeln, welche Nukleinsäuremoleküle umfasst, mit einer Hybridisierungssonde, die wenigstens 25 zusammenhängende Nukleotide von SEQ ID Nr.: 1 umfasst und die spezifisch mit einem Nukleinsäuremolekül nach Anspruch 1 oder Anspruch 2 hybridisiert;
b) Nachweisen des Vorhandenseins eines Nukleinsäuremoleküls in der Probe, das spezifisch mit der Sonde hybridisiert;
c) In-Kontakt-Bringen einer zweiten Probe aus einer Kontrolle, die Nukleinsäuremoleküle umfasst, mit einer Hybridisierungssonde, die wenigstens 25 zusammenhängende Nukleotide von SEQ ID Nr.:1 umfasst und die spezifisch mit einem Nukleinsäuremolekül nach Anspruch 1 oder Anspruch 2 hybridisiert;
d) Nachweisen des Vorhandenseins eines Nukleinsäuremoleküls in der Probe, das spezifisch mit der Sonde hybridisiert;
e) Vergleichen der Menge der Nukleinsäuren, die in der ersten Probe mit der Sonde hybridisieren, mit der Menge der Nukleinsäuren, die in der zweiten Probe mit der Sonde hybridisieren,
wobei eine verringerte Menge an Nukleinsäuren, die in der ersten Probe hybridisieren, relativ zur zweiten Probe anzeigt, dass das Nukleinsäuremolekül in der ersten Probe mit Brustkrebs assoziiert ist.

10. *In*-*vitro*-Verfahren zur Identifizierung einer Nukleinsäure, die mit Lungenkrebs, Hirntumor oder Darmkrebs assoziiert ist, umfassend:
a) In-Kontakt-Bringen einer ersten Probe eines Probanden mit Lungenkrebs, Hirntumor oder Darmkrebs oder mit dem Risiko Lungenkrebs, Hirntumor oder Darmkrebs zu entwickeln, welche Nukleinsäuremoleküle umfasst, mit einer Hybridisierungssonde, die wenigstens 25 zusammenhängende Nukleotide von SEQ ID Nr.: 1 umfasst und die spezifisch mit einem Nukleinsäuremolekül nach Anspruch 1 oder Anspruch 2 hybridisiert;
b) Nachweisen des Vorhandenseins eines Nukleinsäuremoleküls in der Probe, die spezifisch mit der Sonde hybridisiert;
c) In-Kontakt-Bringen einer zweiten Probe aus einer Kontrolle, die Nukleinsäuremoleküle umfasst, mit einer Hybridisierungssonde, die wenigstens 25 zusammenhängende Nukleotide von SEQ ID Nr.: 1 umfasst und die spezifisch mit einem Nukleinsäuremolekül nach Anspruch 1 oder 2 hybridisiert;
d) Nachweisen des Vorhandenseins eines Nukleinsäuremoleküls in der Probe, das spezifisch mit der Sonde hybridisiert;
e) Vergleichen der Menge der Nukleinsäuren, die in der ersten Probe mit der Sonde hybridisieren, mit der Menge der Nukleinsäuren, die in der zweiten Probe mit der Sonde hybridisieren,
wobei eine erhöhte Menge an Nukleinsäuren, die in der ersten Probe hybridisieren, relativ zur zweiten Probe anzeigt, dass das Nukleinsäuremolekül in der ersten Probe mit Lungenkrebs, Hirntumor oder Darmkrebs assoziiert ist.

11. *In-vitro*-Verfahren zur Identifizierung einer Nukleinsäure, die mit Brustkrebs assoziiert ist, umfassend:
a) In-Kontakt-Bringen einer ersten Probe eines Probanden mit Brustkrebs oder mit dem Risiko Brustkrebs zu entwickeln, die Nukleinsäuremoleküle umfasst, mit einem ersten und einem zweiten Vervielfältigungsprimer, wobei der erste Primer wenigstens 25 zusammenhängende Nukleotide von SEQ ID Nr.: umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder 2 hybridisiert;
b) Inkubieren der Probe unter Bedingungen, die eine Nukleinsäure-Vervielfältigung erlauben;
c) In-Kontakt-Bringen einer zweiten Probe aus einer Kontrolle, die Nukleinsäuremoleküle umfasst, mit einem ersten und einem zweiten Vervielfältigungsprimer, wobei der erste Primer wenigstens 25 zusammenhängende Nukleotide von SEQ ID Nr.:1 umfasst und spezifisch mit einem Nukleinsäuremolekül nach Anspruch 1 oder 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert;
d) Inkubieren der Probe unter Bedingungen, die Nukleinsäure-Vervielfältigung erlauben;
e) Vergleichen der Menge an Nukleinsäure-Vervielfältigung in der ersten Probe im Vergleich zur Menge der Nukleinsäure-Vervielfältigung in der zweiten Probe,
wobei eine verringerte Menge der Nukleinsäure-Vervielfältigung in der ersten Probe relativ zur zweiten Probe anzeigt, dass das Nukleinsäuremolekül mit Brustkrebs assoziiert ist.

12. *In-vitro*-Verfahren zur Identifizierung einer Nukleinsäure, die mit Lungenkrebs, Hirntumor oder Darmkrebs assoziiert ist, umfassend:
a) In-Kontakt-Bringen einer ersten Probe eines Probanden mit Lungenkrebs, Hirntumor oder Darmkrebs oder mit dem Risiko Lungenkrebs, Hirntumor oder Darmkrebs zu entwickeln, die Nukleinsäuremoleküle umfasst, mit einem ersten und einem zweiten Vervielfältigungsprimer, wobei der erste Primer wenigstens 25 zusammenhängende Nukleotide von SEQ ID Nr.: umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder 2 hybridisiert;
b) Inkubieren der Probe unter Bedingungen, die eine Nukleinsäure-Vervielfältigung erlauben;
c) In-Kontakt-Bringen einer zweiten Probe aus einer Kontrolle, die Nukleinsäuremoleküle umfasst, mit einem ersten und einem zweiten Vervielfältigungsprimer, wobei der erste Primer wenigstens 25 zusammenhängende Nukleotide von SEQ ID Nr.: umfasst und spezifisch mit einem Nukleinsäuremolekül nach Anspruch 1 oder 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert;
d) Inkubieren der Probe unter Bedingungen, die Nukleinsäure-Vervielfältigung erlauben;
e) Vergleichen der Menge an Nukleinsäure-Vervielfältigung in der ersten Probe relativ zur Menge der Nukleinsäure-Vervielfältigung in der zweiten Probe,
wobei eine erhöhte Menge der Nukleinsäure-Vervielfältigung in der ersten Probe relativ zur zweiten Probe anzeigt, dass das Nukleinsäuremolekül mit Lungenkrebs, Hirntumor oder Darmkrebs assoziiert ist.

13. *In-vitro*-Verfahren zur Identifizierung eines Polypeptids, das mit Brustkrebs assoziiert ist, umfassend:
a) In-Kontakt-Bringen einer ersten Probe eines Probanden mit Brustkrebs oder mit dem Risiko Brustkrebs zu entwickeln, die Polypeptide umfasst, mit einem Antikörper, der spezifisch das Polypeptid nach Anspruch 4 bindet;
b) Nachweisen des Vorhandenseins eines Polypeptids in der Probe, welches an den Antikörper bindet;
c) In-Kontakt-Bringen einer zweiten Probe aus einer Kontrolle, die Polypeptide umfasst, mit einem Antikörper, der spezifisch das Polypeptid nach Anspruch 4 bindet;
d) Nachweisen des Vorhandenseins eines Polypeptids in der Probe, welches an den Antikörper bindet;
e) Vergleichen der Menge an Antikörperbindung in der ersten Probe relativ zur Menge an Antikörperbindung in der zweiten Probe,
wobei eine verringerte Menge an Antikörperbindung in der ersten Probe relativ zur zweiten Probe anzeigt, dass das Polypeptid mit Brustkrebs assozüert ist.

14. *In-vitro*-Verfahren zur Identifizierung eines Polypeptids, das mit Lungenkrebs, Hirntumor oder Darmkrebs assoziiert ist, umfassend:
a) In-Kontakt-Bringen einer ersten Probe eines Probanden mit Lungenkrebs, Hirntumor oder Darmkrebs oder mit dem Risiko Lungenkrebs, Hirntumor oder Darmkrebs zu entwickeln, die Polypeptide umfasst, mit einem Antikörper, der spezifisch das Polypeptid nach Anspruch 4 bindet;
b) Nachweisen des Vorhandenseins eines Polypeptids in der Probe, welches an den Antikörper bindet;
c) In-Kontakt-Bringen einer zweiten Probe aus einer Kontrolle, die Polypeptide umfasst, mit einem Antikörper, der spezifisch das Polypeptid nach Anspruch 4 bindet;
d) Nachweisen des Vorhandenseins eines Polypeptids in der Probe, welches an den Antikörper bindet;
e) Vergleichen der Menge an Antikörperbindung in der ersten Probe relativ zur Menge an Antikörperbindung in der zweiten Probe,
wobei eine erhöhte Menge an Antikörperbindung in der ersten Probe relativ zur zweiten Probe anzeigt, dass das Polypeptid mit Lungenkrebs, Hirntumor oder Darmkrebs assoziiert ist.

15. *In-vitro*-Verfahren zur Identifizierung eines Probanden mit Brustkrebs oder dem Risiko Brustkrebs zu entwickeln umfassend:
a) In-Kontakt-Bringen einer ersten Probe eines Probanden mit Brustkrebs oder dem Risiko Brustkrebs zu entwickeln, die Nukleinsäuremoleküle umfasst, mit einer Hybridisierungssonde, die wenigstens 25 zusammenhängende Nukleotide von SEQ ID Nr.:1 umfasst und die spezifisch mit einem Nukleinsäuremolekül nach Anspruch 1 oder Anspruch 2 hybridisiert;
b) Nachweisen des Vorhandenseins eines Nukleinsäuremoleküls in der Probe, das spezifisch mit der Sonde hybridisiert;
c) In-Kontakt-Bringen einer zweiten Probe aus einer Kontrolle, die Nukleinsäuremoleküle umfasst, mit einer Hybridisierungssonde, die wenigstens 25 zusammenhängende Nukleotide von SEQ ID Nr.:1 umfasst und die spezifisch mit einem Nukleinsäuremolekül nach Anspruch 1 oder Anspruch 2 hybridisiert;
d) Nachweisen des Vorhandenseins eines Nukleinsäuremoleküls in der Probe, das spezifisch mit der Sonde hybridisiert;
e) Vergleichen der Menge an Nukleinsäuren, die in der ersten Probe mit der Sonde hybridisieren, mit der Menge an Nukleinsäuren, die in der zweiten Probe mit der Sonde hybridisieren,
wobei eine verringerte Menge an Nukleinsäuren, die in der ersten Probe hybridisieren, relativ zur zweiten Probe einen Probanden mit Brustkrebs oder dem Risiko Brustkrebs zu entwickeln identifiziert.

16. *In-vitro*-Verfahren zur Identifizierung eines Probanden mit Lungenkrebs, Hirntumor oder Darmkrebs oder dem Risiko Lungenkrebs, Hirntumor oder Darmkrebs zu entwickeln umfassend:
a) In-Kontakt-Bringen einer ersten Probe eines Probanden mit Lungenkrebs, Hirntumor oder Darmkrebs oder dem Risiko Lungenkrebs, Hirntumor oder Darmkrebs zu entwickeln, die Nukleinsäuremoleküle umfasst, mit einer Hybridisierungssonde, die wenigstens 25 zusammenhängende Nukleotide von SEQ ID Nr.:1 umfasst und die spezifisch mit einem Nukleinsäuremolekül nach Anspruch 1 oder Anspruch 2 hybridisiert;
b) Nachweisen des Vorhandenseins eines Nukleinsäuremoleküls in der Probe, das spezifisch mit der Sonde hybridisiert;
c) In-Kontakt-Bringen einer zweiten Probe aus einer Kontrolle, die Nukleinsäuremoleküle umfasst, mit einer Hybridisierungssonde, die wenigstens 25 zusammenhängende Nukleotide von SEQ ID Nr.: 1 umfasst und die spezifisch mit einem Nukleinsäuremolekül aus Anspruch 1 oder Anspruch 2 hybridisiert;
d) Nachweisen des Vorhandenseins eines Nukleinsäuremoleküls in der Probe, das spezifisch mit der Sonde hybridisiert;
e) Vergleichen der Menge an Nukleinsäuren, die in der ersten Probe mit der Sonde hybridisieren, mit der Menge an Nukleinsäuren, die in der zweiten Probe mit der Sonde hybridisieren,
wobei eine erhöhte Menge an Nukleinsäuren, die in der ersten Probe hybridisieren, relativ zur zweiten Probe einen Probanden mit Lungenkrebs, Hirntumor oder Darmkrebs oder dem Risiko, Lungenkrebs, Hirntumor oder Darmkrebs zu entwickeln, identifiziert.

17. *In-vitro*-Verfahren zur Identifizierung eines Probanden mit Brustkrebs oder dem Risiko Brustkrebs zu entwickeln, umfassend:
a) In-Kontakt-Bringen einer ersten Probe eines Probanden mit Brustkrebs oder dem Risiko Brustkrebs zu entwickeln, die Nukleinsäuremoleküle umfasst, mit einem ersten und einem zweiten Vervielfältigungsprimer, wobei der erste Primer wenigstens 25 zusammenhängende Nukleotide von SEQ ID Nr.:1 umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert;
b) Inkubieren der Probe unter Bedingungen, die Nukleinsäure-Vervielfältigung erlauben;
c) In-Kontakt-Bringen einer zweiten Probe aus einer Kontrolle, die Nukleinsäuremoleküle umfasst, mit einem ersten und einem zweiten Vervielfältigungsprimer, wobei der erste Primer wenigstens 25 zusammenhängende Nukleotide von SEQ ID Nr.:1 umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert,
d) Inkubieren der Probe unter Bedingungen, die Nukleinsäure-Vervielfältigung erlauben;
e) Vergleichen der Menge der Nukleinsäure-Vervielfältigung in der ersten Probe relativ zur Menge der Nukleinsäure-Vervielfältigung in der zweiten Probe,
wobei eine verringerte Menge an Nukleinsäure-Vervielfältigung in der ersten Probe relativ zur zweiten Probe einen Probanden mit Brustkrebs oder dem Risiko Brustkrebs zu entwickeln, identifiziert.

18. *In-vitro*-Verfahren zur Identifizierung eines Probanden mit Lungenkrebs, Hirntumor oder Darmkrebs oder dem Risiko Lungenkrebs, Hirntumor oder Darmkrebs zu entwickeln, umfassend:
a) In-Kontakt-Bringen einer ersten Probe eines Probanden mit Lungenkrebs, Hirntumor oder Darmkrebs oder dem Risiko Lungenkrebs, Hirntumor oder Darmkrebs zu entwickeln, die Nukleinsäuremoleküle umfasst, mit einem ersten und einem zweiten Vervielfältigungsprimer, wobei der erste Primer wenigstens 25 zusammenhängende Nukleotide von SEQ ID Nr.: umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert;
b) Inkubieren der Probe unter Bedingungen, die Nukleinsäure-Vervielfältigung erlauben;
c) In-Kontakt-Bringen einer zweiten Probe aus einer Kontrolle, die Nukleinsäuremoleküle umfasst, mit einem ersten und einem zweiten Vervielfältigungsprimer, wobei der erste Primer wenigstens 25 zusammenhängende Nukleotide von SEQ ID Nr.:1 umfasst und spezifisch mit einem Nukleinsäuremolekül nach Anspruch 1 oder Anspruch 2 hybridisiert, und wobei der zweite Primer wenigstens 25 zusammenhängende Nukleotide aus dem zur SEQ ID Nr.: 1 komplementären Nukleinsäuremolekül umfasst und spezifisch mit einem Nukleinsäuremolekül nach Anspruch 1 oder Anspruch 2 hybridisiert,
d) Inkubieren der Probe unter Bedingungen, die Nukleinsäure-Vervielfältigung erlauben;
e) Vergleichen der Menge der Nukleinsäure-Vervielfältigung in der ersten Probe relativ zur Menge der Nukleinsäure-Vervielfältigung in der zweiten Probe,
wobei eine erhöhte Menge an Nukleinsäure-Vervielfältigung in der ersten Probe relativ zur zweiten Probe einen Probanden mit Lungenkrebs, Hirntumor oder Darmkrebs oder dem Risiko Lungenkrebs, Hirntumor oder Darmkrebs zu entwickeln, identifiziert.

19. *In-vitro*-Verfahren zur Identifizierung eines Probanden mit Brustkrebs oder dem Risiko Brustkrebs zu entwickeln, umfassend:
a) In-Kontakt-Bringen einer ersten Probe eines Probanden mit Brustkrebs oder dem Risiko Brustkrebs zu entwickeln, die Polypeptide umfasst, mit einem Antikörper, der spezifisch das Polypeptid nach Anspruch 4 bindet;
b) Nachweisen des Vorhandenseins eines Polypeptids in der Probe, welches an den Antikörper bindet;
c) In-Kontakt-Bringen einer zweiten Probe aus einer Kontrolle, die Polypeptide umfasst, mit einem Antikörper, der spezifisch das Polypeptid nach Anspruch 4 bindet;
d) Nachweisen des Vorhandenseins eines Polypeptids in der Probe, welches an den Antikörper bindet;
e) Vergleichen der Menge der Antikörperbindung in der ersten Probe relativ zur Menge der Antikörperbindung in der zweiten Probe;
wobei eine erniedrigte Menge an Antikörperbindung in der ersten Probe relativ zur zweiten Probe anzeigt, dass das Polypeptid mit Brustkrebs assoziiert ist.

20. *In-vitro*-Verfahren zur Identifizierung eines Probanden mit Lungenkrebs, Hirntumor oder Darmkrebs oder dem Risiko Lungenkrebs, Hirntumor oder Darmkrebs zu entwickeln, umfassend:
a) In-Kontakt-Bringen einer ersten Probe eines Probanden mit Lungenkrebs, Hirntumor oder Darmkrebs oder dem Risiko Lungenkrebs, Hirntumor oder Darmkrebs zu entwickeln, die Polypeptide umfasst, mit einem Antikörper, der spezifisch das Polypeptid nach Anspruch 4 bindet;
b) Nachweisen des Vorhandenseins eines Polypeptids in der Probe, welches an den Antikörper bindet;
c) In-Kontakt-Bringen einer zweiten Probe aus einer Kontrolle, die Polypeptide umfasst, mit einem Antikörper, der spezifisch das Polypeptid nach Anspruch 4 bindet;
d) Nachweisen des Vorhandenseins eines Polypeptids in der Probe, welches den Antikörper bindet;
e) Vergleichen der Menge der Antikörperbindung in der ersten Probe relativ zur Menge der Antikörperbindung in der zweiten Probe;
wobei eine erhöhte Menge an Antikörperbindung in der ersten Probe relativ zur zweiten Probe anzeigt, dass das Polypeptid mit Lungenkrebs, Hirntumor oder Darmkrebs assoziiert ist.

21. Verfahren zur Identifizierung eines Antikörpers, der in der Lage ist, Krebs oder eine Störung der zellulären Proliferation und/oder der Differenzierung, die durch die Aktivität des Polypeptids nach Anspruch 4 **gekennzeichnet** sind, zu behandeln, umfassend das Untersuchen der Fähigkeit des Antikörpers die Aktivität des Polypeptids nach Anspruch 4 zu modulieren, wodurch ein Antikörper identifiziert wird, der in der Lage ist, Krebs oder eine Störung der zellulären Proliferation und/oder der Differenzierung, die durch eine fehlgesteuerte Aktivität des Polypeptids nach Anspruch 4 **gekennzeichnet** sind, zu behandeln.

22. Verfahren zur Identifizierung einer Antisense-Nukleinsäure oder eines Ribozyms, die in der Lage sind, Krebs oder eine Störung der zellulären Proliferation und/oder der Differenzierung, die durch eine fehlgesteuerte Expression der Nukleinsäure nach Anspruch 1 oder Anspruch 2 **gekennzeichnet** sind, zu behandeln, umfassend das Untersuchen der Fähigkeit der Verbindung die Expression der Nukleinsäure nach Anspruch 1 oder 2 zu modulieren, wodurch eine Verbindung identifiziert wird, die in der Lage ist, Krebs oder eine Störung der zellulären Proliferation und/oder der Differenzierung, die durch fehlgesteuerte Expression der Nukleinsäure nach Anspruch 1 oder 2 **gekennzeichnet** sind, zu behandeln.

23. *In-vitro*-Verfahren zum Bewerten der Wirksamkeit einer Behandlung von Krebs oder einer Störung der zellulären Proliferation und/oder der Differenzierung in einer Probe, die von einem Probanden erhalten wurde, der nach einem unter Bewertung stehenden Protokoll behandelt wird, umfassend das Feststellen der Expressionsmenge eines in Anspruch 1 definierten Nukleinsäuremoleküls oder eines Polypeptids, das durch das Nukleinsäuremolekül kodiert wird, in der Probe, wobei eine Änderung in der Expressionsmenge der Nukleinsäure oder des Polypeptids nach der Behandlung relativ zur Menge vor der Behandlung ein Anzeichen für die Wirksamkeit der Behandlung des Krebses oder der Störung der zellulären Proliferation und/oder der Differenzierung ist.

24. Verfahren gemäß Anspruch 23, wobei die Änderung in der Expressionsmenge eine Erhöhung und der Krebs Brustkrebs ist.

25. Verfahren gemäß Anspruch 23, wobei die Änderung in der Expressionsmenge eine Abnahme und der Krebs Lungenkrebs, Hirntumor oder Darmkrebs ist.

26. *In*-*vitro*-Verfahren zur Diagnose von Krebs oder einer Störung der zellulären Proliferation und/oder der Differenzierung in einer Probe, die von einem Probanden erhalten wurde, umfassend:
Bewerten der Expression oder der Aktivität eines in Anspruch 1 definierten Nukleinsäuremoleküls oder eines Polypeptids, das durch das Nukleinsäuremolekül kodiert wird, in der Probe, so dass ein Unterschied in der Menge der Nukleinsäure oder des Polypeptids relativ zu einem normalen Probanden oder einer Kohorte normaler Probanden ein Anzeichen für Krebs oder eine Störung der zellulären Proliferation und/oder der Differenzierung ist.

27. Verfahren gemäß Anspruch 26, wobei der Unterschied in der Expressionsmenge eine Abnahme und der Krebs Brustkrebs ist.

28. Verfahren gemäß Anspruch 26, wobei der Unterschied in der Expressionsmenge eine Erhöhung und der Krebs Lungenkrebs, Hirntumor oder Darmkrebs ist.

## Revendications

1. Molécule d'acide nucléique 14911 isolée choisie dans le groupe consistant en :
(a) une molécule d'acide nucléique qui code pour un polypeptide comprenant la séquence d'acides aminés de SEQ ID No :2, ou la séquence d'acides aminés codée par l'insert d'ADNc du plasmide déposé auprès de l'ATCC sous le Numéro d'Entrée PTA-3435 ; et
(b) une molécule d'acide nucléique consistant en la séquence nucléotidique de SEQ ID No :1, ou une molécule d'acide nucléique comprenant la séquence nucléotidique de SEQ ID No :3, ou la séquence nucléotidique de l'insert d'ADN du plasmide déposé auprès de l'ATCC sous le Numéro d'Entrée PTA-3435.

2. Molécule d'acide nucléique isolée de la revendication 1, qui est la séquence nucléotidique SEQ ID No :1.

3. Cellule hôte qui contient la molécule d'acide nucléique de la revendication 1.

4. Polypeptide 14911 isolé comprenant la séquence d'acides aminés de SEQ ID No :2.

5. Procédé de production d'un polypeptide comprenant la séquence d'acides aminés de SEQ ID No :2, ou la séquence d'acides aminés codée par l'insert d'ADNc du plasmide déposé auprès de l'ATCC sous le Numéro d'Entrée PTA-3435, comprenant la culture de la cellule hôte de la revendication 3 dans des conditions dans lesquelles la molécule d'acide nucléique est exprimée.

6. Procédé in vitro de détection de la présence d'une molécule d'acide nucléique de la revendication 1 ou d'un polypeptide codé par la molécule d'acide nucléique dans un échantillon, comprenant :
(a) mettre en contact l'échantillon avec un composé qui s'hybride de façon sélective à la molécule d'acide nucléique de la revendication 1 ou se lie au polypeptide codé par la molécule d'acide nucléique ; et
(b) déterminer si le composé s'hybride à l'acide nucléique ou se lie au polypeptide dans l'échantillon.

7. Coffret comprenant un composé qui s'hybride de façon sélective à une molécule d'acide nucléique de la revendication 1 ou un anticorps qui se lie de manière spécifique à un polypeptide codé par la molécule d'acide nucléique et des instructions pour son utilisation.

8. Procédé d'identification d'un composé qui se lie à un polypeptide ou module l'activité du polypeptide de la revendication 4, comprenant les étapes de :
(a) mettre en contact un polypeptide, ou une cellule exprimant un polypeptide de la revendication 4 avec un composé d'essai ; et
(b) déterminer si le polypeptide se lie au composé d'essai ou déterminer l'effet du composé d'essai sur l'activité du polypeptide.

9. Procédé in vitro d'identification d'un acide nucléique associé au cancer du sein, comprenant :
(a) mettre en contact un premier échantillon provenant d'un sujet atteint d'un cancer du sein ou présentant un risque de développement du cancer du sein, comprenant des molécules d'acide nucléique, avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(b) détecter la présence d'une molécule d'acide nucléique dans l'échantillon qui s'hybride de manière spécifique à la sonde ;
(c) mettre en contact un second échantillon provenant d'un témoin comprenant des molécules d'acide nucléique avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(d) détecter la présence d'une molécule d'acide nucléique dans l'échantillon qui s'hybride de manière spécifique à la sonde ;
(e) comparer le taux d'acides nucléiques qui s'hybrident à la sonde dans le premier échantillon avec le taux d'acides nucléiques qui s'hybrident à la sonde dans le second échantillon,
dans lequel un taux diminué d'acides nucléiques qui s'hybrident dans le premier échantillon par rapport au second échantillon indique que la molécule d'acide nucléique dans le premier échantillon est associée au cancer du sein.

10. Procédé in vitro d'identification d'un acide nucléique associé au cancer du poumon, du cerveau ou du côlon comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des molécules d'acide nucléique, avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(b) détecter la présence d'une molécule d'acide nucléique dans l'échantillon qui s'hybride de manière spécifique à la sonde ;
(c) mettre en contact un second échantillon provenant d'un témoin comprenant des molécules d'acide nucléique avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(d) détecter la présence d'une molécule d'acide nucléique dans l'échantillon qui s'hybride de manière spécifique à la sonde ;
(e) comparer le taux d'acides nucléiques qui s'hybrident à la sonde dans le premier échantillon avec le taux d'acides nucléiques qui s'hybrident à la sonde dans le second échantillon,
dans lequel un taux accru d'acides nucléiques qui s'hybrident dans le premier échantillon par rapport au second échantillon indique que la molécule d'acide nucléique dans le premier échantillon est associée au cancer du poumon, du cerveau ou du côlon.

11. Procédé in vitro d'identification d'un acide nucléique associé au cancer du sein, comprenant :
(a) mettre en contact un premier échantillon provenant d'un sujet atteint d'un cancer du sein ou présentant un risque de développement d'un cancer du sein, comprenant des molécules d'acide nucléique, avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(b) faire incuber l'échantillon dans des conditions qui permettent une amplification d'acide nucléique ;
(c) mettre en contact un second échantillon provenant d'un témoin comprenant des molécules d'acide nucléique avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(d) faire incuber l'échantillon dans des conditions qui permettent une amplification d'acide nucléique ;
(e) comparer le taux d'amplification d'acide nucléique dans le premier échantillon par rapport au taux d'amplification d'acide nucléique dans le second échantillon,
dans lequel un taux diminué d'amplification d'acide nucléique dans le premier échantillon par rapport au second échantillon indique que la molécule d'acide nucléique est associée au cancer du sein.

12. Procédé in vitro d'identification d'un acide nucléique associé au cancer du poumon, du cerveau ou du côlon comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des molécules d'acide nucléique, avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(b) faire incuber l'échantillon dans des conditions qui permettent une amplification d'acide nucléique ;
(c) mettre en contact un second échantillon provenant d'un témoin comprenant des molécules d'acide nucléique avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(d) faire incuber l'échantillon dans des conditions qui permettent une amplification d'acide nucléique ;
(e) comparer le taux d'amplification d'acide nucléique dans le premier échantillon par rapport au taux d'amplification d'acide nucléique dans le second échantillon,
dans lequel un taux accru d'amplification d'acide nucléique dans le premier échantillon par rapport au second échantillon indique que la molécule d'acide nucléique est associée au cancer du poumon, du cerveau ou du côlon.

13. Procédé in vitro d'identification d'un polypeptide associé au cancer du sein, comprenant :
(a) mettre en contact un premier échantillon provenant d'un sujet atteint d'un cancer du sein ou présentant un risque de développement d'un cancer du sein, comprenant des polypeptides, avec un anticorps qui se lie de manière spécifique au polypeptide de la revendication 4 ;
(b) détecter la présence d'un polypeptide dans l'échantillon qui se lie à l'anticorps ;
(c) mettre en contact un second échantillon provenant d'un témoin comprenant des polypeptides avec un anticorps qui se lie de manière spécifique au polypeptide de la revendication 4 ;
(d) détecter la présence d'un polypeptide dans l'échantillon qui se lie à l'anticorps ;
(e) comparer le taux de liaison d'anticorps dans le premier échantillon par rapport au taux de liaison d'anticorps dans le second échantillon,
dans lequel un taux diminué de liaison d'anticorps dans le premier échantillon par rapport au second échantillon indique que le polypeptide est associé au cancer du sein.

14. Procédé in vitro d'identification d'un polypeptide associé au cancer du poumon, du cerveau ou du côlon, comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des polypeptides, avec un anticorps qui se lie de manière spécifique au polypeptide de la revendication 4 ;
(b) détecter la présence d'un polypeptide dans l'échantillon qui se lie à l'anticorps ;
(c) mettre en contact un second échantillon provenant d'un témoin comprenant des polypeptides, avec un anticorps qui se lie de manière spécifique au polypeptide de la revendication 4 ;
(d) détecter la présence d'un polypeptide dans l'échantillon qui se lie à l'anticorps ;
(e) comparer le taux de liaison d'anticorps dans le premier échantillon par rapport au taux de liaison d'anticorps dans le second échantillon,
dans lequel un taux accru de liaison d'anticorps dans le premier échantillon par rapport au second échantillon indique que le polypeptide est associé au cancer du poumon, du cerveau ou du côlon.

15. Procédé in vitro d'identification d'un sujet atteint d'un cancer du sein ou présentant un risque de développement d'un cancer du sein, comprenant :
(a) mettre en contact un premier échantillon provenant d'un sujet atteint d'un cancer du sein ou présentant un risque de développement d'un cancer du sein, comprenant des molécules d'acide nucléique, avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(b) détecter la présence d'une molécule d'acide nucléique dans l'échantillon qui s'hybride de manière spécifique à la sonde ;
(c) mettre en contact un second échantillon provenant d'un témoin comprenant des molécules d'acide nucléique avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(d) détecter la présence d'une molécule d'acide nucléique dans l'échantillon qui s'hybride de manière spécifique à la sonde ;
(e) comparer le taux d'acides nucléiques qui s'hybrident à la sonde dans le premier échantillon avec le taux d'acides nucléiques qui s'hybrident à la sonde dans le second échantillon,
dans lequel un taux diminué d'acides nucléiques qui s'hybrident dans le premier échantillon par rapport au second échantillon identifie un sujet atteint d'un cancer du sein ou présentant un risque de développement d'un cancer du sein.

16. Procédé in vitro d'identification d'un sujet atteint d'un cancer du poumon, du cerveau ou du côlon ou présentant un risque de développement d'un cancer du poumon, du cerveau ou du côlon, comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des molécules d'acide nucléique, avec une sonde d'hybridation comprenant au moins 25 nucléotides contigus de SEQ ID No :1, qui s'hybride de manière spécifique à une molécule d'acide nucléique de la revendication 1 ou de la revendication 2 ;
(b) détecter la présence d'une molécule d'acide nucléique dans l'échantillon qui s'hybride de manière spécifique à la sonde ;
(c) mettre en contact un second échantillon provenant d'un témoin comprenant des molécules d'acide nucléique avec une sonde d'hybridation comprenant au moins 25 nucléotides contigus de SEQ ID No :1, qui s'hybride de manière spécifique à une molécule d'acide nucléique de la revendication 1 ou de revendication 2 ;
(d) détecter la présence d'une molécule d'acide nucléique dans l'échantillon qui s'hybride de manière spécifique à la sonde ;
(e) comparer le taux d'acides nucléiques qui s'hybrident à la sonde dans le premier échantillon avec le taux d'acides nucléiques qui s'hybrident à la sonde dans le second échantillon,
dans lequel un taux accru d'acides nucléiques qui s'hybrident dans le premier échantillon par rapport au second échantillon identifie un sujet atteint d'un cancer du poumon, du cerveau ou du côlon ou présentant un risque de développement d'un cancer du poumon, du cerveau ou du côlon.

17. Procédé in vitro d'identification d'un sujet atteint d'un cancer du sein ou présentant un risque de développement d'un cancer du sein, comprenant :
(a) mettre en contact un premier échantillon provenant d'un sujet atteint d'un cancer du sein ou présentant un risque de développement d'un cancer du sein, comprenant des molécules d'acide nucléique, avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(b) faire incuber l'échantillon dans des conditions qui permettent une amplification d'acide nucléique ;
(c) mettre en contact un second échantillon provenant d'un témoin comprenant des molécules d'acide nucléique avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(d) faire incuber l'échantillon dans des conditions qui permettent une amplification d'acide nucléique ;
(e) comparer le taux d'amplification d'acide nucléique dans le premier échantillon par rapport au taux d'amplification d'acide nucléique dans le second échantillon,
dans lequel un taux diminué d'amplification d'acide nucléique dans le premier échantillon par rapport au second échantillon identifie un sujet atteint d'un cancer du sein ou présentant un risque de développement d'un cancer du sein.

18. Procédé in vitro d'identification d'un sujet atteint d'un cancer du poumon, du cerveau ou du côlon ou présentant un risque de développement d'un cancer du poumon, du cerveau ou du côlon comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des molécules d'acide nucléique avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(b) faire incuber l'échantillon dans des conditions qui permettent l'amplification d'acide nucléique ;
(c) mettre en contact un second échantillon provenant d'un témoin comprenant des molécules d'acide nucléique avec une première et une seconde amorce d'amplification, la première amorce comprenant au moins 25 nucléotides contigus de SEQ ID No :1, qui s'hybride de manière spécifique à une molécule d'acide nucléique de la revendication 1 ou de la revendication 2 et la seconde amorce comprenant au moins 25 nucléotides contigus provenant du complément de SEQ ID No :1, qui s'hybride de manière spécifique à une molécule d'acide nucléique de la revendication 1 ou de la revendication 2 ;
(d) faire incuber l'échantillon dans des conditions qui permettent une amplification d'acide nucléique ;
(e) comparer le taux d'amplification d'acide nucléique dans le premier échantillon par rapport au taux d'amplification d'acide nucléique dans le second échantillon,
dans lequel un taux accru d'amplification d'acide nucléique dans le premier échantillon par rapport au second échantillon identifie un sujet atteint d'un cancer du poumon, du cerveau ou du côlon ou présentant un risque de développement d'un cancer du poumon, du cerveau ou du côlon.

19. Procédé in vitro d'identification d'un sujet atteint d'un cancer du sein ou présentant un risque de développement d'un cancer du sein, comprenant :
(a) mettre en contact un premier échantillon provenant d'un sujet atteint d'un cancer du sein ou présentant un risque de développement d'un cancer du sein, comprenant des polypeptides, avec un anticorps qui se lie de manière spécifique au polypeptide de la revendication 4 ;
(b) détecter la présence d'un polypeptide dans l'échantillon qui se lie à l'anticorps ;
(c) mettre en contact un second échantillon provenant d'un témoin comprenant des polypeptides avec un anticorps qui se lie de manière spécifique au polypeptide de la revendication 4 ;
(d) détecter la présence d'un polypeptide dans l'échantillon qui se lie à l'anticorps ;
(e) comparer le taux de liaison d'anticorps dans le premier échantillon par rapport au taux de liaison d'anticorps dans le second échantillon,
dans lequel un taux diminué de liaison d'anticorps dans le premier échantillon par rapport au second échantillon indique que le polypeptide est associé au cancer du sein.

20. Procédé in vitro d'identification d'un sujet atteint d'un cancer du poumon, du cerveau ou du côlon ou présentant un risque de développement d'un cancer du poumon, du cerveau ou du côlon, comprenant :
(a) mettre en contact un premier échantillon provenant d'un sujet atteint d'un cancer du poumon, du cerveau ou du côlon ou présentant un risque de développement d'un cancer du poumon, du cerveau ou du côlon, comprenant des polypeptides, avec un anticorps qui se lie de manière spécifique au polypeptide de la revendication 4 ;
(b) détecter la présence d'un polypeptide dans l'échantillon qui se lie à l'anticorps ;
(c) mettre en contact un second échantillon provenant d'un témoin comprenant des polypeptides avec un anticorps qui se lie de manière spécifique au polypeptide de la revendication 4 ;
(d) détecter la présence d'un polypeptide dans l'échantillon qui se lie à l'anticorps ;
(e) comparer le taux de liaison d'anticorps dans le premier échantillon par rapport au taux de liaison d'anticorps dans le second échantillon,
dans lequel un taux accru de liaison d'anticorps dans le premier échantillon par rapport au second échantillon indique que le polypeptide est associé au cancer du poumon, du cerveau ou du côlon.

21. Procédé d'identification d'un anticorps capable de traiter un cancer ou une prolifération cellulaire et/ou un trouble de la différenciation **caractérisé par** une activité du polypeptide de la revendication 4, comprenant la détermination de la capacité de l'anticorps à moduler l'activité du polypeptide de la revendication 4, identifiant de cette façon un anticorps capable de traiter un cancer ou une prolifération cellulaire et/ou un trouble de la différenciation **caractérisé par** une activité aberrante du polypeptide de la revendication 4.

22. Procédé d'identification d'un acide nucléique anti-sens ou d'une ribozyme capable de traiter un cancer ou une prolifération cellulaire et/ou un trouble de la différenciation **caractérisé par** une expression aberrante de l'acide nucléique de la revendication 1 ou 2, comprenant la détermination de la capacité du composé à moduler l'expression de l'acide nucléique de la revendication 1 ou 2, identifiant de cette façon un composé capable de traiter un cancer ou une prolifération cellulaire et/ou un trouble de la différenciation **caractérisé par** une expression aberrante de l'acide nucléique de la revendication 1 ou 2.

23. Procédé in vitro d'évaluation de l'efficacité du traitement d'un cancer ou d'une prolifération cellulaire et/ou d'un trouble de la différenciation, dans un échantillon obtenu à partir d'un sujet qui est traité avec un protocole sous évaluation, comprenant l'appréciation du niveau d'expression d'une molécule d'acide nucléique définie dans la revendication 1 ou d'un polypeptide codé par la molécule d'acide nucléique dans l'échantillon, dans lequel un changement dans le niveau d'expression de l'acide nucléique ou du polypeptide après le traitement, par rapport au niveau avant le traitement, est indicatif de l'efficacité du traitement d'un cancer ou d'une prolifération cellulaire et/ou d'un trouble de la différenciation.

24. Procédé selon la revendication 23, dans lequel le changement dans le niveau d'expression est une augmentation et le cancer est le cancer du sein.

25. Procédé selon la revendication 23, dans lequel le changement dans le niveau d'expression est une diminution et le cancer est le cancer du poumon, du cerveau ou du côlon.

26. Procédé in vitro de diagnostic d'un cancer ou d'une prolifération cellulaire et/ou d'un trouble de la différenciation dans un échantillon obtenu à partir d'un sujet, comprenant :
évaluer l'expression ou l'activité d'une molécule d'acide nucléique définie dans la revendication 1 ou d'un polypeptide codé par la molécule d'acide nucléique dans l'échantillon, de telle sorte qu'une différence dans le niveau de l'acide nucléique ou du polypeptide par rapport à un sujet normal ou à une cohorte de sujets normaux est indicative d'un cancer ou d'une prolifération cellulaire et/ou d'un trouble de la différenciation.

27. Procédé selon la revendication 26, dans lequel la différence dans le niveau d'expression est une diminution et le cancer est le cancer du sein.

28. Procédé selon la revendication 26, dans lequel la différence dans le niveau d'expression est une augmentation et le cancer est un cancer du poumon, du cerveau ou du côlon.


### SEQUENCE LISTING

<110> Millennium Pharmaceuticals, Inc.
<120> 14911 NOVEL PROTEIN KINASE MOLECULES AND USES THEREFOR
<130> 38155-20010.40
<140> PCT/US 01/13785
   <141> 2001-04-25
<160> 11
<170> FastSEQ for Windows Version 4.0
<210> 1
   <211> 1281
   <212> DNA
   <213> Homo sapiens
<220>
   <221> CDS
   <222> (49)...(1239)
<400> 1
   ctgcccggat agtataaatc gaggatccag gtctgggcag attcaacc atg gga gcc       57
                                                        Met Gly Ala
                                                         1
   aac act tca aga aaa cca cca gtg ttt gat gaa aat gaa gat gtc aac       105
   Asn Thr Ser Arg Lys Pro Pro Val Phe Asp Glu Asn Glu Asp Val Asn
       5                  10                  15
   ttt gac cac ttt gaa att ttg cga gcc att ggg aaa ggc agt ttt ggg       153
   Phe Asp His Phe Glu Ile Leu Arg Ala Ile Gly Lys Gly Ser Phe Gly
    20                 25                  30                  35
   aag gtc tgc att gta cag aag aat gat acc aag aag atg tac gca atg       201
   Lys Val Cys Ile Val Gln Lys Asn Asp Thr Lys Lys Met Tyr Ala Met
                   40                  45                  50
   aag tac atg aat aaa caa aag tgc gtg gag cgc aat gaa gtg aga aat       249
   Lys Tyr Met Asn Lys Gln Lys Cys Val Glu Arg Asn Glu Val Arg Asn
               55                  60                  65
   gtc ttc aag gaa ctc cag atc atg cag ggt ctg gag cac cct ttc ctg       297
   Val Phe Lys Glu Leu Gln Ile Met Gln Gly Leu Glu His Pro Phe Leu
           70                  75                  80
   gtt aat ttg tgg tat tcc ttc caa gat gag gaa gac atg ttc atg gtg       345
   Val Asn Leu Trp Tyr Ser Phe Gln Asp Glu Glu Asp Met Phe Met Val
       85                  90                  95
   gtg gac ctc ctg ctg ggt gga gac ctg cgt tat cac ctg caa cag aac       393
   Val Asp Leu Leu Leu Gly Gly Asp Leu Arg Tyr His Leu Gln Gln Asn
   100                 105                 110                 115
   gtc cac ttc aag gaa gaa aca gtg aag ctc ttc atc tgt gag ctg gtc      441
   Val His Phe Lys Glu Glu Thr Val Lys Leu Phe Ile Cys Glu Leu Val
                   120                 125                 130
   atg gcc ctg gac tac ctg cag aac cag cgc atc att cac agg gat atg      489
   Met Ala Leu Asp Tyr Leu Gln Asn Gln Arg Ile Ile His Arg Asp Met
               135                 140                 145
   aag cct gac aat att tta ctt gac gaa cat ggg cac gtg cac atc aca      537
   Lys Pro Asp Asn Ile Leu Leu Asp Glu His Gly His Val His Ile Thr
           150                 155                 160
   gat ttc aac att gct gcg atg ctg ccc agg gag ata cag att acc acc      585
   Asp Phe Asn Ile Ala Ala Met Leu Pro Arg Glu Ile Gln Ile Thr Thr
       165                 170                 175
   atg gct ggc acc aag cct tac atg gca cct gag atg ttc agc tcc aga      633
   Met Ala Gly Thr Lys Pro Tyr Met Ala Pro Glu Met Phe Ser Ser Arg
   180                 185                 190                 195
   aaa gga gca ggc tat tcc ttt gct gtt gac tgg tgg tcc ctg gga gtg      681
   Lys Gly Ala Gly Tyr Ser Phe Ala Val Asp Trp Trp Ser Leu Gly Val
                   200                 205                 210
   acg gca tat gaa ctg ctg aga ggc cgg aga ccg tat cat att cgc tcc      729
   Thr Ala Tyr Glu Leu Leu Arg Gly Arg Arg Pro Tyr His Ile Arg Ser
               215                 220                 225
   agt act tcc agc aag gaa att gta cac acg ttt gag acg act gtt gta      777
   Ser Thr Ser Ser Lys Glu Ile Val His Thr Phe Glu Thr Thr Val Val
           230                 235                 240
   act tac cct tct gcc tgg tca cag gaa atg gtg tca ctt ctt aaa aag      825
   Thr Tyr Pro Ser Ala Trp Ser Gln Glu Met Val Ser Leu Leu Lys Lys
       245                 250                 255
   cta ctc gaa cct aat cca gac caa cga ttt tct cag tta tct gat gtc      873
   Leu Leu Glu Pro Asn Pro Asp Gln Arg Phe Ser Gln Leu Ser Asp Val
   260                 265                 270                 275
   cag aac ttc ccg tat atg aat gat ata aac tgg gat gca gtt ttt cag      921
   Gln Asn Phe Pro Tyr Met Asn Asp Ile Asn Trp Asp Ala Val Phe Gln
                   280                 285                 290
   aag agg ctc att cca ggt ttc att cct aat aaa ggc agg ctg aat tgt      969
   Lys Arg Leu Ile Pro Gly Phe Ile Pro Asn Lys Gly Arg Leu Asn Cys
               295                 300                 305
   gat cct acc ttt gaa ctt gag gaa atg att ttg gag tcc aaa cct cta     1017
   Asp Pro Thr Phe Glu Leu Glu Glu Met Ile Leu Glu Ser Lys Pro Leu
           310                 315                 320
   cat aag aaa aaa aag cgt ctg gca aag aag gag aag gat atg agg aaa     1065
   His Lys Lys Lys Lys Arg Leu Ala Lys Lys Glu Lys Asp Met Arg Lys
       325                 330                 335
   tgc gat tct tct cag aca tgt ctt ctt caa gag cac ctt gac tct gtc      1113
   Cys Asp Ser Ser Gln Thr Cys Leu Leu Gln Glu His Leu Asp Ser Val
   340                 345                 350                 355
   cag aag gag ttc ata att ttc aac aga gaa aaa gta aac agg gac ttt      1161
   Gln Lys Glu Phe Ile Ile Phe Asn Arg Glu Lys Val Asn Arg Asp Phe
                   360                 365                 370
   aac aaa aga caa cca aat cta gcc ttg gaa caa acc aaa gac cca caa      1209
   Asn Lys Arg Gln Pro Asn Leu Ala Leu Glu Gln Thr Lys Asp Pro Gln
               375                 380                 385
   ggt gag gat ggt cag aat aac aac ttg taa aggcctcatg tcttcttctt        1259
   Gly Glu Asp Gly Gln Asn Asn Asn Leu  *
           390                 395
   gggacaatct catgccagaa ac                                             1281
<210> 2
   <211> 396
   <212> PRT
   <213> Homo sapiens
<400> 2
   Met Gly Ala Asn Thr Ser Arg Lys Pro Pro Val Phe Asp Glu Asn Glu
    1               5                  10                  15
   Asp Val Asn Phe Asp His Phe Glu Ile Leu Arg Ala Ile Gly Lys Gly
               20                  25                  30
   Ser Phe Gly Lys Val Cys Ile Val Gln Lys Asn Asp Thr Lys Lys Met
           35                  40                  45
   Tyr Ala Met Lys Tyr Met Asn Lys Gln Lys Cys Val Glu Arg Asn Glu
       50                  55                  60
   Val Arg Asn Val Phe Lys Glu Leu Gln Ile Met Gln Gly Leu Glu His
   65                  70                  75                  80
   Pro Phe Leu Val Asn Leu Trp Tyr Ser Phe Gln Asp Glu Glu Asp Met
                   85                  90                  95
   Phe Met Val Val Asp Leu Leu Leu Gly Gly Asp Leu Arg Tyr His Leu
               100                 105                 110
   Gln Gln Asn Val His Phe Lys Glu Glu Thr Val Lys Leu Phe Ile Cys
           115                 120                 125
   Glu Leu Val Met Ala Leu Asp Tyr Leu Gln Asn Gln Arg Ile Ile His
       130                 135                 140
   Arg Asp Met Lys Pro Asp Asn Ile Leu Leu Asp Glu His Gly His Val
   145                 150                 155                 160
   His Ile Thr Asp Phe Asn Ile Ala Ala Met Leu Pro Arg Glu Ile Gln
                   165                 170                 175
   Ile Thr Thr Met Ala Gly Thr Lys Pro Tyr Met Ala Pro Glu Met Phe
               180                 185                 190
   Ser Ser Arg Lys Gly Ala Gly Tyr Ser Phe Ala Val Asp Trp Trp Ser
           195                 200                 205
   Leu Gly Val Thr Ala Tyr Glu Leu Leu Arg Gly Arg Arg Pro Tyr His
       210                 215                 220
   Ile Arg Ser Ser Thr Ser Ser Lys Glu Ile Val His Thr Phe Glu Thr
   225                 230                 235                 240
   Thr Val Val Thr Tyr Pro Ser Ala Trp Ser Gln Glu Met Val Ser Leu
                   245                 250                 255
   Leu Lys Lys Leu Leu Glu Pro Asn Pro Asp Gln Arg Phe Ser Gln Leu
               260                 265                 270
   Ser Asp Val Gln Asn Phe Pro Tyr Met Asn Asp Ile Asn Trp Asp Ala
           275                 280                 285
   Val Phe Gln Lys Arg Leu Ile Pro Gly Phe Ile Pro Asn Lys Gly Arg
       290                 295                 300
   Leu Asn Cys Asp Pro Thr Phe Glu Leu Glu Glu Met Ile Leu Glu Ser
   305                 310                 315                 320
   Lys Pro Leu His Lys Lys Lys Lys Arg Leu Ala Lys Lys Glu Lys Asp
                   325                 330                 335
   Met Arg Lys Cys Asp Ser Ser Gln Thr Cys Leu Leu Gln Glu His Leu
               340                 345                 350
   Asp Ser Val Gln Lys Glu Phe Ile Ile Phe Asn Arg Glu Lys Val Asn
           355                 360                 365
   Arg Asp Phe Asn Lys Arg Gln Pro Asn Leu Ala Leu Glu Gln Thr Lys
       370                 375                 380
   Asp Pro Gln Gly Glu Asp Gly Gln Asn Asn Asn Leu
   385                 390                 395
<210> 3
   <211> 1191
   <212> DNA
   <213> Homo sapiens
<400> 3
   atgggagcca acacttcaag aaaaccacca gtgtttgatg aaaatgaaga tgtcaacttt      60
   gaccactttg aaattttgcg agccattggg aaaggcagtt ttgggaaggt ctgcattgta     120
   cagaagaatg ataccaagaa gatgtacgca atgaagtaca tgaataaaca aaagtgcgtg     180
   gagcgcaatg aagtgagaaa tgtcttcaag gaactccaga tcatgcaggg tctggagcac     240
   cctttcctgg ttaatttgtg gtattccttc caagatgagg aagacatgtt catggtggtg     300
   gacctcctgc tgggtggaga cctgcgttat cacctgcaac agaacgtcca cttcaaggaa     360
   gaaacagtga agctcttcat ctgtgagctg gtcatggccc tggactacct gcagaaccag     420
   cgcatcattc acagggatat gaagcctgac aatattttac ttgacgaaca tgggcacgtg     480
   cacatcacag atttcaacat tgctgcgatg ctgcccaggg agatacagat taccaccatg     540
   gctggcacca agccttacat ggcacctgag atgttcagct ccagaaaagg agcaggctat     600
   tcctttgctg ttgactggtg gtccctggga gtgacggcat atgaactgct gagaggccgg     660
   agaccgtatc atattcgctc cagtacttcc agcaaggaaa ttgtacacac gtttgagacg     720
   actgttgtaa cttacccttc tgcctggtca caggaaatgg tgtcacttct taaaaagcta     780
   ctcgaaccta atccagacca acgattttct cagttatctg atgtccagaa cttcccgtat     840
   atgaatgata taaactggga tgcagttttt cagaagaggc tcattccagg tttcattcct     900
   aataaaggca ggctgaattg tgatcctacc tttgaacttg aggaaatgat tttggagtcc     960
   aaacctctac ataagaaaaa aaagcgtctg gcaaagaagg agaaggatat gaggaaatgc    1020
   gattcttctc agacatgtct tcttcaagag caccttgact ctgtccagaa ggagttcata    1080
   attttcaaca gagaaaaagt aaacagggac tttaacaaaa gacaaccaaa tctagccttg    1140
   gaacaaacca aagacccaca aggtgaggat ggtcagaata acaacttgta a             1191
<210> 4
   <211> 270
   <212> PRT
   <213> Artificial Sequence
<220>
   <223> Amino acid consensus sequence of the Ser/Thr kinase site
<221> VARIANT
   <222> (1) ... (270)
   <223> Xaa = Any Amino Acid
<400> 4
   Xaa Xaa Xaa Xaa Xaa Xaa Xaa Xaa Xaa Gly Xaa Gly Xaa Xaa Xaa Xaa
    1               5                  10                  15
   Val Xaa Xaa Xaa Xaa Xaa Xaa Xaa Xaa Xaa Xaa Xaa Xaa Lys Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Glu Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa His Xaa Xaa Xaa Xaa Xaa Xaa Xaa Xaa His Arg Asp
           115                 120                 125
   Xaa Lys Xaa Xaa Asn Xaa Xaa Xaa Xaa Xaa Xaa Xaa Xaa Xaa Xaa Xaa
       130                 135                 140
   Xaa Xaa Xaa Xaa Xaa Xaa Lys Xaa Xaa Asp Phe Gly Xaa Xaa Xaa Xaa
   145                 150                 155                 160
   Xaa Xaa Xaa Xaa Xaa Xaa Xaa Xaa Xaa Xaa Xaa Xaa Xaa Xaa Xaa Xaa
                   165                 170                 175
   Xaa Pro Xaa Xaa Xaa Xaa Xaa Xaa Xaa Xaa Xaa Xaa Xaa Xaa Xaa Trp
               180                 185                 190
   Xaa Xaa Xaa Gly Xaa Xaa Xaa Xaa Xaa Xaa Xaa Xaa Xaa Xaa Xaa Xaa
           195                 200                 205
   Xaa Xaa Xaa Xaa Xaa Xaa Xaa Xaa Xaa Xaa Xaa Xaa Xaa Xaa Xaa Xaa
       210                 215                 220
   Xaa Xaa Xaa Xaa Xaa Xaa Xaa Xaa Xaa Xaa Xaa Xaa Xaa Xaa Xaa Xaa
   225                 230                 235                 240
   Xaa Xaa Xaa Xaa Xaa Xaa Xaa Xaa Xaa Xaa Xaa Arg Xaa Xaa Xaa Xaa
                   245                 250                 255
   Xaa Xaa Xaa Xaa Xaa Xaa Xaa Xaa Xaa Xaa His Xaa Xaa Xaa
               260                 265                 270
<210> 5
   <211> 30
   <212> PRT
   <213> Artificial Sequence
<220>
   <223> Consensus amino acid
<221> VARIANT
   <222> (1)... (30)
   <223> Xaa = Any Amino Acid
<400> 5
   Gly Xaa Gly Xaa Xaa Gly Xaa Xaa Xaa Xaa Xaa Xaa Xaa Xaa Xaa Xaa
    1               5                  10                  15
   Xaa Xaa Xaa Xaa Xaa Xaa Xaa Xaa Xaa Xaa Xaa Xaa Xaa Lys
               20                  25                  30
<210> 6
   <211> 278
   <212> PRT
   <213> Artificial Sequence
<220>
   <223> Consensus amino acid
<400> 6
   Tyr Glu Leu Leu Glu Lys Leu Gly Glu Gly Ser Phe Gly Lys Val Tyr
    1              5                  10                  15
   Lys Ala Lys His Lys Thr Gly Lys Ile Val Ala Val Lys Ile Leu Lys
               20                  25                  30
   Lys Glu Ser Leu Ser Leu Arg Glu Ile Gln Ile Leu Lys Arg Leu Ser
           35                  40                  45
   His Pro Asn Ile Val Arg Leu Leu Gly Val Phe Glu Asp Thr Asp Asp
       50                  55                  60
   His Leu Tyr Leu Val Met Glu Tyr Met Glu Gly Gly Asp Leu Phe Asp
   65                  70                  75                  80
   Tyr Leu Arg Arg Asn Gly Pro Leu Ser Glu Lys Glu Ala Lys Lys Ile
                   85                  90                  95
   Ala Leu Gln Ile Leu Arg Gly Leu Glu Tyr Leu His Ser Asn Gly Ile
               100                 105                 110
   Val His Arg Asp Leu Lys Pro Glu Asn Ile Leu Leu Asp Glu Asn Gly
           115                 120                 125
   Thr Val Lys Ile Ala Asp Phe Gly Leu Ala Arg Leu Leu Glu Lys Leu
       130                 135                 140
   Thr Thr Phe Val Gly Thr Pro Trp Tyr Met Met Ala Pro Glu Val Ile
   145                 150                 155                 160
   Leu Glu Gly Arg Gly Tyr Ser Ser Lys Val Asp Val Trp Ser Leu Gly
                   165                 170                 175
   Val Ile Leu Tyr Glu Leu Leu Thr Gly Gly Pro Leu Phe Pro Gly Ala
               180                 185                 190
   Asp Leu Pro Ala Phe Thr Gly Gly Asp Glu Val Asp Gln Leu Ile Ile
           195                 200                 205
   Phe Val Leu Lys Leu Pro Phe Ser Asp Glu Leu Pro Lys Thr Arg Ile
       210                 215                 220
   Asp Pro Leu Glu Glu Leu Phe Arg Ile Lys Lys Arg Arg Leu Pro Leu
   225                 230                 235                 240
   Pro Ser Asn Cys Ser Glu Glu Leu Lys Asp Leu Leu Lys Lys Cys Leu
                   245                 250                 255
   Asn Lys Asp Pro Ser Lys Arg Pro Gly Ser Ala Thr Ala Lys Glu Ile
               260                 265                 270
   Leu Asn His Pro Trp Phe
           275
<210> 7
   <211> 20
   <212> PRT
   <213> Artificial Sequence
<220>
   <223> Consensus amino acid
<400> 7
   Arg Glu Ile Asp Trp Asp Lys Leu Glu Asn Lys Glu Ile Glu Pro Pro
   1 5 10 15
   Phe Lys Pro Lys
   20
<210> 8
   <211> 129
   <212> PRT
   <213> Artificial Sequence
<220>
   <223> Consensus amino acid
<400> 8
   Pro Tyr Tyr Val Ser Met Lys Ser Met Ala Pro Glu Tyr Met Ala Pro
   1 5 10 15
   Glu Ser Ser Ala Thr Asn Tyr Gln Lys Tyr Ser Thr Lys Ser Asp Val
   20 25 30
   Trp Ser Phe Gly Val Ile Leu Tyr Glu Met Leu Thr Gly Lys Pro Pro
   35 40 45
   Phe Phe Pro Gly Glu Ser Glu Val Ser Glu Glu Glu Pro Tyr Gln Ser
   50 55 60
   Met Lys Asn Met Glu Val Leu Glu Met Gly Pro Glu Glu Thr Ile Gln
   65 70 75 80
   Lys Val Met Ser Lys Ile Val Glu Lys Lys Gly Glu Arg Met Pro Gln
   85 90 95
   Pro Ser Ser Ser Asn Cys Pro Glu Val Ser Gln Glu Ala Lys Asp Leu
   100 105 110
   Leu Lys Lys Cys Leu Gln Lys Asp Pro Glu Lys Arg Arg Pro Thr Phe
   115 120 125
   Glu
<210> 9
   <211> 43
   <212> PRT
   <213> Artificial Sequence
<220>
   <223> Consensus amino acid
<400> 9
   Tyr Val His Gln Ile Ala Lys Gly Leu Glu Tyr Leu His Ser Lys Asn
   1 5 10 15
   Gln Lys His Gln Gly Ile Ile His Arg Ala Lys Lys Val Asp Leu Lys
   20 25 30
   Pro Glu Asn Ile Leu Leu Asp Glu Glu Ser His
   35 40
<210> 10
   <211> 53
   <212> PRT
   <213> Artificial Sequence
<220>
   <223> Consensus amino acid
<400> 10
   Tyr Glu Leu Leu Lys Lys Leu Leu Gly Lys Gly Ser Phe Gly Lys Val
    1               5                  10                  15
   Tyr Lys Ala Lys His Lys Ser Thr Ser Thr Thr Gly Glu Val Val Ala
               20                  25                  30
   Val Lys Val Met Lys Lys Lys Lys Val Met Glu Lys Ser Ser Lys Glu
           35                  40                  45
   Ser Ser Ser Lys Lys
       50
<210> 11
   <211> 31
   <212> PRT
   <213> Artificial Sequence
<220>
   <223> Consensus amino acid
<400> 11
   Ser Gln Glu Ala Lys Asp Leu Leu Lys Lys Cys Leu Gln Lys Asp Pro
    1               5                  10                  15
   Glu Lys Arg Arg Pro Thr Phe Glu Glu Ile Leu Gln His Pro Trp
               20                  25                  30